# EUROPEAN PATENT SPECIFICATION

(11) **EP 3 914 617 B1**
(45) Date of publication and mention of the grant of the patent: **13.03.2024**
(21) Application number: 20708762.8
(22) Date of filing: 24.01.2020
(51) Int. Cl.: C07K 16/18, A61P 7/00, C07K 16/40, C07K 16/28, C07K 16/46

(54) **DOSAGE AND ADMINISTRATION OF ANTI-C5 ANTIBODIES FOR TREATMENT OF ATYPICAL HEMOLYTIC UREMIC SYNDROME (AHUS)**
DOSIERUNG UND VERABREICHUNG VON ANTI-C5-ANTIKÖRPERN ZUR BEHANDLUNG DES ATYPISCHEN HÄMOLYTISCH-URÄMISCHEN SYNDROMS (AHUS)
DOSAGE ET ADMINISTRATION D'ANTICORPS ANTI-C5 POUR LE TRAITEMENT DU SYNDROME HÉMOLYTIQUE ET URÉMIQUE ATYPIQUE (AHUS)

(30) Priority: 25.01.2019 US 201962796953 P
(43) Date of publication of application: 01.12.2021
(73) Proprietor: Alexion Pharmaceuticals, Inc., Boston, MA 02210 (US)
(72) Inventor: PAYTON, Lori, Madison, CT 06443 (US); MIX, Christian, Wellesley, MA 02481 (US); PRADHAN, Rajendra, New Haven, CT 06510 (US); DAMOKOSH, Andrew, Hartford, CT 06107 (US); SWENSON, Eugene Scott, Madison, CT 06443 (US); GAO, Xiang, Guilford, CT 06437 (US)
(74) Representative: J A Kemp LLP
(86) International application number: PCT/US2020/014998
(87) International publication number: WO 2020/154626

(56) References cited:
- WO-A1-2017/123636
- WO-A1-2019/084438
- WO-A1-2019/236345
- Anonymous: "Ravulizumab for atypical haemolytic uraemic syndrome in adults and children - first line", , 1 August 2018 (2018-08-01), pages 1-10, XP055699410, Retrieved from the Internet: URL:http://www.io.nihr.ac.uk/wp-content/up loads/2018/08/13606-Ravulizumab-for-aHUS-V 1.0-AUG2018-NON-CONF.pdf [retrieved on 2020-05-28]
- Anonymous: "Alexion Receives FDA Approval for ULTOMIRIS (ravulizumab-cwvz) for Atypical Hemolytic Uremic Syndrome (aHUS)", , 18 October 2019 (2019-10-18), XP055699418, Retrieved from the Internet: URL:https://ir.alexion.com/node/21936/pdf [retrieved on 2020-05-28]
- EDWIN K.S. WONG ET AL: "Anticomplement C5 therapy with eculizumab for the treatment of paroxysmal nocturnal hemoglobinuria and atypical hemolytic uremic syndrome", TRANSLATIONAL RESEARCH, vol. 165, no. 2, 1 February 2015 (2015-02-01), pages 306-320, XP055358380, NL ISSN: 1931-5244, DOI: 10.1016/j.trsl.2014.10.010
- ERIC RONDEAU ET AL: "The long-acting C5 inhibitor, Ravulizumab, is effective and safe in adult patients with atypical hemolytic uremic syndrome naïve to complement inhibitor treatment", KIDNEY INTERNATIONAL, 6 March 2020 (2020-03-06), pages 1-10, XP055683955,

## Description

### BACKGROUND

The complement system acts in conjunction with other immunological systems of the body to defend against intrusion of cellular and viral pathogens. There are at least 25 complement proteins, which are found as a complex collection of plasma proteins and membrane cofactors. The plasma proteins make up about 10% of the globulins in vertebrate serum. Complement components achieve their immune defensive functions by interacting in a series of intricate but precise enzymatic cleavage and membrane binding events. The resulting complement cascade leads to the production of products with opsonic, immunoregulatory, and lytic functions. A concise summary of the biologic activities associated with complement activation is provided, for example, in The Merck Manual, 16th Edition.

While a properly functioning complement system provides a robust defense against infecting microbes, inappropriate regulation or activation of the complement pathways has been implicated in the pathogenesis of a variety of disorders, including atypical hemolytic uremic syndrome (aHUS). aHUS is an ultra-rare disorders driven by chronic uncontrolled complement activation. The resulting inflammation and cellular damage lead to the devastating clinical manifestations of these diseases.

Hemolytic uremic syndrome (HUS) is characterized by thrombocytopenia, microangiopathic hemolytic anemia, and acute renal failure. HUS is classified as one of two types: diarrheal-associated (D+ HUS; also referred to as shiga toxin producing *E. coli* (STEC)-HUS or typical HUS) and non-diarrheal or atypical HUS (aHUS). D+ HUS is the most common form, accounting for greater than 90% of cases and is caused by a preceding illness with a shiga-like toxin-producing bacterium, e.g., *E. coli* O157:H7.

aHUS can be genetic, acquired, or idiopathic. Hereditable forms of aHUS can be associated with mutations in a number of human complement components including, e.g., complement factor H (CFH), membrane cofactor protein (MCP), complement factor I (CFI), C4b-binding protein (C4BP), complement factor B (CFB), and complement component 3 (C3). See, e.g., Caprioli et al. (2006) Blood 108: 1267-1279. Certain mutations in the gene encoding CD55, though not yet implicated in aHUS, are associated with the severity of aHUS. See, e.g., Esparza-Gordillo et al. (2005) Hum Mol Genet 14:703-712.

aHUS is rare and has a mortality rate of up to 25%. Many patients with this disease will sustain permanent neurological or renal impairment, e.g., at least 50% of aHUS patients progress to end-stage renal failure (ESRF). See, e.g., Kavanagh et al. (2006) British Medical Bulletin 77 and 78:5-22. Until recently, treatment options for patients with aHUS were limited and often involved plasma infusion or plasma exchange. In some cases, aHUS patients undergo uni- or bilateral nephrectomy or renal transplantation (see Artz et al. (2003) Transplantation 76:821-826). However, recurrence of the disease in treated patients is common.

Patients with aHUS are at risk of substantial morbidity and mortality.

It has been reported that Ravulizumab is being developed for the treatment of atypical haemolytic uraemic syndrome (aHUS) in adults and children (1 August 2018, pages 1-10, URL:http://www.io.nihr.ac.uk/wp-content/uploads/2018/08/13606-Ravulizumab-for-aHUS-V1.0-AUG2018-NON-CONF.pdf).

Accordingly, it is an object of the present invention to provide improved methods for treating patients with aHUS.

**SUMMARY** The scope of the invention is defined by the appended claims.

Provided herein is an anti-C5 antibody for use in a method for treating aHUS in a human adult patient who is18 years or older, wherein the anti-C5 antibody is ravulizumab, and the anti-C5 antibody is administered intravenously (a) once on Day 1 at a dose of: 2400 mg to a patient weighing ≥ 40 to < 60 kg, 2700 mg to a patient weighing ≥ 60 to < 100 kg, or 3000 mg to a patient weighing ≥ 100 kg; and (b) on Day 15 and every eight weeks thereafter at a dose of 3000 mg to a patient weighing ≥ 40 to < 60 kg, 3300 mg to a patient weighing ≥ 60 to < 100 kg, or 3600 mg to a patient weighing ≥ 100 kg.

The anti-C5 antibody may be administered for one or more administration cycles. The administration cycle may be 26 weeks. The anti-C5 antibody may be administered once on Day 1 of the administration cycle, once on Day 15 of the administration cycle, and every eight weeks thereafter. The anti-C5 antibody may be administered every eight weeks after the administration cycle for an extension period up to two years (*e.g*., at a dose of 3000 mg, 3300 mg, or 3600 mg).

The anti-C5 antibody may be administered for one or more administration cycles. The administration cycle may be 26 weeks. The treatment may comprise at least 1, 2, 3, 4, 5, 6, 7, 8, 9, 10, or 11 cycles. The treatment may continue for the lifetime of the human patient.

In another embodiment, the anti-C5 antibody is administered to a patient weighing ≥ 40 to < 60 kg:
(a) once on Day 1 at a dose of 2400 mg; and
(b) on Day 15 and every eight weeks thereafter at a dose of 3000 mg.

In another embodiment, the anti-C5 antibody is administered to a patient weighing ≥ 60 to < 100 kg:
(a) once on Day 1 at a dose of 2700 mg; and
(b) on Day 15 and every eight weeks thereafter at a dose of 3300 mg.

In another embodiment, the anti-C5 antibody is administered to a patient weighing ≥ 100 kg:
(a) once on Day 1 at a dose of 3000 mg; and
(b) on Day 15 and every eight weeks thereafter at a dose of 3600 mg.

The patient may not have previously been treated with a complement inhibitor (*e.g.,* the patient is a complement inhibitor treatment-naive patient).

The patient may have previously been treated with one anti-C5 antibody, or antigen binding fragment thereof, and is switched to ravulizumab during the course of treatment. For example, different anti-C5 antibodies may be administered during the course of treatment. Different anti-C5 antibodies may be administered during separate treatment and extension periods. For example, the patient may be treated with eculizumab during a treatment period (*e.g.,* for 26 weeks), followed by treatment with ravulizumab, for example, during an extension period. Eculizumab may be administered to the patient at a dose of 600 mg on Days 1, 8, 15, and 22 of the administration cycle during an induction phase, followed by a maintenance dose of 900 mg of eculizumab on Day 19 of the administration cycle and every two weeks thereafter (*e.g.,* for a total of 26 weeks), followed by treatment with ravulizumab for an extension period of up to two years. The patient may be treated with ravulizumab (*e.g.,* for 26 weeks), followed by treatment with another anti-C5 antibody (*e.g.,* eculizumab, 7086 antibody, 8110 antibody, 305LO5 antibody, SKY59 antibody, or REGN3918 antibody) during, for example, an extension period.

The patient may have previously been treated for at least 1 month, at least 2 months, at least 3 months, at least 4 months, at least 5 months, at least 6 months, at least 7 months, at least 8 months, at least 9 months, at least 10 months, at least 11 months, at least 12 months, at least 18 months, or at least 24 months with an anti-C5 antibody, or antigen binding fragment thereof, (*e.g.,* eculizumab) before switching to ravulizumab. In a particular embodiment, the patient has previously been treated for at least 6 months with eculizumab.

Where the patient is treated with a first anti-C5 antibody and then switched to treatment with a second different anti-C5 antibody, especially where the second different anti-C5 antibody binds to a different epitope on C5 than the first anti-C5 antibody, and wherein the first or second anti-C5 antibody is ravulizumab, the administration schedules may take into account the half-life of the first anti-C5 antibody. For example, to ensure that the first anti-C5 antibody is cleared (*e.g.,* "washed out") from the patient before the second (different) anti-C5 antibody is administered (*e.g.,* to avoid issues associated with aggregation, immune complex formation, etc.), the half-life of the first anti-C5 antibody may be taken into consideration. The second (different) anti-C5 antibody may not be administered until a duration of time corresponding to 2, 2.5, 3, 3.5, 4, 4.5, 5, 5.5, 6, 6.5, 7, or 7.5 times the half-life of the first anti-C5 antibody has passed after the final administration of the first anti-C5 antibody.

In another embodiment, the patient has previously been treated with eculizumab and then is switched to treatment with a second (different) anti-C5 antibody which is ravulizumab. Where eculizumab is the first administered antibody, ravulizumab may not be administered, for example, until at least 36, 45, 54, 63, 72, 81, 90, 99, 108, 117, or 126 days have passed after the final administration of eculizumab.

After treatment with ravulizumab, the patient may be switched to treatment with a different anti-C5 antibody (*e.g.,* eculizumab, 7086 antibody, 8110 antibody, 305LO5 antibody, SKY59 antibody, or REGN3918 antibody). Where ravulizumab is the first administered antibody, the second (different) anti-C5 antibody is not administered, for example, until at least 100, 125, 150, 175, 200, 225, 250, 275, 300, 325, 375, or 400 days have passed after the final administration of ravulizumab.

Additionally, or alternatively, techniques are used to clear or enhance clearance of the first anti-C5 antibody before switching to treatment with a second (different) anti-C5 antibody. Exemplary techniques include, but are not limited to, plasmapheresis or blood transfusions. An antibody against the first anti-C5 antibody (*e.g.,* an anti-eculizumab antibody, an anti-ravulizumab antibody, an anti-7086 antibody, an anti-8110 antibody, an anti-305LO5 antibody, an anti-SKY59 antibody, or an anti-REGN3918 antibody) may be administered to clear or enhance clearance of the first anti-C5 antibody before a second (different) anti-C5 antibody is administered.

The treatment (e.g., administration cycle) relating to administration of ravulizumab may start at least about two weeks, at least about three weeks, at least about four weeks, at least about six weeks, at least about seven weeks, or at least about eight weeks after the patient's last dose of eculizumab. In another embodiment, the treatment (*e.g.,* administration cycle) starts at least two weeks after the patient's last dose of eculizumab.

The patients treated according to the methods described herein may have been vaccinated against meningococcal infections within 3 years prior to, or at the time of, initiating treatment. Patients who received treatment less than 2 weeks after receiving a meningococcal vaccine may also be treated with appropriate prophylactic antibiotics until 2 weeks after vaccination. Patients treated according to the methods described herein may be vaccinated against meningococcal serotypes A, C, Y, W135, and/or B.

The treatment regimens described are sufficient to maintain particular serum trough concentrations of the anti-C5 antibody, or antigen binding fragment thereof. For example, the treatment maintains a serum trough concentration of the anti-C5 antibody of 50, 55, 60, 65, 70, 75, 80, 85, 90, 95, 100, 105, 110, 115, 120, 125, 130, 135, 140, 145, 150, 155, 160, 165, 170, 175, 180, 185, 190, 200, 205, 210, 215, 220, 225, 230, 240, 245, 250, 255, 260, 265, 270, 280, 290, 300, 305, 310, 315, 320, 325, 330, 335, 340, 345, 350, 355, 360, 365, 370, 375, 380, 385, 390, 395, or 400 µg/ml or greater during the treatment. In one embodiment, the treatment maintains a serum trough concentration of the anti-C5 antibody of 100 µg/ml or greater. The treatment may maintain a serum trough concentration of the anti-C5 antibody of 150 µg/ml or greater. In another embodiment, the treatment maintains a serum trough concentration of the anti-C5 antibody of 200 µg/ml or greater. The treatment may maintain a serum trough concentration of the anti-C5 antibody of 250 µg/ml or greater, or of 300 µg/ml or greater. The treatment may maintain a serum trough concentration of the anti-C5 antibody of between 100 µg/ml and 200 µg/ml, such as of about 175 µg/ml.

To obtain an effective response, the anti-C5 antibody may be administered to the patient in an amount and with a frequency to maintain at least 50 µg, 55µg, 60 µg, 65 µg, 70 µg, 75 µg, 80 µg, 85 µg, 90 µg, 95 µg, 100 µg, 105 µg, 110 µg, 115 µg, 120 µg, 125 µg, 130 µg, 135 µg, 140 µg, 145 µg, 150 µg, 155 µg, 160 µg, 165 µg, 170 µg, 175 µg, 180 µg, 185 µg, 190 µg, 195 µg, 200 µg, 205 µg, 210 µg, 215 µg, 220 µg, 225 µg, 230 µg, 235 µg, 240 µg, 245 µg, 250 µg, 255 µg, or 260 µg of antibody per milliliter of the patient's blood. The anti-C5 antibody may be administered to the patient in an amount and with a frequency to maintain between 50 µg and 250 µg of antibody per milliliter of the patient's blood, such as between 100 µg and 200 µg of antibody per milliliter of the patient's blood. The anti-C5 antibody may be administered to the patient in an amount and with a frequency to maintain about 175 µg of antibody per milliliter of the patient's blood.

To obtain an effective response, the anti-C5 antibody may be administered to the patient in an amount and with a frequency to maintain a minimum free C5 concentration. For example, the anti-C5 antibody may be administered to the patient in an amount and with a frequency to maintain a free C5 concentration of 0.2 µg/mL, 0.3 µg/mL, 0.4 µg/mL, 0.5 µg/mL or below. In another embodiment, the anti-C5 antibody is administered to the patient in an amount and with a frequency to maintain a free C5 concentration of 0.309 to 0.5 µg/mL or below. The treatment described herein may reduce free C5 concentration by greater than 99% throughout the treatment period. In another embodiment, the treatment reduces free C5 concentration greater than 99.5% throughout the treatment period.

The anti-C5 antibodies can be administered to a patient by any suitable means. The antibodies may be formulated for intravenous administration.

The efficacy of the treatment methods described herein can be assessed using any suitable means. In one embodiment, for an aHUS patient, the treatment produces at least one therapeutic effect selected from the group consisting of a reduction or cessation in severe hypertension, proteinuria, uremia, lethargy/fatigue, irritability, thrombocytopenia, microangiopathic hemolytic anemia, and renal function impairment (*e.g.,* acute renal failure).

In other embodiments, the treatment results in terminal complement inhibition.

In other embodiments, the treatment produces a shift toward normal levels of a hemolysis-related hematologic biomarker selected from the group consisting of free hemoglobin, haptoglobin, reticulocyte count, PNH red blood cell (RBC) clone and D-dimer.

In another embodiment, the treatment produces an increase in hemoglobin stabilization from the patient's pre-treatment baseline. The treatment may result in a ≥ 20 g/L increase in hemoglobin. The treatment may result in avoidance of a ≥ 2 g/dL decrease in hemoglobin level from baseline in the absence of transfusion from baseline to Day 183. 9

In other embodiments, the treatment results in platelet normalization (≥150 × 10⁹ /L). 9 The treatment may result in platelet normalization (≥150 × 10⁹ /L) for at least 28 days (*e.g.*, at least 28 days, 1 month, 2 months, 3 months, 4 months, 5 months, 6 months, 7 months, 8 months, 9 months, 10 months, 11 months, 1 year, or two years).

In other embodiments, the treatment results in LDH normalization (≤246 U/L). The treatment may result in LDH normalization (≤246 U/L) for at least 28 days (*e.g.,* at least 28 days, 1 month, 2 months, 3 months, 4 months, 5 months, 6 months, 7 months, 8 months, 9 months, 10 months, 11 months, 1 year, or two years).

In other embodiments, the treatment results in a ≥25% improvement from baseline in serum creatinine. The treatment may result in a ≥25% improvement from baseline in serum creatinine for at least 28 days (*e.g.,* at least 28 days, 1 month, 2 months, 3 months, 4 months, 5 months, 6 months, 7 months, 8 months, 9 months, 10 months, 11 months, 1 year, or two years).

In other embodiments, the treatment results in a complete TMA response (*i.e.,* platelet 9 normalization (≥150 × 10 /L), LDH normalization (≤246 U/L), and a ≥25% improvement from baseline in serum creatinine). The treatment may result in a complete TMA response for at least 28 days (*e.g.,* at least 28 days, 1 month, 2 months, 3 months, 4 months, 5 months, 6 months, 7 months, 8 months, 9 months, 10 months, 11 months, 1 year, or two years).

In other embodiments, the treatment results in a modified complete TMA Response 9 (*i.e.,* platelet normalization (≥150 × 10 /L), LDH normalization (≤246 U/L), and the patient is off dialysis if they were on dialysis at baseline or a ≥25% improvement from baseline in serum creatinine for a patient who was off dialysis at baseline). The treatment may result in a modified complete TMA Response for at least 28 days (*e.g.,* at least 28 days, 1 month, 2 months, 3 months, 4 months, 5 months, 6 months, 7 months, 8 months, 9 months, 10 months, 11 months, 1 year, or two years).

In other embodiments, the treatment produces a reduction in the need for blood transfusions. The treatment may produce a greater than 70% increase in transfusion avoidance. The treatment may result in transfusion avoidance from baseline to Day 183.

In other embodiments, the treatment results in an elimination of breakthrough hemolysis during the treatment period. The treatment may result in a reduction of breakthrough hemolysis compared to pretreatment baseline amount of breakthrough hemolysis.

In other embodiments, the treatment produces a reduction in major adverse vascular events (MAVEs).

In other embodiments, the treatment produces a change from baseline in quality of life as assessed via the Functional Assessment of Chronic Illness Therapy (FACIT)-Fatigue Scale, version 4 and the European Organisation for Research and Treatment of Cancer, Quality of Life Questionnaire-Core 30 Scale. The treatment may produce a change from baseline in quality of life as assessed via the FACIT-Fatigue Scale by one or more (*e.g.,* 1, 2, or 3) points. The treatment may produce a change from baseline in quality of life as assessed via the FACIT-Fatigue Scale by 3 points, 150 days or more (*e.g.,* 150 days, 151 days, 152 days, 153 days, 154 days, 155 days, 156 days, 157 days, 158 days, 159 days, 160 days, 161 days, 162 days, 163 days, 164 days, 165 days, 166 days, 167 days, 168 days, 169 days, 170 days, 171 days, 172 days, 173 days, 174 days, 175 days, 176 days, 177 days, 178 days, 179 days, 180 days, 181 days, 182 days 183 days, 184 days, 185 days, 186 days, 187 days, 188 days, 189 days, 190 days, 191 days, 192 days, 193 days, 194 days, 195 days, 196 days, 197 days, 198 days, 199 days, 200 days, 205 days, 210 days, 215 days, 220 days, or 225 days) after initiating treatment.

Chronic kidney disease (CKD) stage is classified based on the National Kidney Foundation Chronic Kidney Disease Stage. The stages of CKD and corresponding estimated glomerular filtration rate (eGFR) values are as follows: Stage 1: eGFR >=90 (normal), Stage 2: eGFR 60-89, Stage 3A: eGFR 45-59, Stage 3B: eGFR 30-44, Stage 4: eGFR 15-29, and Stage 5: eGFR <15 (including dialysis: End stage). Stage 1 is considered the best category. Stage 5 is considered the worst category. An improvement in eGFR (*e.g.,* > 15) corresponds with an improvement in CKD stage (*e.g.,* a lower CKD stage). Accordingly, in other embodiments, the patient's chronic kidney disease (CKD) improves by one or more stages after initiating treatment. For example, the patient's CKD may improve by one, two, three, four, or five stages). The patient's CKD may improve by one or more stages 150 days or more (*e.g.,* 150 days, 151 days, 152 days, 153 days, 154 days, 155 days, 156 days, 157 days, 158 days, 159 days, 160 days, 161 days, 162 days, 163 days, 164 days, 165 days, 166 days, 167 days, 168 days, 169 days, 170 days, 171 days, 172 days, 173 days, 174 days, 175 days, 176 days, 177 days, 178 days, 179 days, 180 days, 181 days, 182 days 183 days, 184 days, 185 days, 186 days, 187 days, 188 days, 189 days, 190 days, 191 days, 192 days, 193 days, 194 days, 195 days, 196 days, 197 days, 198 days, 199 days, 200 days, 205 days, 210 days, 215 days, 220 days, or 225 days) after initiating treatment.

The treatment may result in an increase in eGFR compared to baseline. In other embodiments, the treatment results in a shift towards normal levels of eGFR (*e.g.*, ≥ 90). The treatment may result in an increase in eGFR compared to baseline and the patient's CKD improves by one or more stages. The treatment may result in a shift towards normal levels of eGFR (*e.g.*, ≥ 90) compared to baseline and the patient's CKD improves by one or more stages.

In other embodiments, the treatment results in a EQ-5D-3L Time Trade-Off value set for the United States (US TTO) of > 0.94.

The antibody may be determined to be safe, tolerable and sufficiently non-immunogenic after multiple IV doses for use in aHUS patients.

Further described are kits suitable for treating atypical hemolytic uremic syndrome (aHUS) in a human adult patient who is 18 years or older by intravenously administering a pharmaceutical composition comprising ravulizumab: (a) once on Day 1 at a dose of: 2400 mg to a patient weighing ≥ 40 to < 60 kg, 2700 mg to a patient weighing ≥ 60 to < 100 kg, or 3000 mg to a patient weighing ≥ 100 kg; and (b) on Day 15 and every eight weeks thereafter at a dose of 3000 mg to a patient weighing ≥ 40 to < 60 kg, 3300 mg to a patient weighing ≥ 60 to < 100 kg, or 3600 mg to a patient weighing ≥ 100 kg. The kits include a pharmaceutical composition containing an anti-C5 antibody, which is antibody ravulizumab, and a pharmaceutically-acceptable carrier.

### BRIEF DESCRIPTION OF THE DRAWINGS

**Figure 1** depicts the study design for ALXN1210-aHUS-311.
**Figure 2** summarizes the primary, secondary, and safety endpoints for ALXN1210-aHUS-311.
**Figure 3** summarizes the inclusion and exclusion criteria for ALXN1210-aHUS-311.
**Figure 4** shows the patient disposition for ALXN1210-aHUS-311.
**Figure 5** sets forth data for a derivation example with a confirmed complete TMA response.
**Figure 6** is a Venn diagram showing the key efficacy data relating to the primary complete TMA response during the primary evaluation period.
**Figure 7** is a graph depicting the time to complete TMA response.
**Figure 8** is a graph depicting the mean serum concentration (µg/mL) versus time (linear scale). Weight based dosing resulted in maximal, steady state and trough exposures as predicted with no unexpected pharmaokinetic findings.
**Figure 9** is a series of bar graphs depicting the key efficacy data relating to the primary Complete TMA Response during the primary evaluation period and through the data-cut. 95% confidence intervals are represented by the lines at the top of each bar.
**Figure 10** shows the complete TMA response overall and by subgroups during the initial 26-week evaluation period.
**Figure 11** depicts the key efficacy results for complete TMA response status over time (open circle), including platelet count normalization (open triangle), hematologic normalization (+), 25% improvement in serum creatinine from baseline (open square), and LDH normalization (X).
**Figure 12** shows the mean eGFR change (mL/min/1.73 m2) from baseline and 95% confidence interval over time.
**Figure 13** shows the chronic kidney disease (CKD) stage shift from baseline to Day 183.
**Figure 14** shows the observed and model based mean change in platelets (10⁹/L) from baseline and 95% confidence interval over time.
**Figure 15** shows the observed mean platelets (10⁹/L) and 95% confidence interval over time.
**Figure 16** shows the observed and model based mean change in LDH (U/L) from baseline and 95% confidence interval over time.
**Figure 17** shows the observed mean LDH (U/L) and 95% confidence interval over time.
**Figure 18** shows the observed and model based mean change in hemoglobin (HGB) (g/L) from baseline and 95% confidence interval over time.
**Figure 19** shows the observed mean HGB (g/L) and 95% confidence interval over time.
**Figure 20** shows the mean FACIT fatigue change from baseline and 95% confidence interval over time. FACIT score ranges from 0-52, with a higher score indicating less Fatigue.
**Figure 21** shows the mean EQ-5D-3L change from baseline and 95% confidence interval over time.
**Figure 22** depicts serum free complement C5 concentrations (µg/L) over time from Baseline to Day 183.
**Figure 23** compares the study population and results between study ALXN1210-aHUS-311 and the eculizumab adult study (C10-004).

### DETAILED DESCRIPTION

### I. Anti-C5 Antibodies

The anti-C5 antibodies described herein bind to complement component C5 (*e.g.,* human C5) and inhibit the cleavage of C5 into fragments C5a and C5b. As described above, such antibodies also have, for example, improved pharmacokinetic properties relative to other anti-C5 antibodies (*e.g*., eculizumab) used for therapeutic purposes.

The term "antibody" describes polypeptides comprising at least one antibody derived antigen binding site (*e.g.,* VH/VL region or Fv, or CDR). Antibodies include known forms of antibodies. For example, an antibody can be a human antibody, a humanized antibody, a bispecific antibody, or a chimeric antibody. An antibody also can be a Fab, Fab'2, ScFv, SMIP, Affibody^{®}, nanobody, or a domain antibody. An antibody also can be of any of the following isotypes: IgG1, IgG2, IgG3, IgG4, IgM, IgA1, IgA2, IgAsec, IgD, and IgE. An antibody may be a naturally occurring antibody or may be an antibody that has been altered by a protein engineering technique (*e.g.,* by mutation, deletion, substitution, conjugation to a non-antibody moiety). For example, an antibody may include one or more variant amino acids (compared to a naturally occurring antibody) which changes a property (*e.g.,* a functional property) of the antibody. For example, numerous such alterations are known in the art which affect, *e.g.,* half-life, effector function, and/or immune responses to the antibody in a patient. The term antibody also includes artificial or engineered polypeptide constructs which comprise at least one antibody-derived antigen binding site.

Anti-C5 antibodies suitable for use in the invention can be generated using methods well known in the art. Alternatively, art recognized anti-C5 antibodies can be used.

Eculizumab (also known as SOLIRIS^{®}) is an anti-C5 antibody comprising heavy chain CDR1, CDR2 and CDR3 domains having the sequences set forth in SEQ ID NOs: 1, 2, and 3, respectively, and light chain CDR1, CDR2 and CDR3 domains having the sequences set forth in SEQ ID NOs: 4, 5, and 6, respectively. Eculizumab comprises a heavy chain variable region having the amino acid sequence set forth in SEQ ID NO: 7 and a light chain variable region having the amino acid sequence set forth in SEQ ID NO: 8. The variable regions of eculizumab are described in PCT/US1995/005688 and US Patent No.:6,355,245. Eculizumab comprises a heavy chain comprising the amino acid sequence set forth in SEQ ID NO:10 and a light chain having the amino acid sequence set forth in SEQ ID NO:11. The full heavy and light chains of eculizumab are described in PCT/US2007/006606.

The anti-C5 antibody ravulizumab comprises heavy and light chains having the sequences shown in SEQ ID NOs:14 and 11, respectively. Ravulizumab (also known as ULTOMIRIS^{®}, BNJ441 and ALXN1210) is described in PCT/US2015/019225 and US Patent No.: 9,079,949. The terms ravulizumab, BNJ441, and ALXN1210 may be used interchangeably throughout this document, but all refer to the same antibody. Ravulizumab selectively binds to human complement protein C5, inhibiting its cleavage to C5a and C5b during complement activation. This inhibition prevents the release of the proinflammatory mediator C5a and the formation of the cytolytic pore-forming membrane attack complex (MAC) C5b-9 while preserving the proximal or early components of complement activation (*e.g.,* C3 and C3b) essential for the opsonization of microorganisms and clearance of immune complexes.

The VH region of ravulizumab has the sequence set forth in SEQ ID NO:12, and the VL region of ravulizumab has the sequence set forth in SEQ ID NO:8. The heavy chain CDR1, CDR2 and CDR3 domains have the sequences set forth in SEQ ID NOs:19, 18, and 3, respectively, and light chain CDR1, CDR2 and CDR3 domains have the sequences set forth in SEQ ID NOs:4, 5, and 6, respectively.

Antibody BNJ421 is an anti-C5 antibody comprising heavy and light chains having the sequences shown in SEQ ID NOs:20 and 11, respectively, or antigen binding fragments and variants thereof. BNJ421 (also known as ALXN1211) is described in PCT/US2015/019225 and US Patent No.9,079,949.

The VH region of BNJ421 has the sequence set forth in SEQ ID NO: 12, and the VL region of BNJ421 has the sequence set forth in SEQ ID NO:8. The heavy chain CDR1, CDR2 and CDR3 domains have the sequences set forth in SEQ ID NOs:19, 18, and 3, respectively, and the light chain CDR1, CDR2 and CDR3 domains have the sequences set forth in SEQ ID NOs:4, 5, and 6, respectively.

The exact boundaries of CDRs have been defined differently according to different methods. The positions of the CDRs or framework regions within a light or heavy chain variable domain can be as defined by Kabat et al. [(1991) "Sequences of Proteins of Immunological Interest." NIH Publication No. 91-3242, U.S. Department of Health and Human Services, Bethesda, MD]. In such cases, the CDRs can be referred to as "Kabat CDRs" (*e.g.,* "Kabat LCDR2" or "Kabat HCDR1"). The positions of the CDRs of a light or heavy chain variable region can be as defined by Chothia et al. (1989) Nature 342:877-883. Accordingly, these regions can be referred to as "Chothia CDRs" (*e.g.,* "Chothia LCDR2" or "Chothia HCDR3"). The positions of the CDRs of the light and heavy chain variable regions can be as defined by a Kabat-Chothia combined definition. These regions can be referred to as "combined Kabat-Chothia CDRs". Thomas et al. [(1996) Mol Immunol 33(17/18):1389-1401] exemplifies the identification of CDR boundaries according to Kabat and Chothia definitions.

An anti-C5 antibody described herein comprises a heavy chain CDR1 comprising, or consisting of, the following amino acid sequence: GHIFSNYWIQ (SEQ ID NO: 19). An anti-C5 antibody described herein comprises a heavy chain CDR2 comprising, or consisting of, the following amino acid sequence: EILPGSGHTEYTENFKD (SEQ ID NO:18).

The antibody may bind to human C5 at pH 7.4 and 25°C with an affinity dissociation constant (K_{D}) that is in the range 0.1 nM to 1 nM. The antibody may bind to human C5 at pH 6.0 and 25°C with a K_{D} ≥ 10 nM. The [(K_{D} of the antibody or antigen-binding fragment thereof for human C5 at pH 6.0 and at 25°C)/(K_{D} of the antibody or antigen-binding fragment thereof for human C5 at pH 7.4 and at 25°C)] of the antibody may be greater than 25.

The anti-C5 antibody 7086 is described in US Patent Nos. 8,241,628 and 8,883,158. The heavy chain CDR1, CDR2 and CDR3 domains of the 7086 antibody have the sequences set forth in SEQ ID NOs: 21, 22, and 23, respectively, and the light chain CDR1, CDR2 and CDR3 domains of the 7086 antibody have the sequences set forth in SEQ ID NOs: 24, 25, and 26, respectively. The VH region of the 7086 antibody has the sequence set forth in SEQ ID NO:27, and the VL region of the 7086 antibody has the sequence set forth in SEQ ID NO:28.

The anti-C5 antibody 8110 antibody is described in US Patent Nos. 8,241,628 and 8,883,158. The heavy chain CDR1, CDR2 and CDR3 domains of the 8110 antibody have the sequences set forth in SEQ ID NOs: 29, 30, and 31, respectively, and the light chain CDR1, CDR2 and CDR3 domains of the 8110 antibody have the sequences set forth in SEQ ID NOs: 32, 33, and 34, respectively. The VH region of the 8110 antibody has the sequence set forth in SEQ ID NO: 35, and the VL region of the 8110 antibody has the sequence set forth in SEQ ID NO: 36.

The anti-C5 antibody 305LO5 antibody is in US2016/0176954A1. The heavy chain CDR1, CDR2 and CDR3 domains of the 305LO5 antibody have the sequences set forth in SEQ ID NOs: 37, 38, and 39, respectively, and the light chain CDR1, CDR2 and CDR3 domains of the 305LO5 antibody have the sequences set forth in SEQ ID NOs: 40, 41, and 42, respectively. The VH region of the 305LO5 antibody has the sequence set forth in SEQ ID NO: 43, and the VL region of the 305LO5 antibody has the sequence set forth in SEQ ID NO: 44.

The anti-C5 antibody SKY59 antibody is described in Fukuzawa T., et al., Rep. 2017 Apr 24;7(1):1080). The SKY59 antibody comprises a heavy chain comprising SEQ ID NO: 45 and a light chain comprising SEQ ID NO: 46.

The anti-C5 antibody REGN3918 antibody (also known as H4H12166PP) is described in US20170355757. The REGN3918 antibody comprises a heavy chain variable region comprising SEQ ID NO:47 and a light chain variable region comprising SEQ ID NO:48. The REGN3918 antibody comprises a heavy chain comprising SEQ ID NO:49 and a light chain comprising SEQ ID NO:50.

An antibody may compete for binding with, and/or binds to the same epitope on C5 as, the above-mentioned antibodies (*e.g.,* eculizumab, ravulizumab, 7086 antibody, 8110 antibody, 305LO5 antibody, SKY59 antibody, or REGN3918 antibody). An antibody may have at least about 90% variable region amino acid sequence identity with the above-mentioned antibodies (*e.g.,* at least about 90%, 91%, 92%, 93%, 94%, 95%, 96%, 97%, 98% or 99% variable region identity).

An anti-C5 antibody described herein can comprise a variant human Fc constant region that binds to human neonatal Fc receptor (FcRn) with greater affinity than that of the native human Fc constant region from which the variant human Fc constant region was derived. For example, the Fc constant region can comprise one or more (*e.g.,* two, three, four, five, six, seven, or eight or more) amino acid substitutions relative to the native human Fc constant region from which the variant human Fc constant region was derived. The substitutions can increase the binding affinity of an IgG antibody containing the variant Fc constant region to FcRn at pH 6.0, while maintaining the pH dependence of the interaction. Methods for testing whether one or more substitutions in the Fc constant region of an antibody increase the affinity of the Fc constant region for FcRn at pH 6.0 (while maintaining pH dependence of the interaction) are known in the art and exemplified in the working examples. See, e.g., PCT/US2015/019225 and US Patent No. 9,079,949.

Substitutions that enhance the binding affinity of an antibody Fc constant region for FcRn are known in the art and include, *e.g.,* (1) the M252Y/S254T/T256E triple substitution described by Dall'Acqua et al. (2006) J Biol Chem 281: 23514-23524; (2) the M428L or T250Q/M428L substitutions described in Hinton et al. (2004) J Biol Chem 279:6213-6216 and Hinton et al. (2006) J Immunol 176:346-356; and (3) the N434A or T307/E380A/N434A substitutions described in Petkova et al. (2006) Int Immunol 18(12):1759-69. The additional substitution pairings: P257VQ3111, P257I/N434H, and D376V/N434H are described in, *e.g.,* Datta-Mannan et al. (2007) J Biol Chem 282(3): 1709-1717.

The variant constant region may have a substitution at EU amino acid residue 255 for valine. The variant constant region may have a substitution at EU amino acid residue 309 for asparagine. The variant constant region may have a substitution at EU amino acid residue 312 for isoleucine. The variant constant region may have a substitution at EU amino acid residue 386.

A variant Fc constant region may comprise no more than 30 (*e.g.,* no more than 29, 28, 27, 26, 25, 24, 23, 22, 21, 20, 19, 18, 17, 16, 15, 14, 13, 12, 11, 10, nine, eight, seven, six, five, four, three, or two) amino acid substitutions, insertions, or deletions relative to the native constant region from which it was derived. A variant Fc constant region may comprise one or more amino acid substitutions selected from the group consisting of: M252Y, S254T, T256E, N434S, M428L, V259I, T250I, and V308F. A variant human Fc constant region may comprise a methionine at position 428 and an asparagine at position 434, each in EU numbering. A variant Fc constant region may comprise a 428L/434S double substitution as described in, *e.g.,* U.S. Patent No. 8.088,376.

The precise location of these mutations may be shifted from the native human Fc constant region position due to antibody engineering. For example, the 428L/434S double substitution when used in a IgG2/4 chimeric Fc may correspond to 429L and 435S as in the M429L and N435S variants found in BNJ441 (ravulizumab) and described in US Patent Number 9,079,949.

A variant constant region may comprise a substitution at amino acid position 237, 238, 239, 248, 250, 252, 254, 255, 256, 257, 258, 265, 270, 286, 289, 297, 298, 303, 305, 307, 308, 309, 311, 312, 314, 315, 317, 325, 332, 334, 360, 376, 380, 382, 384, 385, 386, 387, 389, 424, 428, 433, 434, or 436 (EU numbering) relative to the native human Fc constant region. The substitution may be selected from the group consisting of: methionine for glycine at position 237; alanine for proline at position 238; lysine for serine at position 239; isoleucine for lysine at position 248; alanine, phenylalanine, isoleucine, methionine, glutamine, serine, valine, tryptophan, or tyrosine for threonine at position 250; phenylalanine, tryptophan, or tyrosine for methionine at position 252; threonine for serine at position 254; glutamic acid for arginine at position 255; aspartic acid, glutamic acid, or glutamine for threonine at position 256; alanine, glycine, isoleucine, leucine, methionine, asparagine, serine, threonine, or valine for proline at position 257; histidine for glutamic acid at position 258; alanine for aspartic acid at position 265; phenylalanine for aspartic acid at position 270; alanine, or glutamic acid for asparagine at position 286; histidine for threonine at position 289; alanine for asparagine at position 297; glycine for serine at position 298; alanine for valine at position 303; alanine for valine at position 305; alanine, aspartic acid, phenylalanine, glycine, histidine, isoleucine, lysine, leucine, methionine, asparagine, proline, glutamine, arginine, serine, valine, tryptophan, or tyrosine for threonine at position 307; alanine, phenylalanine, isoleucine, leucine, methionine, proline, glutamine, or threonine for valine at position 308; alanine, aspartic acid, glutamic acid, proline, or arginine for leucine or valine at position 309; alanine, histidine, or isoleucine for glutamine at position 311; alanine or histidine for aspartic acid at position 312;lysine or arginine for leucine at position 314; alanine or histidine for asparagine at position 315; alanine for lysine at position 317; glycine for asparagine at position 325; valine for isoleucine at position 332; leucine for lysine at position 334; histidine for lysine at position 360; alanine for aspartic acid at position 376; alanine for glutamic acid at position 380; alanine for glutamic acid at position 382; alanine for asparagine or serine at position 384; aspartic acid or histidine for glycine at position 385; proline for glutamine at position 386; glutamic acid for proline at position 387; alanine or serine for asparagine at position 389; alanine for serine at position 424; alanine, aspartic acid, phenylalanine, glycine, histidine, isoleucine, lysine, leucine, asparagine, proline, glutamine, serine, threonine, valine, tryptophan, or tyrosine for methionine at position 428; lysine for histidine at position 433; alanine, phenylalanine, histidine, serine, tryptophan, or tyrosine for asparagine at position 434; and histidine for tyrosine or phenylalanine at position 436, all in EU numbering.

Suitable anti-C5 antibodies for use in the methods described herein comprise a heavy chain polypeptide comprising the amino acid sequence depicted in SEQ ID NO: 14 and/or a light chain polypeptide comprising the amino acid sequence depicted in SEQ ID NO: 11.

The antibody may bind to C5 at pH 7.4 and 25°C (and, otherwise, under physiologic conditions) with an affinity dissociation constant (K_{D}) that is at least 0.1 (*e.g.,* at least 0.15, 0.175, 0.2, 0.25, 0.275, 0.3, 0.325, 0.35, 0.375, 0.4, 0.425, 0.45, 0.475, 0.5, 0.525, 0.55, 0.575, 0.6, 0.625, 0.65, 0.675, 0.7, 0.725, 0.75, 0.775, 0.8, 0.825, 0.85, 0.875, 0.9, 0.925, 0.95, or 0.975) nM. The K_{D} of the anti-C5 antibody may be no greater than 1 (*e.g.,* no greater than 0.9, 0.8, 0.7, 0.6, 0.5, 0.4, 0.3, or 0.2) nM.

The [(K_{D} of the antibody for C5 at pH 6.0 at C)/(K_{D} of the antibody for C5 at pH 7.4 at 25°C)] may be greater than 21 (*e.g.,* greater than 22, 23, 24, 25, 26, 27, 28, 29, 30, 35, 40, 45, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290, 300, 350, 400, 450, 500, 600, 700, 800, 900, 1000, 1500, 2000, 2500, 3000, 3500, 4000, 4500, 5000, 5500, 6000, 6500, 7000, 7500, or 8000).

Methods for determining whether an antibody binds to a protein antigen and/or the affinity for an antibody to a protein antigen are known in the art. For example, the binding of an antibody to a protein antigen can be detected and/or quantified using a variety of techniques such as, but not limited to, Western blot, dot blot, surface plasmon resonance (SPR) method (*e.g.,* BIAcore system; Pharmacia Biosensor AB, Uppsala, Sweden and Piscataway, N.J.), or enzyme-linked immunosorbent assay (ELISA). See, *e.g.,* Benny K. C. Lo (2004) "Antibody Engineering: Methods and Protocols," Humana Press (ISBN: 1588290921); Johne et al. (1993) J Immunol Meth 160:191-198; Jonsson et al. (1993) Ann Biol Clin 51:19-26; and Jonsson et al. (1991) Biotechniques 11:620-627. In addition, methods for measuring the affinity (*e.g.,* dissociation and association constants) are set forth in the working examples.

As used herein, the term "kₐ" refers to the rate constant for association of an antibody to an antigen. The term "k_{d}" refers to the rate constant for dissociation of an antibody from the antibody/antigen complex. And the term "K_{D}" refers to the equilibrium dissociation constant of an antibody-antigen interaction. The equilibrium dissociation constant is deduced from the ratio of the kinetic rate constants, K_{D} = kₐ/k_{d}. Such determinations preferably are measured at 25° C or 37°C (see the working examples). For example, the kinetics of antibody binding to human C5 can be determined at pH 8.0, 7.4, 7.0, 6.5 and 6.0 via surface plasmon resonance (SPR) on a BIAcore 3000 instrument using an anti-Fc capture method to immobilize the antibody.

An anti-C5 antibody may block the generation or activity of the C5a and/or C5b active fragments of a C5 protein *(e.g.,* a human C5 protein). Through this blocking effect, the antibodies inhibit, *e.g.,* the pro-inflammatory effects of C5a and the generation of the C5b-9 membrane attack complex (MAC) at the surface of a cell.

Methods for determining whether a particular antibody described herein inhibits C5 cleavage are known in the art. Inhibition of human complement component C5 can reduce the cell-lysing ability of complement in a subject's body fluids. Such reductions of the cell-lysing ability of complement present in the body fluid(s) can be measured by methods well known in the art such as, for example, by a conventional hemolytic assay such as the hemolysis assay described by Kabat and Mayer (eds.), "Experimental Immunochemistry, 2nd Edition," 135-240, Springfield, IL, CC Thomas (1961), pages 135-139, or a conventional variation of that assay such as the chicken erythrocyte hemolysis method as described in, *e.g.,* Hillmen et al. (2004) N Engl J Med 350(6):552. Methods for determining whether a candidate compound inhibits the cleavage of human C5 into forms C5a and C5b are known in the art and described in Evans et al. (1995) Mol Immunol 32(16):1183-95. For example, the concentration and/or physiologic activity of C5a and C5b in a body fluid can be measured by methods well known in the art. For C5b, hemolytic assays or assays for soluble C5b-9 as discussed herein can be used. Other assays known in the art can also be used. Using assays of these or other suitable types, candidate agents capable of inhibiting human complement component C5 can be screened.

Immunological techniques such as, but not limited to, ELISA can be used to measure the protein concentration of C5 and/or its split products to determine the ability of an anti-C5 antibody to inhibit conversion of C5 into biologically active products. C5a generation may be measured. C5b-9 neoepitope-specific antibodies may be used to detect the formation of terminal complement.

Hemolytic assays can be used to determine the inhibitory activity of an anti-C5 antibody on complement activation. In order to determine the effect of an anti-C5 antibody on classical complement pathway-mediated hemolysis in a serum test solution *in vitro,* for example, sheep erythrocytes coated with hemolysin or chicken erythrocytes sensitized with anti-chicken erythrocyte antibody are used as target cells. The percentage of lysis is normalized by considering 100% lysis equal to the lysis occurring in the absence of the inhibitor. The classical complement pathway may be activated by a human IgM antibody, for example, as utilized in the Wieslab^{®} Classical Pathway Complement Kit (Wieslab^{®} COMPL CP310, Euro-Diagnostica, Sweden). Briefly, the test serum is incubated with an anti-C5 antibody in the presence of a human IgM antibody. The amount of C5b-9 that is generated is measured by contacting the mixture with an enzyme conjugated anti-C5b-9 antibody and a fluorogenic substrate and measuring the absorbance at the appropriate wavelength. As a control, the test serum is incubated in the absence of the anti-C5 antibody, or antigen binding fragment thereof. The test serum may be a C5-deficient serum reconstituted with a C5 polypeptide.

To determine the effect of an anti-C5 antibody on alternative pathway-mediated hemolysis, unsensitized rabbit or guinea pig erythrocytes can be used as the target cells. The serum test solution may be a C5-deficient serum reconstituted with a C5 polypeptide. The percentage of lysis is normalized by considering 100% lysis equal to the lysis occurring in the absence of the inhibitor. The alternative complement pathway may be activated by lipopolysaccharide molecules, for example, as utilized in the Wieslab^{®} Alternative Pathway Complement Kit (Wieslab^{®} COMPL AP330, Euro-Diagnostica, Sweden). Briefly, the test serum is incubated with an anti-C5 antibody in the presence of lipopolysaccharide. The amount of C5b-9 that is generated is measured by contacting the mixture with an enzyme conjugated anti-C5b-9 antibody and a fluorogenic substrate and measuring the fluorescence at the appropriate wavelength. As a control, the test serum is incubated in the absence of the anti-C5 antibody, or antigen binding fragment thereof.

C5 activity, or inhibition thereof, may be quantified using a CH50eq assay. The CH50eq assay is a method for measuring the total classical complement activity in serum. This test is a lytic assay, which uses antibody-sensitized erythrocytes as the activator of the classical complement pathway and various dilutions of the test serum to determine the amount required to give 50% lysis (CH50). The percent hemolysis can be determined, for example, using a spectrophotometer. The CH50eq assay provides an indirect measure of terminal complement complex (TCC) formation, since the TCC themselves are directly responsible for the hemolysis that is measured.

The assay is well known and commonly practiced by those of skill in the art. Briefly, to activate the classical complement pathway, undiluted serum samples (*e.g.,* reconstituted human serum samples) are added to microassay wells containing the antibody-sensitized erythrocytes to thereby generate TCC. Next, the activated sera are diluted in microassay wells, which are coated with a capture reagent (*e.g.,* an antibody that binds to one or more components of the TCC). The TCC present in the activated samples bind to the monoclonal antibodies coating the surface of the microassay wells. The wells are washed and to each well is added a detection reagent that is detectably labeled and recognizes the bound TCC. The detectable label can be, *e.g.,* a fluorescent label or an enzymatic label. The assay results are expressed in CH50 unit equivalents per milliliter (CH50 U Eq/mL).

Inhibition, e.g., as it pertains to terminal complement activity, includes at least a 5 (*e.g.,* at least a 6, 7, 8, 9, 10, 15, 20, 25, 30, 35, 40, 45, 50, 55, or 60) % decrease in the activity of terminal complement in, *e.g.,* a hemolytic assay or CH50eq assay as compared to the effect of a control antibody (or antigen-binding fragment thereof) under similar conditions and at an equimolar concentration. Substantial inhibition, as used herein, refers to inhibition of a given activity (*e.g.,* terminal complement activity) of at least 40 (*e.g.,* at least 45, 50, 55, 60, 65, 70, 75, 80, 85, 90, or 95 or greater) %. An anti-C5 antibody described herein may contain one or more amino acid substitutions relative to the CDRs of eculizumab (i.e., SEQ ID NOs: 1-6), yet retains at least 30 (*e.g.,* at least 31, 32, 33, 34, 35, 36, 37, 38, 39, 40, 41, 42, 43, 44, 45, 46, 47, 48, 49, 50, 55, 60, 65, 70, 75, 80, 85, 90, or 95) % of the complement inhibitory activity of eculizumab in a hemolytic assay or CH50eq assay.

An anti-C5 antibody described herein has a serum half-life in humans that is at least 20 (*e.g.,* at least 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, or 55) days. The anti-C5 antibody described herein may have a serum half-life in humans that is at least 40 days. The anti-C5 antibody described herein may have a serum half-life in humans that is approximately 43 days. The anti-C5 antibody described herein may have a serum half-life in humans that is between 39-48 days. Methods for measuring the serum half-life of an antibody are known in the art. An anti-C5 antibody described herein may have a serum half-life that is at least 20 (*e.g.,* at least 30, 35, 40, 45, 50, 55, 60, 65, 70, 75, 80, 85, 90, 95, 100, 125, 150, 175, 200, 250, 300, 400, 500) % greater than the serum half-life of eculizumab, *e.g.,* as measured in one of the mouse model systems described in the working examples (*e.g.,* the C5-deficient/NOD/scid mouse or hFcRn transgenic mouse model system).

The antibody may compete for binding with, and/or binds to the same epitope on C5 as, the antibodies described herein. The term "binds to the same epitope" with reference to two or more antibodies means that the antibodies bind to the same segment of amino acid residues, as determined by a given method. Techniques for determining whether antibodies bind to the "same epitope on C5" with the antibodies described herein include, for example, epitope mapping methods, such as, x-ray analyses of crystals of antigen: antibody complexes which provides atomic resolution of the epitope and hydrogen/deuterium exchange mass spectrometry (HDX-MS). Other methods monitor the binding of the antibody to peptide antigen fragments or mutated variations of the antigen where loss of binding due to a modification of an amino acid residue within the antigen sequence is often considered an indication of an epitope component. In addition, computational combinatorial methods for epitope mapping can also be used. These methods rely on the ability of the antibody of interest to affinity isolate specific short peptides from combinatorial phage display peptide libraries. Antibodies having the same VH and VL or the same CDR1, 2 and 3 sequences are expected to bind to the same epitope.

Antibodies that "compete with another antibody for binding to a target" refer to antibodies that inhibit (partially or completely) the binding of the other antibody to the target. Whether two antibodies compete with each other for binding to a target, *i.e.,* whether and to what extent one antibody inhibits the binding of the other antibody to a target, may be determined using known competition experiments. An antibody may compete with, and may inhibit binding of another antibody to a target by at least 10%, 20%, 30%, 40%, 50%, 60%, 70%, 80%, 90% or 100%. The level of inhibition or competition may be different depending on which antibody is the "blocking antibody" (i.e., the cold antibody that is incubated first with the target). Competing antibodies bind to the same epitope, an overlapping epitope or to adjacent epitopes (*e.g.,* as evidenced by steric hindrance).

Anti-C5 antibodies, or antigen-binding fragments thereof described herein, used in the methods described herein can be generated using a variety of art-recognized techniques. Monoclonal antibodies may be obtained by various techniques familiar to those skilled in the art. Briefly, spleen cells from an animal immunized with a desired antigen are immortalized, commonly by fusion with a myeloma cell (see, Kohler & Milstein, Eur. J. Immunol. 6: 511-519 (1976)). Alternative methods of immortalization include transformation with Epstein Barr Virus, oncogenes, or retroviruses, or other methods well known in the art. Colonies arising from single immortalized cells are screened for production of antibodies of the desired specificity and affinity for the antigen, and yield of the monoclonal antibodies produced by such cells may be enhanced by various techniques, including injection into the peritoneal cavity of a vertebrate host. Alternatively, one may isolate DNA sequences which encode a monoclonal antibody or a binding fragment thereof by screening a DNA library from human B cells according to the general protocol outlined by Huse, et al., Science 246: 1275-1281 (1989).

### II. Compositions

Also, described herein are compositions comprising the anti-C5 antibody ravulizumab. The anti-C5 antibody comprises the CDR1, CDR2 and CDR3 domains in a heavy chain variable region having the sequence set forth in SEQ ID NO:12, and the CDR1, CDR2 and CDR3 domains in a light chain variable region having the sequence set forth in SEQ ID NO:8. The anti-C5 antibody comprises heavy and light chains having the sequences shown in SEQ ID NOs:14 and 11, respectively.

The compositions can be formulated as a pharmaceutical solution, *e.g.,* for administration to a subject for the treatment or prevention of a complement-associated disorder, such as aHUS. The pharmaceutical compositions will generally include a pharmaceutically acceptable carrier. As used herein, a "pharmaceutically acceptable carrier" refers to, and includes, any and all solvents, dispersion media, coatings, antibacterial and antifungal agents, isotonic and absorption delaying agents, and the like that are physiologically compatible. The compositions can include a pharmaceutically acceptable salt, *e.g.,* an acid addition salt or a base addition salt, sugars, carbohydrates, polyols and/or tonicity modifiers.

The compositions can be formulated according to standard methods. Pharmaceutical formulation is a well-established art, and is further described in, *e.g.,* Gennaro (2000) "Remington: The Science and Practice of Pharmacy," 20th Edition, Lippincott, Williams & Wilkins (ISBN: 0683306472); Ansel et al. (1999) "Pharmaceutical Dosage Forms and Drug Delivery Systems," 7th Edition, Lippincott Williams & Wilkins Publishers (ISBN: 0683305727); and Kibbe (2000) "Handbook of Pharmaceutical Excipients American Pharmaceutical Association," 3rd Edition (ISBN: 091733096X). A composition can be formulated, for example, as a buffered solution at a suitable concentration and suitable for storage at 2-8°C (*e.g.,* 4°C). A composition can be formulated for storage at a temperature below 0°C (e.g., -20°C or -80°C). The composition can be formulated for storage for up to 2 years (*e.g.,* one month, two months, three months, four months, five months, six months, seven months, eight months, nine months, 10 months, 11 months, 1 year, 1½ years, or 2 years) at 2-8°C (*e.g.,* 4°C). Thus, the compositions described herein are stable in storage for at least 1 year at 2-8°C (*e.g.,* 4°C).

The pharmaceutical compositions can be in a variety of forms. These forms include, *e.g.,* liquid, semi-solid and solid dosage forms, such as liquid solutions (*e.g.,* injectable and infusible solutions), dispersions or suspensions, tablets, pills, powders, liposomes and suppositories. The preferred form depends, in part, on the intended mode of administration and therapeutic application. For example, compositions containing a composition intended for systemic or local delivery can be in the form of injectable or infusible solutions. Accordingly, the compositions can be formulated for administration by a parenteral mode (*e.g.,* intravenous, subcutaneous, intraperitoneal, or intramuscular injection). "Parenteral administration," "administered parenterally," and other grammatically equivalent phrases, as used herein, refer to modes of administration other than enteral and topical administration, usually by injection, and include, without limitation, intravenous, intranasal, intraocular, pulmonary, intramuscular, intraarterial, intrathecal, intracapsular, intraorbital, intracardiac, intradermal, intrapulmonary, intraperitoneal, transtracheal, subcutaneous, subcuticular, intraarticular, subcapsular, subarachnoid, intraspinal, epidural, intracerebral, intracranial, intracarotid and intrasternal injection and infusion.

The composition comprises ravulizumab (also known as ULTOMIRIS^{®}, antibody BNJ441, or ALXN1210) for injection. The injection may be a sterile, clear to translucent, slight whitish color, preservative-free solution for intravenous use. Each single-dose vial may contain 300 mg ravulizumab for injection at a concentration of 10 mg/mL with a pH of 7.0. Ravulizumab for injection may require dilution to a final concentration of 5 mg/mL. Each mL may further comprise polysorbate 80 (0.2 mg) (vegetable origin), sodium chloride (8.77 mg), sodium phosphate dibasic (1.78 mg), sodium phosphate monobasic (0.46 mg), and Water for Injection.

### III. Methods of Treatment

Provided herein is an anti-C5 antibody for use in methods for treating aHUS in a human adult patient who is 18 years or older, wherein the anti-C5 antibody is ravulizumab, wherein the anti-C5 antibody is administered intravenously: (a) once on Day 1 at a dose of: 2400 mg to a patient weighing ≥ 40 to < 60 kg, 2700 mg to a patient weighing ≥ 60 to < 100 kg, or 3000 mg to a patient weighing ≥ 100 kg; and (b) on Day 15 and every eight weeks thereafter at a dose of 3000 mg to a patient weighing ≥ 40 to < 60 kg, 3300 mg to a patient weighing ≥ 60 to < 100 kg, or 3600 mg to a patient weighing ≥ 100 kg.

As used herein, the terms "induction" and "induction phase" are used interchangeably and refer to the first phase of treatment in the clinical trial.

As used herein, the terms "maintenance" and "maintenance phase" are used interchangeably and refer to the second phase of treatment in the clinical trial. Treatment may be continued as long as clinical benefit is observed or until unmanageable toxicity or disease progression occurs.

As used herein, the term "subject" or "patient" is a human patient (*e.g.,* a patient having complement-associated condition). The complement-associated condition is atypical hemolytic uremic syndrome (aHUS). The pathology and clinical presentations of patients with aHUS are also driven by terminal complement activation. More specifically, activation of C5 and dysregulation of complement activation lead to endothelial damage, platelet consumption, and thrombotic microangiopathic (TMA) events, characterized by thrombocytopenia, mechanical intravascular hemolysis, and kidney injury. Importantly, approximately 20% of patients experience extra-renal manifestations of disease as well, including central nervous system, cardiac, gastrointestinal, distal extremities, and severe systemic organ involvement (Loirat, et al., Orphanet. J. Rare Dis. 2011;6:60). Symptoms of aHUS are well-known to those of skill in the art of rare disease or kidney disease medicine and include, *e.g.,* severe hypertension, proteinuria, uremia, lethargy/fatigue, irritability, thrombocytopenia, microangiopathic hemolytic anemia, and renal function impairment (*e.g.,* acute renal failure).

aHUS can be genetic, acquired, or idiopathic. aHUS can be considered genetic when two or more (e.g., three, four, five, or six or more) members of the same family are affected by the disease at least six months apart and exposure to a common triggering agent has been excluded, or when one or more aHUS-associated gene mutations (e.g., one or more mutations in CFH, MCP/CD46, CFB, or CFI) are identified in a subject. For example, a subject can have CFH-associated aHUS, CFB-associated aHUS, CFI-associated aHUS, or MCP-associated aHUS. Up to 30% of genetic aHUS is associated with mutations in CFH, 12% with mutations in MCP, 5-10% with mutations in CFI, and less than 2% with mutations in CFB. Genetic aHUS can be multiplex (i.e., familial; two or more affected family members) or simplex (i.e., a single occurrence in a family). aHUS can be considered acquired when an underlying environmental factor (*e.g.,* a drug, systemic disease, or viral or bacterial agents that do not result in Shiga-like exotoxins) or trigger can be identified. aHUS can be considered idiopathic when no trigger (genetic or environmental) is evident.

Laboratory tests can be performed to determine whether a human subject has thrombocytopenia, microangiopathic hemolytic anemia, or acute renal insufficiency. Thrombocytopenia can be diagnosed by a medical professional as one or more of: (i) a platelet count that is less than 150,000/mm³ (e.g., less than 60,000/mm³); (ii) a reduction in platelet survival time that is reduced, reflecting enhanced platelet disruption in the circulation; and (iii) giant platelets observed in a peripheral smear, which is consistent with secondary activation of thrombocytopoiesis. Microangiopathic hemolytic anemia can be diagnosed by a medical professional as one or more of: (i) hemoglobin concentrations that are less than 10 mg/dL (e.g., less than 6.5 mg/dL); (ii) increased serum lactate dehydrogenase (LDH) concentrations (>460 U/L); (iii) hyperbilirubinemia, reticulocytosis, circulating free hemoglobin, and low or undetectable haptoglobin concentrations; and (iv) the detection of fragmented red blood cells (schistocytes) with the typical aspect of burr or helmet cells in the peripheral smear together with a negative Coombs test. See, e.g., Kaplan et al. (1992) "Hemolytic Uremic Syndrome and Thrombotic Thrombocytopenic Purpura," Informa Health Care (ISBN 0824786637) and Zipfel (2005) "Complement and Kidney Disease," Springer (ISBN 3764371668). Blood concentrations of C3 and C4 can also be used as a measure of complement activation or dysregulation. In addition, a subject's condition can be further characterized by identifying the subject as harboring one or more mutations in a gene associated with aHUS such as CFI, CFB, CFH, or MCP (*supra*)*.* Suitable methods for detecting a mutation in a gene include, e.g., DNA sequencing and nucleic acid array techniques. See, e.g., Breslin et al. (2006) Clin Am Soc Nephrol 1:88-99 and Goicoechea de Jorge et al. (2007) Proc Natl Acad Sci USA 104:240-245.

As used herein, "effective treatment" refers to treatment producing a beneficial effect, *e.g.,* amelioration of at least one symptom of a disease or disorder. A beneficial effect can take the form of an improvement over baseline, *i.e.,* an improvement over a measurement or observation made prior to initiation of therapy according to the method. In the context of aHUS, for example, effective treatment may refer to the alleviation of one or more symptoms selected from the group consisting of severe hypertension, proteinuria, uremia, lethargy/fatigue, irritability, thrombocytopenia, microangiopathic hemolytic anemia, and/or renal function impairment (*e.g.,* acute renal failure)).

The term "effective amount" refers to an amount of an agent that provides the desired biological, therapeutic, and/or prophylactic result. That result can be reduction, amelioration, palliation, lessening, delaying, and/or alleviation of one or more of the signs, symptoms, or causes of a disease, or any other desired alteration of a biological system. In one example, an "effective amount" is the amount of anti-C5 antibody clinically proven to alleviate at least one symptom of aHUS (*e.g.,* severe hypertension, proteinuria, uremia, lethargy/fatigue, irritability, thrombocytopenia, microangiopathic hemolytic anemia, and renal function impairment (*e.g.,* acute renal failure)). An effective amount can be administered in one or more administrations.

The dose of the anti-C5 antibody is based on the weight of the patient. 2400 mg or 3000 mg of the anti-C5 antibody may be administered to a patient weighing ≥ 40 to < 60 kg. 2700 mg or 3300 mg of the anti-C5 antibody may administered to a patient weighing ≥ 60 to < 100 kg. 3000 mg or 3600 mg of the anti-C5 antibody may administered to a patient weighing ≥ 100 kg. dosage regimens may adjusted to provide the optimum desired response (*e.g.,* an effective response).

The anti-C5 antibody may be administered for one or more administration cycles. The administration cycle may be 26 weeks. The anti-C5 antibody may be administered once on Day 1 of the administration cycle, once on Day 15 of the administration cycle, and every eight weeks thereafter. The anti-C5 antibody may be administered every eight weeks after the administration cycle for an extension period up to two years (*e.g.,* at a dose of 3000 mg, 3300 mg, or 3600 mg).

In another embodiment, the anti-C5 antibody, or antigen binding fragment thereof, is administered to a patient weighing ≥ 40 to < 60 kg:
(a) once on Day 1 at a dose of 2400 mg; and
(b) on Day 15 and every eight weeks thereafter at a dose of 3000 mg.

In another embodiment, the anti-C5 antibody is administered to a patient weighing ≥ 60 to < 100 kg:
(a) once on Day 1 at a dose of 2700 mg; and
(b) on Day 15 and every eight weeks thereafter at a dose of 3300 mg.

In another embodiment, the anti-C5 antibody is administered to a patient weighing ≥ 100 kg:
(a) once on Day 1 at a dose of 3000 mg; and
(b) on Day 15 and every eight weeks thereafter at a dose of 3600 mg.

The patient may not have previously been treated with a complement inhibitor (*e.g.,* the patient is a complement inhibitor treatment-naive patient).

The patient may have previously been treated with one anti-C5 antibody, or antigen binding fragment thereof, and is switched to ravulizumab during the course of treatment. For example, different anti-C5 antibodies may be administered during the course of treatment. Different anti-C5 antibodies may administered during separate treatment and extension periods. For example, the patient may be treated with eculizumab during a treatment period (*e.g.,* for 26 weeks), followed by treatment with ravulizumab, for example, during an extension period. Eculizumab may be administered to the patient at a dose of 600 mg on Days 1, 8, 15, and 22 of the administration cycle during an induction phase, followed by a maintenance dose of 900 mg of eculizumab on Day 19 of the administration cycle and every two weeks thereafter (*e.g.,* for a total of 26 weeks), followed by treatment with ravulizumab for an extension period of up to two years. The patient may be treated with ravulizumab (*e.g.,* for 26 weeks), followed by treatment with another anti-C5 antibody (*e.g.,* eculizumab, 7086 antibody, 8110 antibody, 305LO5 antibody, SKY59 antibody, or REGN3918 antibody) during, for example, an extension period.

The patient may have previously been treated for at least 1 month, at least 2 months, at least 3 months, at least 4 months, at least 5 months, at least 6 months, at least 7 months, at least 8 months, at least 9 months, at least 10 months, at least 11 months, at least 12 months, at least 18 months, or at least 24 months with an anti-C5 antibody, or antigen binding fragment thereof, (*e.g.,* eculizumab) before switching to ravulizumab. In a particular embodiment, the patient has previously been treated for at least 6 months with eculizumab.

Where the patient is treated with a first anti-C5 antibody and then switched to treatment with a second different anti-C5 antibody, especially where the second different anti-C5 antibody binds to a different epitope on C5 than the first anti-C5 antibody, and wherein the first or second anti-C5 antibody is ravulizumab, the administration schedules may take into account the half-life of the first anti-C5 antibody. For example, to ensure that the first anti-C5 antibody is cleared (*e.g.,* "washed out") from the patient before the second (different) anti-C5 antibody may be administered (*e.g.,* to avoid issues associated with aggregation, immune complex formation, etc.), the half-life of the first anti-C5 antibody is taken into consideration. The second (different) anti-C5 antibody may not be administered until a duration of time corresponding to 2, 2.5, 3, 3.5, 4, 4.5, 5, 5.5, 6, 6.5, 7, or 7.5 times the half-life of the first anti-C5 antibody has passed after the final administration of the first anti-C5 antibody.

In another embodiment, the patient has previously been treated with eculizumab and then is switched to treatment with a second (different) anti-C5 antibody which is ravulizumab. Where eculizumab is the first administered antibody, ravulizumab may not be administered, for example, until at least 36, 45, 54, 63, 72, 81, 90, 99, 108, 117, or 126 days have passed after the final administration of eculizumab.

After treatment with ravulizumab, the patient may be switched to treatment with a different anti-C5 antibody (*e.g.,* eculizumab, 7086 antibody, 8110 antibody, 305LO5 antibody, SKY59 antibody, or REGN3918 antibody). Where ravulizumab is the first administered antibody, the second (different) anti-C5 antibody may not be administered, for example, until at least 100, 125, 150, 175, 200, 225, 250, 275, 300, 325, 375, or 400 days have passed after the final administration of ravulizumab.

Additionally, or alternatively, techniques are used to clear or enhance clearance of the first anti-C5 antibody before switching to treatment with a second (different) anti-C5 antibody. Exemplary techniques include, but are not limited to, plasmapheresis or blood transfusions. An antibody against the first anti-C5 antibody may be administered to clear or enhance clearance of the first anti-C5 antibody (*e.g.,* an anti-eculizumab antibody, an anti-ravulizumab antibody, an anti-7086 antibody, an anti-8110 antibody, an anti-305LO5 antibody, an anti-SKY59 antibody, or an anti-REGN3918 antibody) before a second (different) anti-C5 antibody is administered.

The administration cycle relating to administration of ravulizumab may start at least about two weeks, at least about three weeks, at least about four weeks, at least about six weeks, at least about seven weeks, or at least about eight weeks after the patient's last dose of eculizumab. In another embodiment, the treatment (*e.g.,* administration cycle) starts at least two weeks after the patient's last dose of eculizumab.

The patients treated according to the methods described herein may have been vaccinated against meningococcal infections within 3 years prior to, or at the time of, initiating treatment. Patients who received treatment less than 2 weeks after receiving a meningococcal vaccine may also be treated with appropriate prophylactic antibiotics until 2 weeks after vaccination. Patients treated according to the methods described herein may be vaccinated against meningococcal serotypes A, C, Y, W135, and/or B.

As used herein, the term "serum trough level" refers to the lowest level that the agent (*e.g.,* the anti-C5 antibody, or antigen binding fragment thereof) or medicine is present in the serum. In contrast, a "peak serum level", refers to the highest level of the agent in the serum. The "average serum level", refers to the mean level of the agent in the serum over time.

The treatment regimens described are sufficient to maintain particular serum trough concentrations of the anti-C5 antibody, or antigen binding fragment thereof. For example, the treatment maintains a serum trough concentration of the anti-C5 antibody of 50, 55, 60, 65, 70, 75, 80, 85, 90, 95, 100, 105, 110, 115, 120, 125, 130, 135, 140, 145, 150, 155, 160, 165, 170, 175, 180, 185, 190, 200, 205, 210, 215, 220, 225, 230, 240, 245, 250, 255, 260, 265, 270, 280, 290, 300, 305, 310, 315, 320, 325, 330, 335, 340, 345, 350, 355, 360, 365, 370, 375, 380, 385, 390, 395, or 400 µg/ml or greater. In one embodiment, the treatment maintains a serum trough concentration of the anti-C5 antibody of 100 µg/ml or greater. The treatment may maintain a serum trough concentration of the anti-C5 antibody of 150 µg/ml or greater. In another embodiment, the treatment maintains a serum trough concentration of the anti-C5 antibody of 200 µg/ml or greater. The treatment may maintain a serum trough concentration of the anti-C5 antibody of 250 µg/ml or greater, or of 300 µg/ml or greater. The treatment may maintain a serum trough concentration of the anti-C5 antibody of between 100 µg/ml and 200 µg/ml, such as of about 175 µg/ml.

To obtain an effective response, the anti-C5 antibody may be administered to the patient in an amount and with a frequency to maintain at least 50 µg, 55µg, 60 µg, 65 µg, 70 µg, 75 µg, 80 µg, 85 µg, 90 µg, 95 µg, 100 µg, 105 µg, 110 µg, 115 µg, 120 µg, 125 µg, 130 µg, 135 µg, 140 µg, 145 µg, 150 µg, 155 µg, 160 µg, 165 µg, 170 µg, 175 µg, 180 µg, 185 µg, 190 µg, 195 µg, 200 µg, 205 µg, 210 µg, 215 µg, 220 µg, 225 µg, 230 µg, 235 µg, 240 µg, 245 µg, 250 µg, 255 µg, or 260 µg of antibody per milliliter of the patient's blood. The anti-C5 antibody may be administered to the patient in an amount and with a frequency to maintain between 50 µg and 250 µg of antibody per milliliter of the patient's blood. The anti-C5 antibody may be administered to the patient in an amount and with a frequency to maintain between 100 µg and 200 µg of antibody per milliliter of the patient's blood. The anti-C5 antibody may be administered to the patient in an amount and with a frequency to maintain about 175 µg of antibody per milliliter of the patient's blood.

To obtain an effective response, the anti-C5 antibody may be administered to the patient in an amount and with a frequency to maintain a minimum free C5 concentration. For example, the anti-C5 antibody may be administered to the patient in an amount and with a frequency to maintain a free C5 concentration of 0.2 µg/mL, 0.3 µg/mL, 0.4 µg/mL, 0.5 µg/mL or below. In another embodiment, the anti-C5 antibody is administered to the patient in an amount and with a frequency to maintain a free C5 concentration of 0.309 to 0.5 µg/mL or below. The treatment described herein may reduce free C5 concentration by greater than 99% throughout the treatment period. In another embodiment, the treatment reduces free C5 concentration greater than 99.5% throughout the treatment period.

### IV. Outcomes

Symptoms of aHUS include, but are not limited to, severe hypertension, proteinuria, uremia, lethargy/fatigue, irritability, thrombocytopenia, microangiopathic hemolytic anemia, and renal function impairment (*e.g.,* acute renal failure). Patients treated according to the methods disclosed herein preferably experience improvement in at least one sign of aHUS.

In other embodiments, the treatment results in terminal complement inhibition.

In other embodiments, the treatment produces a shift toward normal levels of a hemolysis-related hematologic biomarker selected from the group consisting of free hemoglobin, haptoglobin, reticulocyte count, PNH red blood cell (RBC) clone and D-dimer.

In another embodiment, the treatment produces an increase in hemoglobin stabilization from the patient's pre-treatment baseline. The treatment may result in a ≥ 20 g/L increase in hemoglobin. The treatment may result in avoidance of a ≥ 2 g/dL decrease in hemoglobin level from baseline in the absence of transfusion from baseline to Day 183. 9

In other embodiments, the treatment results in platelet normalization (≥150 × 10 /L). 9 The treatment may result in platelet normalization (≥150 × 10 /L) for at least 28 days (*e.g.,* at least 28 days, 1 month, 2 months, 3 months, 4 months, 5 months, 6 months, 7 months, 8 months, 9 months, 10 months, 11 months, 1 year, or two years).

In other embodiments, the treatment results in LDH normalization (≤246 U/L). The treatment may result in LDH normalization (≤246 U/L) for at least 28 days (*e.g.,* at least 28 days, 1 month, 2 months, 3 months, 4 months, 5 months, 6 months, 7 months, 8 months, 9 months, 10 months, 11 months, 1 year, or two years).

In other embodiments, the treatment results in a ≥25% improvement from baseline in serum creatinine. The treatment may result in a ≥25% improvement from baseline in serum creatinine for at least 28 days (*e.g.,* at least 28 days, 1 month, 2 months, 3 months, 4 months, 5 months, 6 months, 7 months, 8 months, 9 months, 10 months, 11 months, 1 year, or two years).

In other embodiments, the treatment results in a complete TMA response (*i.e.,* platelet 9 normalization (≥150 × 10⁹/L), LDH normalization (≤246 U/L), and a ≥25% improvement from baseline in serum creatinine). The treatment may result in a complete TMA response for at least 28 days (*e.g.,* at least 28 days, 1 month, 2 months, 3 months, 4 months, 5 months, 6 months, 7 months, 8 months, 9 months, 10 months, 11 months, 1 year, or two years).

In other embodiments, the treatment results in a modified complete TMA Response 9 (*i.e.,* platelet normalization (≥150 × 10 /L), LDH normalization (≤246 U/L), and the patient is off dialysis if they were on dialysis at baseline or a ≥25% improvement from baseline in serum creatinine for a patient who was off dialysis at baseline). The treatment may result in a modified complete TMA Response for at least 28 days (*e.g.,* at least 28 days, 1 month, 2 months, 3 months, 4 months, 5 months, 6 months, 7 months, 8 months, 9 months, 10 months, 11 months, 1 year, or two years).

In other embodiments, the treatment produces a reduction in the need for blood transfusions. The treatment may produce a greater than 70% increase in transfusion avoidance. The treatment may result in transfusion avoidance from baseline to Day 183.

In other embodiments, the treatment results in an elimination of breakthrough hemolysis during the treatment period. The treatment may result in a reduction of breakthrough hemolysis compared to pretreatment baseline amount of breakthrough hemolysis.

In other embodiments, the treatment produces a reduction in major adverse vascular events (MAVEs).

In other embodiments, the treatment produces a change from baseline in quality of life as assessed via the Functional Assessment of Chronic Illness Therapy (FACIT)-Fatigue Scale, version 4 and the European Organisation for Research and Treatment of Cancer, Quality of Life Questionnaire-Core 30 Scale. The treatment may produce a change from baseline in quality of life as assessed via the FACIT-Fatigue Scale by one or more (*e.g.,* 1, 2, or 3) points. The treatment may produce a change from baseline in quality of life as assessed via the FACIT-Fatigue Scale by 3 points, 150 days or more (*e.g.,* 150 days, 151 days, 152 days, 153 days, 154 days, 155 days, 156 days, 157 days, 158 days, 159 days, 160 days, 161 days, 162 days, 163 days, 164 days, 165 days, 166 days, 167 days, 168 days, 169 days, 170 days, 171 days, 172 days, 173 days, 174 days, 175 days, 176 days, 177 days, 178 days, 179 days, 180 days, 181 days, 182 days 183 days, 184 days, 185 days, 186 days, 187 days, 188 days, 189 days, 190 days, 191 days, 192 days, 193 days, 194 days, 195 days, 196 days, 197 days, 198 days, 199 days, 200 days, 205 days, 210 days, 215 days, 220 days, or 225 days) after initiating treatment.

Chronic kidney disease (CKD) stage is classified based on the National Kidney Foundation Chronic Kidney Disease Stage. The stages of CKD and corresponding estimated glomerular filtration rate (eGFR) values are as follows: Stage 1: eGFR >=90 (normal), Stage 2: eGFR 60-89, Stage 3A: eGFR 45-59, Stage 3B: eGFR 30-44, Stage 4: eGFR 15-29, and Stage 5: eGFR <15 (including dialysis: End stage). Stage 1 is considered the best category. Stage 5 is considered the worst category. An improvement in eGFR (*e.g.,* > 15) corresponds with an improvement in CKD stage (*e.g.,* a lower CKD stage). Accordingly, in other embodiments, the patient's chronic kidney disease (CKD) improves by one or more stages after initiating treatment. For example, the patient's CKD may improve by one, two, three, four, or five stages). The patient's CKD may improve by one or more stages 150 days or more (*e.g*., 150 days, 151 days, 152 days, 153 days, 154 days, 155 days, 156 days, 157 days, 158 days, 159 days, 160 days, 161 days, 162 days, 163 days, 164 days, 165 days, 166 days, 167 days, 168 days, 169 days, 170 days, 171 days, 172 days, 173 days, 174 days, 175 days, 176 days, 177 days, 178 days, 179 days, 180 days, 181 days, 182 days 183 days, 184 days, 185 days, 186 days, 187 days, 188 days, 189 days, 190 days, 191 days, 192 days, 193 days, 194 days, 195 days, 196 days, 197 days, 198 days, 199 days, 200 days, 205 days, 210 days, 215 days, 220 days, or 225 days) after initiating treatment.

The treatment may result in an increase in eGFR compared to baseline. In other embodiments, the treatment results in a shift towards normal levels of eGFR (*e*.*g*., ≥ 90). The treatment may result in an increase in eGFR compared to baseline and the patient's CKD improves by one or more stages. The treatment may result in a shift towards normal levels of eGFR (*e*.*g*., ≥ 90) compared to baseline and the patient's CKD improves by one or more stages.

In other embodiments, the treatment results in a EQ-5D-3L Time Trade-Off value set for the United States (US TTO) of > 0.94.

### V. Kits and Unit Dosage Forms

Also described herein are kits which include a pharmaceutical composition containing the anti-C5 antibody ravulizumab and a pharmaceutically-acceptable carrier, in a therapeutically effective amount adapted for use in the methods described herein. The kits optionally also can include instructions, *e.g.,* comprising administration schedules, to allow a practitioner (*e.g.,* a physician, nurse, or patient) to administer the composition contained therein to administer the composition to a patient having aHUS. The kit also can include a syringe.

Optionally, the kits include multiple packages of the single-dose pharmaceutical compositions each containing an effective amount of the anti-C5 antibody for a single administration in accordance with the methods described above. Instruments or devices necessary for administering the pharmaceutical composition(s) also may be included in the kits. For instance, a kit may provide one or more pre-filled syringes containing an amount of the anti-C5 antibody, or antigen binding fragment thereof.

Described herein is a kit for treating aHUS in a human adult patient who is 18 years or older, the kit comprising:
(a) a dose of the anti-C5 antibody ravulizumab; and
(b) instructions for using the anti-C5 antibody according to any of the methods described herein.

The kit may comprise a dose of an anti-C5 antibody wherein the anti-C5 antibody is administered to a patient weighing ≥ 40 to < 60 kg:
(a) once on Day 1 at a dose of 2400 mg; and
(b) on Day 15 and every eight weeks thereafter at a dose of 3000 mg.

The kit may comprise a dose of an anti-C5 antibody wherein the anti-C5 antibody is administered to a patient weighing ≥ 60 to < 100 kg:
(a) once on Day 1 at a dose of 2700 mg; and
(b) on Day 15 and every eight weeks thereafter at a dose of 3300 mg.

The kit may comprise a dose of an anti-C5 antibody wherein the anti-C5 antibody is administered to a patient weighing ≥ 100 kg:
(a) once on Day 1 at a dose of 3000 mg; and
(b) on Day 15 and every eight weeks thereafter at a dose of 3600 mg.

### EXAMPLES

### EXAMPLE 1: A Phase 3, Single Arm, Multicenter Study of ravulizumab (ALXN1210) in Complement Inhibitor-Naive Adult Patients with Atypical Hemolytic Uremic Syndrome (aHUS)

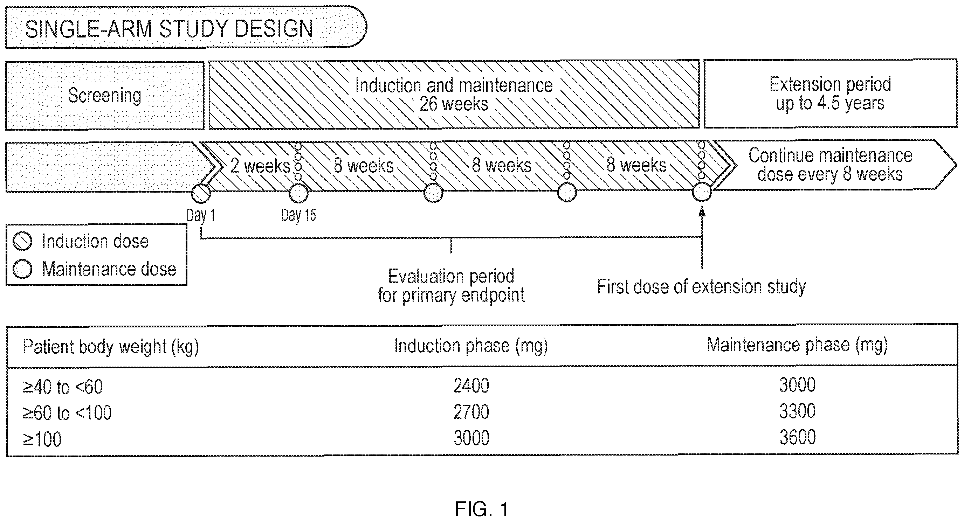

A single arm study of ravulizumab (ALXN1210-aHUS-311) is conducted in complement inhibitor treatment-naive adult and adolescent patients with atypical hemolytic uremic syndrome (aHUS). Figure 1 illustrates the study design.

aHUS is a thrombotic microangiopathy (TMA), most often caused by mutations in genes encoding proteins involved in the alternative pathway of complement (APC) or by autoantibodies against APC regulatory proteins (Noris, et al., Clin. J. Am. Soc. Nephrol. 2010;5:1844-59). Patients with aHUS are at risk for life-threatening manifestations of disease resulting from endothelial damage, including thrombocytopenia, intravascular hemolysis, acute renal failure, and extra-renal tissue damage. Importantly, approximately 20% of patients experience extra-renal manifestations of disease, including central nervous system, cardiac, GI, distal extremity, and severe systemic organ involvement (Loirat, et al., Orphanet J. Rare Dis. 2011;6:60 and Brodsky, Blood. 2015;126:2459-65). Before the availability of eculizumab, mortality rates among patients with aHUS were as high as 15% during the acute progressing phase of the disease (Noris, et al., Clin. J. Am. Soc. Nephrol. 2010;5:1844-59) and Sellier-Leclerc, J. Am. Soc. Nephrol. 2007;18:2392-2400). Up to 50% of patients progressed to end-stage kidney disease (ESKD), often within a year of disease onset, and required dialysis or kidney transplant to sustain life. Chronic, uncontrolled terminal complement activation, specifically, activation of complement component 5 (C5) and dysregulation of complement activity, is central to the pathogenesis of aHUS and the devastating manifestations of this disease. As a result, the targeted blockade of C5, with selective inhibition of generation of C5a and C5b-9, represents an important therapeutic mechanism of treatment.

### 1. Objectives

The primary objective of the study is to assess the efficacy of ravulizumab in complement inhibitor treatment naive adolescent and adult patients with aHUS to inhibit complement-mediated TMA as characterized by thrombocytopenia, hemolysis, and renal impairment.

The secondary objectives of the study are to (1) characterize the safety and tolerability of ravulizumab in this patient population, (2) evaluate the efficacy of ravulizumab by additional measures (*e.g*., dialysis requirement status, time to complete TMA response, complete TMA Response status over time, observed value and change from baseline in estimated glomerular filtration rate (eGFR), chronic kidney disease (CKD) stage (as evaluated at select target days and classified as improved, stable (no change), or worsened compared to baseline), observed value and change from baseline in hematologic parameters (platelets, LDH, hemoglobin), increase in hemoglobin of ≥ 20 g/L from baseline (sustained for at least 2 consecutive measurements obtained at least 4 weeks apart), change from baseline in quality of life (QoL) (as measured by EuroQol 5 dimensions 3 level (EQ-5D-3L; all patients), Functional Assessment of Chronic Therapy (FACIT) Fatigue version 4 (patients < 18 years of age), and Pediatric FACIT Fatigue (patients < 18 years of age) questionnaires)), (3) characterize the PK/pharmacodynamics (PD) of ravulizumab by changes in serum ravulizumab concentration over time and changes in free C5 concentrations over time, and (4) evaluate the long-term safety and efficacy of ravulizumab.

### 2. Endpoints

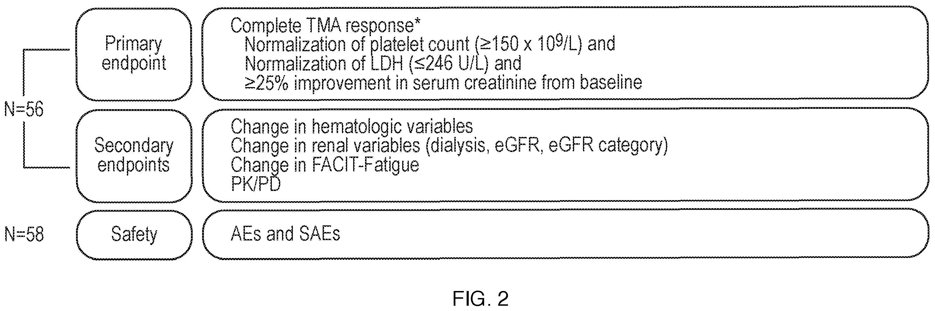

The primary, secondary, and safety endpoints for the study are summarized in Figure 2. The primary efficacy endpoint is Complete TMA Response during the 26-week Initial Evaluation Period, as evidenced by normalization of hematological parameters (platelet count and LDH) and ≥ 25% improvement in serum creatinine from baseline and confirmed by 2 consecutive measurements obtained at least 4 weeks apart.

The secondary efficacy endpoints of the study the following:
A. Dialysis requirement status;
B. Time to Complete TMA Response;
C. Complete TMA Response status over time;
D. Observed value and change from baseline in eGFR;
E. CKD stage, as evaluated by the Investigator at select target days and classified as improved, stable (no change), or worsened compared to baseline;
F. Observed value and change from baseline in hematologic parameters (platelets, LDH, hemoglobin);
G. Increase in hemoglobin of ≥ 20 g/L from baseline, sustained for at least 2 consecutive measurements obtained at least 4weeks apart;
H. Change from baseline in QoL, as measured by EQ-5D-3L (all patients), FACIT Fatigue version 4 (patients ≥ 18 years of age), and Pediatric FACIT Fatigue (patients < 18 years of age) questionnaires.

The Pharmacokinetic (PK) and Pharmacodynamic (PD) endpoints of this study are changes in serum ravulizumab concentration over time and changes in free C5 concentrations over time.

The safety and tolerability of ravulizumab is evaluated by physical examinations, vital signs, electrocardiograms (ECGs), laboratory assessments, and incidence of AEs and SAEs. The proportion of patients who develop antidrug antibodies (ADA) are also assessed.

Exploratory biomarkers of PD effect include, but are not limited to, change from baseline in levels of markers of complement dysregulation (*e.g.,* Factor Ba), vascular inflammation (*e.g.,* soluble tumor necrosis factor receptor 1 [sTNFR1]), endothelial activation/damage (*e.g.,* soluble vascular adhesion molecule 1 [sVCAM1], thrombomodulin), coagulation (*e.g.,* D-dimer), and renal injury (*e.g.,* cystatin C). Additional assessments may include measurements of ravulizumab excretion in urine, chicken red blood cell (cRBC) hemolysis, total C5, autoantibodies to complement proteins (*e.g.,* anti-factor H) and APC activity i*(e.g.,* Modified Ham's test, complement deposition assays).

Exploratory genetics can be performed to investigate genetic variants in genes known to be associated with aHUS, as well as to identify novel genetic variants associated with aHUS, complement dysregulation, or metabolism or efficacy of ravulizumab. Patients can opt-out from providing a sample for exploratory genetics and still participate in the study.

### 3. Summary of Study Design

Study ALXN1210-aHUS-311 is a Phase 3, open-label, single arm, multicenter study to evaluate the safety and efficacy of ravulizumab administered by intravenous (IV) infusion to adolescent (12 to < 18 years of age) and adult (≥ 18 years of age) patients with aHUS. The study is enrolling approximately 55 patients to receive ravulizumab. Figure 1 illustrates the study design. All patients are naive to complement inhibitor treatment and include at least 6 and up to 10 adolescent (12 to < 18 years of age at Screening) patients and at least 10 and up to 25 patients with prior kidney transplants.

The study consists of an up to 7-day Screening Period, a 26-week Initial Evaluation Period, and an Extension Period of up to 2 years. Dosages are based on the patient's last recorded study visit body weight (Table 5). Patients receive a loading dose of ravulizumab IV (2400 mg for patients weighing ≥ 40 to < 60 kg, 2700 mg for patients weighing ≥ 60 to < 100 kg, 3000 mg for patients weighing ≥ 100 kg) on Day 1, followed by maintenance doses of ravulizumab IV (3000 mg for patients weighing ≥ 40 to < 60 kg, 3300 mg for patients weighing ≥ 60 to < 100 kg, 3600 mg for patients weighing ≥ 100 kg) on Day 15 and once every 8 weeks (q8w) thereafter for a total of 26 weeks of treatment. After the Initial Evaluation Period, patients enter an Extension Period and receive ravulizumab until the product is registered or approved (in accordance with country specific regulations) or for up to 2 years, whichever occurs first. The end of trial is defined as the last patient's last visit.

This Phase 3, open-label, single arm study evaluates the safety and efficacy of treatment with ravulizumab. Although no formal comparison analyses are planned for this study, results from ravulizumab treated patients are evaluated in the context of results observed in a historical control group of patients treated with eculizumab. The historical control group is comprised of patients with aHUS who were treated with eculizumab in the C08-002A/B, C10-003 and C10-004 prospective registrational studies, for which study design and conduct features of interest that might influence the effect size were similar to the current study. In addition, the control group is restricted to patients ≥ 12 years of age and with 4 weeks or less on PE/PI prior to eculizumab treatment to further align with eligibility criteria for the current study.

The Schedule of Assessments for Screening and the Initial Evaluation Period is shown in Table 1. The Schedule of Assessments for the Extension Period is shown in Table 2. Additional (unscheduled) visits outside the specified visits are permitted at the discretion of the Investigator. Procedures, tests, and assessments are performed at the discretion of the Investigator. Any tests, procedures, or assessments performed at the Unscheduled Visits are recorded on the electronic Case Report Forms (eCRFs). Local laboratory or central laboratory analysis are used for Unscheduled Visit tests. However, if local laboratory tests are to be used, duplicate samples are collected at the Unscheduled Visit for central laboratory testing.

**Table 1: Schedule of Study Visits and Assessments: Screening Through End of Initial Evaluation Period**

| **Period** | **Screeni ng** | **Initial Evaluation Period** | | | | | | | | | | | | | | | |
|---|---|---|---|---|---|---|---|---|---|---|---|---|---|---|---|---|---|
| **Study Day** | **-7 to -1** | **1** | **8** | **15** | **2 2** | **29** | **43** | **57** | **71** | **85** | **99** | **11 3** | **12 7** | **14 1** | **15 5** | **16 9** | **183^{v}/E T** |
| **Window (day)** | **N/A** | | **± 2** | **±3** | **± 3** | **±3** | **±3** | **±3** | **±3** | **±3** | **±5** | **±5** | **±5** | **±5** | **±5** | **±5** | **±2** |
| Informed consent | X | | | | | | | | | | | | | | | | |
| Confirmation or administratio n of meningococc al vaccination^{a} | X | | | | | | | | | | | | | | | | |
| Medical history and demographics | X | | | | | | | | | | | | | | | | |
| ADAMTS13 | X | | | | | | | | | | | | | | | | |
| HIV screen^{b} | X | | | | | | | | | | | | | | | | |
| Streptococcal | X | | | | | | | | | | | | | | | | |
| pneumoniae HUS testing | | | | | | | | | | | | | | | | | |
| ST-HUS screen^{c} | X | | | | | | | | | | | | | | | | |
| Height | X | | | | | | | | | | | | | | | | |
| Weight | X | X | X | X | X | X | X | X | X | X | X | X | X | X | X | X | X |
| Pregnancy test^{d} | X | X | | X | | | | | X | | | | X | | | | X |
| FACIT-Fatigue questionnaire /Pediatric FACIT-Fatigue questionnaire^{e ,f} | | X | X | | | X | | | X | | | | X | | | | X |
| EQ-5D-3L questionnaire^{f} | | X | X | | | X | | | X | | | | X | | | | X |
| Patient-reported aHUS symptoms questionnaire^{f} | | X | X | X | X | X | X | X | X | X | X | X | X | X | X | X | X |
| Resource utilization patient questionnaire^{f} | | X | | | | X | | | X | | | | X | | | | X |
| Physical examination | X | | | | | | | | | | | | | | | | X |
| Abbreviated physical examination^{g} | | X | X | X | X | X | X | X | X | X | X | X | X | X | X | X | |
| Assessment of extra-renal signs or symptoms of aHUS | X | X | X | X | X | X | X | X | X | X | X | X | X | X | X | X | X |
| Vital signs^{h} | X | X | X | X | X | X | X | X | X | X | X | X | X | X | X | X | X |
| Safety 12-Lead ECG' | X | | | | | | | X | | | | | | | | | X |
| Chemistry^{j} | X^{u} | X | X | X | X | X | X | X | X | X | X | X | X | X | X | X | X |
| LDH isozymes^{k} | X | X | X | X | X | X | X | X | X | X | X | X | X | X | X | X | X |
| Hematology including free hemoglobin and coagulation¹ | X^{u} | X | X | X | X | X | X | X | X | X | X | X | X | X | X | X | X |
| Urinalysis and urine chemistry | X | X | | X | | X | | X | | X | | X | | X | | | X |
| PK/PD sampling^{m} | | X ₘ | | X ₘ | | X ₘ | X ₘ | X ₘ | X ₘ | X ₘ | X ₘ | X^{m} | X^{m} | X^{m} | X^{m} | X^{m} | X^{m} |
| Urine sampleⁿ | | X | | X | | X | | | X | | | | | | | | |
| Exploratory APC activity^{o} | | X | | X | | | X | | X | | X | | X | | X | | X |
| Exploratory biomarkers^{p} | | X | | X | | | | | X | | | | X | | | | X |
| Exploratory genetic sample^{q} | | X | | | | | | | | | | | | | | | |
| Immunogenic ity (ADA)^{r} | | X | | | | | | | X | | | | X | | | | X |
| Review safety card | | X | X | X | X | X | X | X | X | X | X | X | X | X | X | X | X |
| Concomitant medications^{s} | | ←Monitor continuously→ | | | | | | | | | | | | | | | |
| Record plasma exchange | | ←Monitor continuously- | | | | | | | | | | | | | | | |
| Adverse events | | ←Monitor continuously→ | | | | | | | | | | | | | | | |
| ALXN1210 administratio n^{t} | | X | | X | | | | | X | | | | X | | | | |

| | | | | | | | | | | | | | | | | | |
|---|---|---|---|---|---|---|---|---|---|---|---|---|---|---|---|---|---|
| Abbreviations: ADA = antidrug antibody; ADAMTS13 = a disintegrin and metalloproteinase with a thrombospondin type 1 motif, member 13; aHUS = atypical hemolytic uremic syndrome; APC = alternative pathway of complement; ECG = electrocardiogram; EQ-5D-3L = EuroQol5 dimensions 3 level; ET = early termination; FACIT = functional assessment of chronic illness therapy; HUS = hemolytic uremic syndrome; LDH = lactate dehydrogenase; N/A = not applicable; PD = pharmacodynamics; PK = pharmacokinetics; QoL = quality of life; ST-HUS = Shiga toxin-related hemolytic uremic syndrome. ^{a} All patients are vaccinated against meningococcal infections within 3 years prior to, or at the time of, initiating study drug. Patients who initiate study drug treatment less than 2 weeks after receiving a meningococcal vaccine receive treatment with appropriate prophylactic antibiotics until 2 weeks after vaccination. Patients who have not been vaccinated prior to initiating ravulizumab treatment receive prophylactic antibiotics prior to and for at least 2 weeks after meningococcal vaccination. ^{b} Human Immunodeficiency Virus Type 1 and Human Immunodeficiency Virus Type 2 screening. ^{c} Stool sample for Shiga toxin enzyme immunoassay. ^{d} Female patients of childbearing potential only. Serum pregnancy test at Screening and Day 183; urine pregnancy test at all other required time points. A negative urine test result is required prior to administering study drug to female patients of childbearing potential at the potential study visits. ^{e} FACIT Fatigue version 4 is used for patients ≥ 18 years of age at Screening. Pediatric FACIT Fatigue is used for patients < 18 years of age at Screening. ^{f} On dosing days, patient-reported assessments are performed prior to dosing. ^{g} Abbreviated physical examination consists of a body system relevant examination based upon Investigator (or designee) judgment and patient symptoms. At least 1 body system is checked for an abbreviated exam. ^{h} Vital sign measurements are taken after the patient has been resting for at least 5 minutes and include systolic and diastolic BP (millimeters of mercury [mmHg]), pulse oximetry, heart rate (beats/minute), respiratory rate (breaths/minute), and oral or tympanic temperature (degrees Celsius [°C] or degrees Fahrenheit [°F]). On dosing days, vital signs are taken predose. ⁱ Single 12-lead ECG is collected at Screening, predose on Day 57, and Day 183. Patients are supine for approximately 5 to 10 minutes before ECG collection and remain supine but awake during ECG collection. ^{j} Clinical safety laboratory measurements are collected predose on dosing days. LDH for eligibility is determined from the chemistry assessment. Follicle stimulating hormone levels are measured during Screening only in order to confirm postmenopausal status. ^{k} Serum sample for LDH isozyme testing is only collected at selected sites at any/all timepoints prior to ravulizumab dosing, dependent on sample testing availability. ¹ Assessment for safety, as well as the primary and secondary endpoints. ^{m} Serum samples for PK/PD analyses are collected predose (within 0.5 hours prior to the start of infusion) and at end of infusion (EOI) (within 0.5 hours after the EOI) on Days 1, 15, 71, and 127; and at any time on Days 29, 43, 57, 85, 99, 113, 141, 155, and 169; and predose on Day 183 (note additional samples for PK/PD are collected on Day 183 as part of the Extension Period). End of infusion samples are drawn from the patient's opposite, noninfused arm. All collection times are recorded in the eCRF. ⁿ Urine sample for drug measurement are collected and at end of infusion (EOI) (within 0.5 hours after the EOI) on Days 1, 15, and 71; and at any time on Day 29. ^{o} Collection of serum samples are predose on days of dosing and for days without dosing at any time of day. All collection times are recorded in the eCRF. ^{p} Collection of serum, plasma and urine for exploratory biomarker analysis is collected at Baseline and at post-treatment timepoints just prior to ravulizumab dosing. ^{q} A single whole blood collection from those patients who consent to genetic testing can be collected anytime during the study. ^{r} ADA serum samples are collected predose on Days 1, 71, and 127. Day 183 collection occurs prior to first dose in the Extension Period. All collection times are recorded in the eCRF. If results of the test are positive, the test is repeated every 3 months until results become negative or stabilize, based on the measured titer and the safety assessments. ^{s} Concomitant medications must be collected at all study visits and checked against the prohibited medication list. ^{t} The dose of ravulizumab is based on the patient's last recorded study visit body weight. ^{u} Local laboratory or central laboratory analysis can be used to determine eligibility at Screening. However, if local laboratory tests are used, duplicate samples for LDH, platelet count, hemoglobin, and serum creatinine are collected at this visit for central laboratory testing. ^{v} The primary efficacy endpoint assessment is before dosing on Day 183. Dosing on Day 183 is the start of the Extension Period. | | | | | | | | | | | | | | | | | |

**Table 2: Schedule of Study Visits and Assessments: Extension Period**

| **Period** | **Extension Period** | | | | | | | | | | | | | |
|---|---|---|---|---|---|---|---|---|---|---|---|---|---|---|
| **Study Day** | **183'** | **239** | **295** | **351** | **407** | **463** | **519** | **575** | **631** | **687** | **743** | **799** | **855** | **911/ET/EOS** |
| **Window (day)** | **±2** | **±7** | **±7** | **±7** | **±7** | **±7** | **±7** | **±7** | **±7** | **±7** | **±7** | **±7** | **±7** | **±7** |
| Weight | | X | X | X | X | X | X | X | X | X | X | X | X | X |
| Pregnancy test^{a} | | X | X | X | X | X | X | X | X | X | X | X | X | X |
| FACIT-Fatigue questionnaire /Pediatric FACIT-Fatigue questionnaire^{b,c} | | | | X | | | | X | | | X | | | X |
| EQ-5D-3L questionnaire^{c} | | | | X | | | | X | | | X | | | X |
| Patient-reported aHUS symptoms questionnaire^{c} | | X | X | X | X | X | X | X | X | X | X | X | X | X |

| **Study Day** | **183¹** | **239** | **295** | **351** | **407** | **463** | **519** | **575** | **631** | **687** | **743** | **799** | **855** | **911/ET/EOS** |
|---|---|---|---|---|---|---|---|---|---|---|---|---|---|---|
| **Window (day)** | **±2** | **±7** | **±7** | **±7** | **±7** | **±7** | **±7** | **±7** | **±7** | **±7** | **±7** | **±7** | **±7** | **±7** |
| Resource utilization patient questionnaire^{c} | | X | X | X | X | X | X | X | X | X | X | X | X | X |
| Physical examination | | | | | | | | | | | | | | X |
| Abbreviated physical examination^{d} | | X | X | X | X | X | X | X | X | X | X | X | X | |
| Assessment of extra-renal signs or symptoms of aHUS | | X | X | X | X | X | X | X | X | X | X | X | X | X |
| Vital signs^{e} | | X | X | X | X | X | X | X | X | X | X | X | X | X |
| Safety 12-Lead ECG^{f} | | | | | | | | | | | | | | X |
| Chemistry | | X | X | X | X | X | X | X | X | X | X | X | X | X |
| Hematology and coagulation^{g} | | X | X | X | X | X | X | X | X | X | X | X | X | X |
| Urinalysis and urine chemistry | | X | X | X | X | X | X | X | X | X | X | X | X | X |
| PK/PD sampling^{h} | X^{h} | | | X^{h} | | | | X^{h} | | | X^{h} | | | X^{h} |
| Exploratory biomarkers' | | X | X | X | X | X | X | X | X | X | X | X | X | X |
| Immunogenicity (ADA)^{j} | | | | X^{j} | | | | X^{j} | | | X^{j} | | | X^{j} |
| Review safety card | | X | X | X | X | X | X | X | X | X | X | X | X | X |
| Concomitant medications^{k} | ←Monitor continuously→ | | | | | | | | | | | | | |
| Record plasma exchange | ←Monitor continuously→ | | | | | | | | | | | | | |
| Adverse events | ←Monitor continuously→ | | | | | | | | | | | | | |
| ALXN1210 administration¹ | X¹ | X | X | X | X | X | X | X | X | X | X | X | X | |

| | | | | | | | | | | | | | | |
|---|---|---|---|---|---|---|---|---|---|---|---|---|---|---|
| Abbreviations: ADA = antidrug antibody; aHUS = atypical hemolytic uremic syndrome; ECG = electrocardiogram; EOS = end of study; EQ-5D = EuroQol five dimensions; ET = early termination; FACIT = functional assessment of chronic illness therapy; PD =pharmacodynamics; PK = pharmacokinetics; QoL = quality of life ^{a} Female patients of childbearing potential only. Serum pregnancy test at ET only; urine pregnancy test at all other required time points. A negative urine test result is required prior to administering ravulizumab to female patients of childbearing potential at the indicated study visits. ^{b} FACIT Fatigue version 4 is used for patients ≥ 18 years of age at Screening. Pediatric FACIT Fatigue is used for patients < 18 years of age at Screening. ^{c} On dosing days, patient-reported assessments are performed prior to dosing. ^{d} Abbreviated physical examination consists of a body system relevant examination based upon Investigator judgment and patient symptoms. ^{e} Vital sign measurements are taken after the patient has been resting for at least 5 minutes and include systolic and diastolic BP (millimeters of mercury [mmHg]), pulse oximetry, heart rate (beats/minute), respiratory rate (breaths/minute), and oral or tympanic temperature (degrees Celsius [°C] or degrees Fahrenheit [°F]). On dosing days, vital signs are taken predose. ^{f} Single 12-lead ECG is collected at Day 911 or ET. Patients must be supine for approximately 5 to 10 minutes before ECG collection and remain supine but awake during ECG collection. ^{g} Assessment for safety, as well as the primary and secondary endpoints. ^{h} Serum samples for PK/PD analysis are collected predose (within 0.5 hours prior to the start of infusion) and EOI (within 0.5 hours after the EOI) on Days 351, 575, and 743; EOI (within 0.5 hours after the EOI) on Day 183; and at any time on Day 911 or ET. End of infusion samples are drawn from the patient's opposite, noninfused arm. All collection times are recorded in the eCRF· ⁱ Serum, plasma and urine for exploratory biomarker analysis is collected at the indicated timepoints just prior to ravulizumab dosing; and at any time on Day 911 or ET. All collection times are recorded in the eCRF. ^{j} A predose serum sample is collected on Days 351, 575, and 743. A serum sample is also collected at any time on Day 911 or ET. All collection times are recorded in the eCRF. If results of the test are positive, the test is repeated every 3 months until results become negative or stabilize, based on the measured titer and the safety assessments. ^{k} Concomitant medications are collected at all study visits and checked against the prohibited medication list. ^{l} Extension Period begins at the start of Day 183 dosing. The dose of ravulizumab is based on the patient's last recorded study visit body weight. | | | | | | | | | | | | | | |

### 4. Study Population

A total of approximately 55 patients with documented aHUS are enrolled and assigned to treatment with ravulizumab at approximately 200 investigative sites globally. The study enrolls at least 6 and up to 10 adolescent (12 to < 18 years of age at Screening) patients and at least 10 and up to 25 patients with prior kidney transplants.

Individuals who do not meet the criteria for participation in this study (screen failure) can be rescreened. Patients can be rescreened a maximum of 2 times. Prospective approval of protocol deviations to recruitment and enrollment criteria, also known as protocol waivers or exemptions, is not permitted.

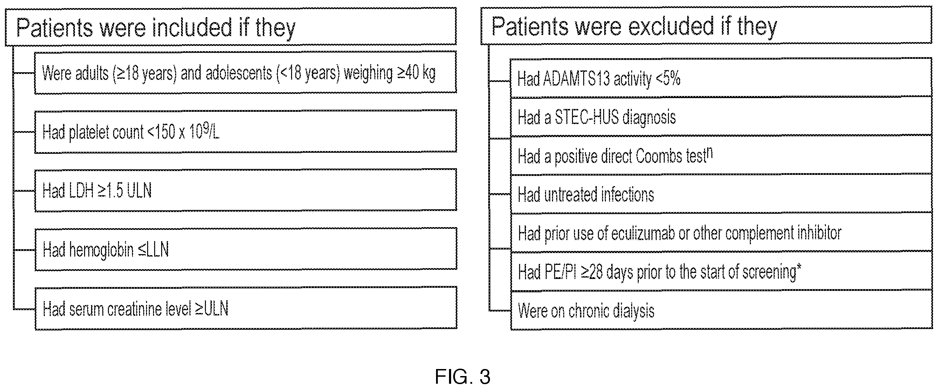

A summary of the inclusion and exclusion criteria is set forth in Figure 3. Patients are eligible for enrollment in the study if they meet all of the following criteria and none of the exclusion criteria:
Male or female patients ≥ 12 years of age and weighing ≥ 40 kg at the time of consent.
- Evidence of TMA, including thrombocytopenia, evidence of hemolysis, and kidney dysfunction, based on the following Screening Visit laboratory findings: Platelet count < 150,000 per microliter (µL), and LDH ≥ 1.5 × upper limit of normal (ULN), and hemoglobin ≤ lower limit of normal (LLN) for age and gender, and serum creatinine level ≥ ULN in adults (≥ 18 years of age), or ≥ 97.5^{th} percentile for age at Screening in adolescents (12 to < 18 years of age) (patients who require dialysis for acute kidney injury are also eligible).
- Among patients with a kidney transplant: known history of aHUS prior to current kidney transplant, or no known history of aHUS, and persistent evidence of TMA after suspension of dosing of calcineurin inhibitor ([CNI]; *e*.*g*., cyclosporine, tacrolimus) or mammalian target of rapamycin inhibitor ([mTORi]; *e.g.,* sirolimus, everolimus) for a minimum of 4 days and a maximum of 7 days.
- Among patients with onset of TMA postpartum, persistent evidence of TMA for > 3 days after the day of childbirth.
- To reduce the risk of meningococcal infection (*Neisseria meningitidis*)*,* all patients are be vaccinated against meningococcal infections within 3 years prior to, or at the time of, initiating study drug. Patients who receive a meningococcal vaccine less than 2 weeks before initiating ravulizumab treatment receive treatment with appropriate prophylactic antibiotics until 2 weeks after vaccination. Patients who have not been vaccinated prior to initiating ravulizumab treatment receive prophylactic antibiotics prior to and for at least 2 weeks after meningococcal vaccination.
- Patients < 18 years of age must have been vaccinated against *Haemophilus influenzae* type b (Hib) and *Streptococcus pneumoniae* according to national and local vaccination schedule guidelines.
- Female patients of childbearing potential and male patients with female partners of childbearing potential must follow protocol-specified guidance for avoiding pregnancy while on treatment and for 8 months after last dose of study drug.
- Willing and able to give written informed consent and comply with the study visit schedule. For patients < 18 years of age, patient's legal guardian must be willing and able to give written informed consent and the patient must be willing to give written informed assent.

Samples collected at Screening can be tested at either a local or central laboratory. If local laboratory tests are used for LDH, platelet count, hemoglobin, and serum creatinine, duplicate samples are collected for central laboratory testing to ensure baseline and postbaseline measurements for analyses are resulted from the central laboratory. Although local laboratory results can be used to expedite assessment of eligibility, the final determination of these Inclusion Criteria is be based on the central laboratory results.

Patients are excluded from study enrollment if they meet any of the following criteria:
A. Known 'a disintegrin and metalloproteinase with a thrombospondin type 1 motif, member 13' (ADAMTS13) deficiency (activity < 5%).
B. Shiga toxin-related hemolytic uremic syndrome (ST-HUS).
C. *Streptococcal pneumoniae-*related hemolytic uremic syndrome (HUS), as evidenced by a positive direct Coombs test and infection by *Streptococcal pneumoniae* (e.g., culture, antigen test).
D. Known Human Immunodeficiency Virus (HIV) infection.
E. Unresolved systemic meningococcal disease.
F. Patients with a confirmed diagnosis of ongoing sepsis defined as positive blood cultures within 7 days prior to the start of Screening and untreated with antibiotics.
G. Presence or suspicion of active and untreated systemic bacterial infection that, in the opinion of the Investigator, confounds an accurate diagnosis of aHUS or impedes the ability to manage the aHUS disease.
H. Pregnancy or lactation.
I. Heart, lung, small bowel, or liver transplant.
J. Among patients with kidney transplant, any of the following:
   a. Acute kidney dysfunction within 4 weeks of transplant consistent with the diagnosis of acute antibody-mediated rejection (AMR) according to Banff 2013 criteria, or
   b. Acute kidney dysfunction within 4 weeks of transplant and a rising donor-specific antibody (DSA) consistent with a clinical diagnosis of acute AMR.
   c. History of polycystic kidney disease.
K. Among patients ≥ 18 years of age presenting with systolic blood pressure (SBP) ≥ 170 mmHg, or patients 12 to < 18 years of age presenting with a clinical diagnosis of hypertension, any of the following:
   a. Persistent evidence of TMA (inclusion criterion number 2) after less than 4 days of blood pressure (BP) reduction to ≤ 140 mmHg.
   b.Known left ventricular hypertrophy.
   c. Known small and hyperechoic kidneys on ultrasound.
L. Identified drug exposure-related HUS.
M. Receiving PE/PI, for 28 days or longer, prior to the start of Screening for the current TMA.
N. History of malignancy within 5 years of Screening with the exception of a nonmelanoma skin cancer or carcinoma in situ of the cervix that has been treated with no evidence of recurrence.
O. Bone marrow transplant (BMT)/hematopoietic stem cell transplant (HSCT) within the last 90 days prior to the start of Screening.
P. HUS related to vitamin B12 deficiency.
Q. Known systemic sclerosis (scleroderma), systemic lupus erythematosus (SLE), or antiphospholipid antibody positivity or syndrome.
R. Chronic dialysis (defined as dialysis on a regular basis as renal replacement therapy for ESKD).
S. Patients receiving chronic intravenous immunoglobulin (IVIg) within 8 weeks prior the start of Screening, unless for unrelated medical condition (eg, hypogammaglobinemia); or chronic rituximab therapy within 12 weeks prior to the start of Screening.
T. Patients receiving other immunosuppressive therapies such as steroids, mTORi (e.g., sirolimus, everolimus), CNI (e.g., cyclosporine or tacrolimus) are excluded unless: a) part of an established post-transplant antirejection regime, or b) patient has confirmed anti-complement factor antibodies requiring immunosuppressive therapy, or c) steroids are being used for a condition other than aHUS (*e.g.,* asthma).
U. Participation in another interventional treatment study or use of any experimental therapy within 30 days before initiation of study drug on Day 1 in this study or within 5 half-lives of that investigational product, whichever is greater.
V. Prior use of eculizumab or other complement inhibitors.
W. Hypersensitivity to murine proteins or to one of the excipients.
X. Any medical or psychological condition that, in the opinion of the Investigator, could increase the risk to the patient by participating in the study or confound the outcome of the study.
Y. Known or suspected history of drug or alcohol abuse or dependence within 1 year prior to the start of Screening.
Laboratory results for Exclusion Criterion number 1 may not be available prior to first dose. Later results for Exclusion Criterion number A may lead to discontinuation and replacement of the patient.

A patient has the right to withdraw from the study at any time. If a patient withdraws consent, the assessments specified for the Early Termination (ET) visit are performed. Patients who withdraw from the study are not replaced. A patient can be discontinued from study drug if the Investigator or Sponsor have reason to believe it is in the best interest of the patient to stop treatment.

Patients with prior kidney transplant developing AMR (C4d positive renal biopsy) and for whom rituximab is deemed the appropriate therapy must withdraw from the study and receive standard of care therapy. The primary reason and any other reason(s) for the discontinuation are recorded on the eCRF.

If a patient is discontinued from the study with an ongoing AE or an unresolved laboratory result that is significantly outside of the reference range and clinically significant, the Investigator attempts to provide follow-up until a satisfactory clinical resolution of the laboratory result or adverse event is achieved.

The Sponsor or Competent Authority can terminate the study for reasonable cause. Conditions that warrant termination of the study include, but are not limited to: (1) discovery of an unexpected, serious, or unacceptable risk to patients enrolled in the study, (2) sponsor decision to suspend or discontinue testing, evaluation, or development of the study drug, (3) failure of the Investigator to comply with the approved protocol, pertinent guidelines, and/or regulations, and (4) submission of knowingly false information from the Investigator to the Sponsor and/or regulatory authorities.

If it is determined at any point that a patient's Screening data does not satisfy one or more of the following inclusion/exclusion criteria (Inclusion Criterion number 2 or Exclusion Criterion number 1), after receiving at least 1 dose of investigational product (e.g., patient local laboratory data used to confirm eligibility criteria are subsequently determined by a central laboratory to no longer meet eligibility criteria), the patient is discontinued from the study and can be replaced. Early termination procedures are performed on patients who are terminated early and all AEs are collected until 60 days after the patient's last dose of study drug.

The end of the study is defined as the date of the last patient's last visit in the Extension Period.

### 5. Study Treatment

Ravulizumab, a humanized anti-C5 monoclonal antibody composed of two 448 amino acid heavy chains and two 214 amino acid light chains, is an IgG2/4 kappa immunoglobulin consisting of human constant regions, and murine complementarity-determining regions grafted onto human framework light- and heavy-chain variable regions. Ravulizumab and eculizumab share over 99% primary amino acid sequence identity and have very similar pharmacology.

Ravulizumab drug product is supplied for clinical studies as a sterile, preservative-free 10-mg/mL solution in single-use vials and designed for infusion by diluting into commercially available saline (0.9% sodium chloride injection; country-specific pharmacopeia) for administration via IV infusion. Table 3 and the current IB provide additional information.

**Table 3: Study Drug**

| **Product Name** | **Ravulizumab** |
|---|---|
| **Dosage Form** | Concentrated solution (10 mg/mL) for infusion |
| **Route of Administration** | Intravenous infusion |
| **Physical Description** | Clear to translucent, slight whitish color, practically free from particles |
| **Manufacturer** | Alexion Pharmaceuticals, Inc. or Contracted Manufacturing Organization |

Ravulizumab is packaged in United States Pharmacopeia (USP)/ European Union Pharmacopeia (EP) Type 1 borosilicate glass vials and stoppered with a butyl rubber stopper with an aluminum overseal and a flip-off cap. Study drug are supplied in kits. Ravulizumab is released to each site upon receipt of all required essential documents based upon applicable regulations.

Upon arrival of the study drug kits at the study site, the pharmacist (or trained designee) promptly removes the study drug kits from the shipping cooler and stores them in their original cartons under refrigerated conditions at 2°C to 8°C (35°F to 47°F) and protected from light. Ravulizumab is not frozen. Study drug are stored in a secure, limited-access storage area, and the temperature is monitored daily.

The drug product is at room temperature prior to administration. The material is not heated (*e.g.,* by using a microwave or other heat source) other than by ambient air temperature.

Ravulizumab is not administered as an IV push or bolus injection. Infusions of study drug are prepared using aseptic technique. The patient's required dose of Ravulizumab is further diluted into commercially available saline (0.9% sodium chloride; country-specific pharmacopeia) at the volume specified in Table 4. Ravulizumab solution in diluent is administered to the patient using an IV tubing administration set via an infusion pump. Use of an in-line filter for infusion is required.

**Table 4: Dosing Reference Chart for Ravulizumab Dose Preparation**

| **Dose Type** | **Body Weight (kg)^{a}** | **Dose (mg)** | **ALXN 1210 Vol. (mL)** | **Saline Vol. (mL)** | **Total Vol. (mL)** | **Min. Infusion Duration minutes (hours)** | **Max. Infusion Rate (mL/hour)** |
|---|---|---|---|---|---|---|---|
| Loading | ≥ 40 to < 60 | 2400 | 240 | 240 | 480 | 114 (1.9) | 253 |
| | ≥ 60 to < 100 | 2700 | 270 | 270 | 540 | 102 (1.7) | 333 |
| | ≥ 100 | 3000 | 300 | 300 | 600 | 108 (1.8) | 333 |
| Maintenance | ≥ 40 to < 60 | 3000 | 300 | 300 | 600 | 138 (2.3) | 267 |
| | ≥ 60 to < 100 | 3300 | 330 | 330 | 660 | 120 (2.0) | 333 |
| | ≥ 100 | 3600 | 360 | 360 | 720 | 132 (2.2) | 333 |

| | | | | | | | |
|---|---|---|---|---|---|---|---|
| Refer to the Pharmacy Manual for additional dose preparation instructions. ^{a} Body weight as recorded at the last study visit. | | | | | | | |

Doses of study drug are only prepared and dispensed by a pharmacist or a medically qualified staff member. Study drug is dispensed only to enrolled patients who are confirmed eligible for participation in this study. Once study drug is prepared for a patient, it is only administered to that patient. Vials of study drug are for one-time use only and any drug product remaining in the vial is not used for another patient. Any drug remaining in the infusion tubing or infusion bag is not used for another patient.

All clinical study material is stored in a secure place and allocated and dispensed by appropriately trained persons. Detailed records of the amounts of the investigational product received, dispensed, and destroyed are maintained. Unless otherwise notified, empty vials and vials with residual materials are kept for inspection and accountability by the study monitor prior to their destruction or handled per local pharmacy standard operating procedures (SOPs) for clinical study drugs. To satisfy regulatory requirements regarding drug accountability, at the end of the study all remaining ravulizumab inventory is reconciled and destroyed or returned to Alexion according to applicable regulations.

Patients receive ravulizumab for 26 weeks. Ravulizumab is administered as a slow IV infusion over approximately 2 hours. Ravulizumab is not administered as an IV push or bolus injection.

The dose regimen for ravulizumab during the Initial Evaluation Period is based on the patient's last recorded study visit body weight (Table 5). Patients receive a loading dose of ravulizumab IV on Day 1, followed by maintenance dosing of ravulizumab IV on Day 15 and q8w (every eight weeks) thereafter.

**Table 5: Loading and Maintenance Treatment Regimens**

| **Body Weight^{a}** | **Loading Dose (Day 1)** | **Maintenance Dose (Days 15, 71, and 127)** |
|---|---|---|
| ≥ 40 to < 60 kg | 2400 mg | 3000 mg |
| ≥ 60 to < 100 kg | 2700 mg | 3300 mg |
| ≥ 100 kg | 3000 mg | 3600 mg |

| | | |
|---|---|---|
| ^{a} Body weight as recorded at the last study visit. | | |

After the Initial Evaluation Period, all patients roll over into an Extension Period of up to 2 years during which all patients receive ravulizumab q8w (every eight weeks). The actual time of all dose administrations is recorded in the patient's eCRF.

This is an open-label study. Patients who meet all criteria for enrollment are assigned to study treatment with ravulizumab at the Baseline Visit (Day 1). The interactive voice- or web-response system (IxRS) is used to assign vials containing ravulizumab to each patient.

Infusion of other monoclonal antibodies has been associated with infusion reactions, with onset typically during or shortly after completion of the infusion.

Prior medications (including vitamins and herbal preparations)-including those discussed in the exclusion criteria and procedures (any therapeutic intervention, such as surgery/biopsy or physical therapy) the patient takes or undergoes within 28 days (or 3 years for documentation of meningococcal vaccination) prior to the start of Screening until the first dose of ravulizumab -are recorded on the patient's eCRF.

For analytical purposes, any dialysis in the 14-day period immediately following the first ravulizumab dose is not considered "new dialysis."

All medication use and procedures undertaken during the study are recorded in the patient's source document/medical chart and eCRF. This record includes all prescription drugs, herbal products, vitamins, minerals, over-the-counter medications, and current medications. Concomitant medications are recorded from the first infusion of study drug through 56 days after the patient's last dose of study drug. Any changes in concomitant medications also are recorded in the patient's source document/medical chart and eCRF. Any concomitant medication deemed necessary for the patient's standard of care during the study, or for the treatment of any AE, along with the allowed medications described below are given at the discretion of the Investigator. However, it is the responsibility of the Investigator to ensure that details regarding all medications are recorded in full in the patient's source document/medical chart and eCRF.

Patients are prohibited from receiving any of the following medications and procedures at any time after the first dose of study drug: eculizumab or other complement inhibitors, use of any other investigational drug or device as part of a clinical trial, IVIg (unless for an unrelated medical need *e.g.,* hypogammaglobinemia), Rituximab, PE/PI after first dose, and new dialysis with the first 48-hour period following the first dose of ravulizumab, unless there is a compelling medical need as assessed by (1) hypervolemia unresponsive to diuretics, (2) refractory electrolyte imbalance, or (3) new-onset uremic encephalopathy. Exceptions must be approved prior to administration of dialysis on a case-by-case basis by the Sponsor.

The following concomitant medications and procedures are allowed under certain circumstances and with the following restrictions: use of other immunosuppressive therapies (such as steroids, mTORi [*e.g.,* sirolimus, everolimus], CNI [e.g., cyclosporine or tacrolimus]) prior to screening or during the study are not allowed unless: a) part of an established post-transplant anti-rej ection regime, or b) patient has confirmed anti-complement factor antibodies antibody requiring immunosuppressive therapy, or c) steroids are being used for a condition other than aHUS (*e.g.,* asthma).

Any patients receiving other complement inhibitors (including eculizumab) or undergoing PE/PI after the first dose of study drug are withdrawn from the study.

Due to its mechanism of action, the use of ravulizumab increases the patient's susceptibility to infection. To reduce the risk of infection, all patients are vaccinated against *N. meningitidis,* Hib, and *Streptococcus pneumoniae.*

Patients are vaccinated against *N. meningitidis* within 3 years prior to, or at the time of, receiving the first dose of ravulizumab. Patients who are treated with drug less than 2 weeks after receiving a meningococcal vaccine receive treatment with appropriate prophylactic antibiotics until 2 weeks after vaccination. Vaccines against serotypes A, C, Y, W135, and B, where available, are recommended to prevent common pathogenic meningococcal serotypes. Patients are vaccinated or revaccinated according to current national vaccination guidelines or local practice for vaccination use with complement inhibitors (*e.g.,* eculizumab).

It is recognized that some patients who have not been vaccinated against *N*. *meningiditis* within 3 years prior to receiving the first dose of ravulizumab may not be able to receive a vaccination at the time of the first dose. Patients who have not been vaccinated prior to initiating ravulizumab treatment receive prophylactic antibiotics prior to and for at least 2 weeks after meningococcal vaccination.

Vaccination may not be sufficient to prevent meningococcal infection. Consideration should be given per official guidance and local practice on the appropriate use of antibacterial agents. All patients are monitored for early signs of meningococcal infection, evaluated immediately if infection is suspected, and treated with appropriate antibiotics, if necessary. To increase risk awareness and promote quick disclosure of any potential signs or symptoms of infection experienced by the patients during the course of the study, patients are provided a safety card to carry with them at all times. Additional discussion and explanation of the potential risks, signs, and symptoms occur at specific time points as part of the review of the patient safety card and throughout the study as described in the Schedule of Assessments (Table 1 and Table 2).

Patients are vaccinated against *Haemophilus influenzae* type b (Hib) and *Streptococcus pneumoniae* according to national and local vaccination schedule guidelines, prior to, or at the time of, receiving the first dose of ravulizumab. Vaccination status for *N. meningitidis, Hib,* and *S. pnemoniae* is recorded on the patient's eCRF.

Patients are administered study drug in a controlled setting under the supervision of the Investigator or designee, thereby ensuring compliance with study drug administration. The Investigator or designee ensures that all patients are adequately informed on the specific dosing regimen required for compliance with the study protocol, ensure that the patient receives the appropriate dose at the designated time points during the study and that adequate safety monitoring occurs during the infusion.

Before receiving study drug, female patients who consider themselves to be postmenopausal must provide evidence of menopause based on a combination of amenorrhea for at least 1 year and increased serum follicle-stimulating hormone (FSH) level (> 30 IU/L) (*e.g.,* in the absence of hormone replacement therapy, dietary phytoestrogens).

Female patients of childbearing potential use a highly effective method of contraception (as defined below), starting at Screening and continuing for at least 8 months after the last dose of study drug. Highly effective contraceptive methods* include: hormonal contraception associated with inhibition of ovulation, intrauterine device, intrauterine hormone-releasing system, bilateral tubal occlusion, vasectomized partner (provided that the partner is the patient's sole sexual partner), sexual abstinence (defined as refraining from heterosexual intercourse during the entire period of risk associated with the study drug treatment; reliability of sexual abstinence needs to be evaluated in relation to the duration of the clinical study and the preferred and usual lifestyle of the patient), combination of male condom with either cap, diaphragm, or sponge with spermicide (double barrier methods). Male patients with a female spouse/partner of childbearing potential or a pregnant or breastfeeding spouse or partner agree to use double barrier contraception (male condom plus appropriate barrier method for the female partner) while on treatment and for at least 8 months after the last dose of study drug. Double barrier contraception is required even with documented medical assessment of surgical success of a vasectomy.

Male patients do not donate sperm while on treatment and for at least 8 months after the last dose of study drug.

### 6. Efficacy Assessments

The primary efficacy assessment is Complete TMA Response during the 26-week Initial Evaluation Period. The criteria for Complete TMA Response are (1) normalization of platelet count, (2) normalization of LDH, and (3) ≥ 25% improvement in serum creatinine from baseline.

Patients who meet all Complete TMA Response criteria, confirmed by 2 consecutive measurements obtained at least 4 weeks apart, are classified as having met the primary efficacy endpoint.

The following secondary efficacy assessments are measured during the study:
A. Dialysis requirement status
B. Time to Complete TMA Response
C. Complete TMA Response status over time
D. Observed value and change from baseline in eGFR
E. CKD stage, as evaluated by the Investigator at select target days and classified as improved, stable (no change), or worsened compared to baseline
F. Observed value and change from baseline in hematologic parameters (platelets, LDH, hemoglobin)
G. Increase in hemoglobin of ≥ 20 g/L from baseline, sustained for at least 2 consecutive measurements obtained at least 4 weeks apart
H. Change from baseline in QoL, as measured by EQ-5D-3L (all patients), FACIT Fatigue Version 4 (patients ≥ 18 years of age), and Pediatric FACIT Fatigue (patients < 18 years of age) questionnaires.

### 7. Safety Assessments

The Investigator or his/her designee meet with patients to discuss the potential safety risks of ravulizumab and to give the Investigator the opportunity to address any of the patient's safety concerns regarding the study.

The collection of AEs is monitored from the time informed consent is obtained until study completion. Investigators follow any AEs through to their conclusion (resolution or stabilization). In the event of patient withdrawal from the study, AE monitoring continues through the last patient's last study visit if possible. The timing of the clinical and laboratory assessments is performed per the Schedule of Assessments (Tables 1 and 2). Any clinically significant abnormal results is followed until resolution or stabilization.

A review of demographic parameters, including age, gender, race, and ethnicity is performed. A complete medical history is taken and documented. Weight and height are recorded. Height is measured at Screening only.

The patient's aHUS medical history, including onset of first aHUS symptom and date of diagnosis, is documented at the Screening Visit.

The patient's medical history, including prior and concomitant conditions/disorders, is recorded at the Screening Visit. Medication (prescription or over-the-counter, including vitamins and/or herbal supplements) use over the 28 days (or 3 years for documentation of meningococcal vaccination) prior to the start of Screening is also recorded, in addition to meningococcal vaccination.

A physical examination includes the following assessments: general appearance; skin; head, ear, eye, nose, and throat; neck; lymph nodes; chest; heart; abdominal cavity; limb; central nervous system; and musculoskeletal system. An abbreviated physical examination consists of a body system relevant examination based upon Investigator judgment and patient symptoms. Vital sign measurements are taken after the patient has been resting for at least 5 minutes, and include systolic and diastolic BP (millimeters of mercury [mmHg]), pulse oximetry, heart rate (beats/minute), respiratory rate (breaths/minute), and oral or tympanic temperature (degrees Celsius [°C] or degrees Fahrenheit [°F]).

Samples for serum pregnancy, hematology, chemistry, coagulation, and urinalysis are performed at the times specified in the Schedule of Assessments (Tables 1 and 2). Samples for laboratory assessments are collected before each study drug administration.

Samples collected at Screening can be tested at either a local or central laboratory. If local laboratory tests are used for LDH, platelet count, hemoglobin, and serum creatinine, duplicate samples are collected for central laboratory testing to ensure baseline and postbaseline measurements for analyses are resulted from the central laboratory. In the event of duplicate samples from local and central laboratories, central laboratory results are used for analysis.

It is anticipated that some laboratory values may be outside the normal value range due to the underlying disease. The Investigators should use medical judgment when assessing the clinical significance of these values. Clinical significance is defined as any variation in laboratory measurements that has medical relevance, and which results in a change in medical care. If clinically significant laboratory changes from baseline value are noted, the changes are documented as AEs on the AE eCRF. The Investigator assesses the relationship to study treatment for all clinically significant out-of-range values. The Investigator continues to monitor the patient through additional laboratory assessments until (1) values have returned to the normal range or baseline level, or (2) in the judgment of the Investigator, values that are outside the normal range are not related to the administration of study drug or other protocol-specific procedures.

For females of childbearing potential, a serum or urine pregnancy test (i.e., beta-human chorionic gonadotropin [β-hCG]) are performed according to the Schedule of Assessments (Tables 1 and 2). Blood samples are analyzed for hematology parameters.

Blood samples are analyzed for the serum chemistry parameters. Indirect bilirubin is calculated from total and direct bilirubin values; therefore, indirect bilirubin results are not available if direct bilirubin is below the limit of quantification. Serum FSH level is measured during Screening for postmenopausal female patients to confirm their postmenopausal status.

Chemistry assessments are performed at the time points specified in the Schedule of Assessments Tables 1 and 2). The eGFR is calculated for all visits at which serum chemistries are collected using the Modification of Diet in Renal Disease formula in patients ≥18 years of age and Modification of Diet in Renal Disease Schwartz formula in patients < 18 years of age.

Blood samples are analyzed for coagulation parameters.

Urine samples are analyzed. A microscopic examination of urine samples is performed if the results of the macroscopic analysis are abnormal. Urine samples are also analyzed to measure proteins and creatinine in order to calculate the urine total protein:creatinine ratio.

For each patient, single 12-lead digital ECGs are collected according to the Schedule of Assessments (Tables 1 and 2). Patients must be supine for approximately 5 to 10 minutes before ECG collection and remain supine but awake during ECG collection. The Investigator or designee responsible for reviewing the ECG to assess whether the ECG is within normal limits and to determine the clinical significance of the results. These assessments are indicated on the CRF.

Blood samples are collected to test for presence and titer of ADAs to ravulizumab in serum prior to study drug administration as indicated in the Schedule of Assessments (see Tables 1 and 2). If results of the test are positive, the test can be repeated every 3 months until results become negative or stabilize, based on the measured titer and the safety assessments. Further characterization of antibody responses can be conducted as appropriate, including binding and neutralizing antibodies, PK/PD, safety, and activity of ravulizumab.

An AE is any untoward medical occurrence in a patient administered a pharmaceutical product and which does not necessarily have a causal relationship with this treatment. An AE can therefore be any unfavorable or unintended sign (e.g., an abnormal laboratory finding), symptom, or disease temporally associated with the use of a medicinal product, whether or not considered related to the medicinal product.

Situations in which an untoward medical occurrence did not occur (*e*.*g*" hospitalization for elective surgery if planned before the start of the study, admissions for social reasons or convenience), and anticipated day-to-day fluctuations of pre-existing disease(s) or condition(s) present or detected at the start of the study that do not worsen are not AEs.

Lack of drug effect is not an AE in clinical studies, because the purpose of the clinical study is to establish drug effect.

A medication error (including intentional misuse, abuse, and overdose of the product) or use other than what is defined in the protocol is not considered an AE unless there is an untoward medical occurrence as a result of a medication error.

Cases of pregnancy that occur during maternal or paternal exposure to investigational product are to be reported within 24 hours of Investigator/site awareness. Data on fetal outcome and breastfeeding is collected for regulatory reporting and safety evaluation.

Adverse events are recorded from the time of signed consent. An AE reported after informed consent but before study drug administration is considered a pretreatment AE.

The following events are important identified risks in this study: Meningococcal infections.

The severity of AEs is graded using Common Terminology Criteria for Adverse Events (CTCAE) version 4.03 or higher. A grading (severity) scale is provided for each AE term. Each CTCAE term is a Lowest Level Term (LLT) per the Medical Dictionary for Regulatory Activities (MedDRA^{®}). Each LLT is coded to a MedDRA preferred term. Grade refers to the severity of the AE. The CTCAE assigns a grade of 1 through 5, with unique clinical descriptions of severity for each AE (Table 6).

**Table 6: Adverse Event Severity Grading Scale**

| **Grade** | **Description** |
|---|---|
| Grade 1 | Mild; asymptomatic or mild symptoms; clinical or diagnostic observations only; intervention not indicated |
| Grade 2 | Moderate; minimal, local or noninvasive intervention indicated; limiting age-appropriate instrumental activities of daily living (ADL)^{a} |
| Grade 3 | Severe or medically significant, but not immediately life-threatening; hospitalization or prolongation of hospitalization indicated; disabling; limiting self-care ADL^{b} |
| Grade 4 | Life-threatening consequences; urgent intervention indicated. |
| Grade 5 | Death related to AE. |

| | |
|---|---|
| Abbreviations: ADL = activities of daily living; AE = adverse event ^{a} Instrumental ADL refers to preparing meals, shopping for groceries or clothes, using the telephone, managing money, etc. ^{b} Self-care ADL refers to bathing, dressing and undressing, feeding self, using the toilet, taking medications, and not bedridden. | |

Any change in the severity of an AE is documented based on specific guidelines in the eCRF Completion Guidelines. Severity and seriousness are differentiated: severity describes the intensity of an AE, while the term seriousness refers to an AE that has met specific criteria for a serious adverse event (SAE).

An Investigator must provide a causality assessment (Unrelated, Unlikely, Possible, Probable, or Definite) for all AEs (both serious and nonserious) based upon the Investigator's medical judgment and the observed symptoms associated with the event (Table 7). This assessment is recorded on the eCRF and any additional forms as appropriate.

**Table 7: Causality Assessment Descriptions**

| **Assessment** | **Description** |
|---|---|
| Not Related/Unrelated | Suggests that there is no causal association between the investigational product and the reported event. |
| Unlikely Related | Suggests that the clinical picture is highly consistent with a cause other than the investigational product but attribution cannot be made with absolute certainty and a relationship between the investigational product and AE cannot be excluded with complete confidence. |
| Possibly Related | Suggests that treatment with the investigational product may have caused or contributed to the AE (ie, the event follows a reasonable temporal sequence from the time of drug administration and/or follows a known response pattern to the investigational product but could also have been produced by other factors). |
| Probably Related | Suggests that a reasonable temporal sequence of the event with the investigational product administration exists and the likely causal association of the event with the investigational product. This will be based upon the known pharmacological action of the investigational product, known or previously reported adverse reactions to the investigational product or class of drugs, or judgment based on the Investigator's clinical experience. |
| Definitely Related | Temporal relationship to the investigational product, other conditions (concurrent illness, concurrent medication reaction, or progression/expression of disease state) do not appear to explain event, corresponds with the known pharmaceutical profile, improvement on discontinuation, reappearance on re-challenge. |

A serious adverse event (SAE) is any untoward medical occurrence that:
- Results in death
- Is life-threatening (i.e., patient was at risk of death at the time of the event)
- Requires inpatient hospitalization or prolongation of existing hospitalization
- Results in persistent or significant disability/incapacity
- Is a congenital anomaly/birth defect

Important medical events that may not result in death, be immediately life-threatening, or require hospitalization, may be considered a serious adverse event when, based upon appropriate medical judgment, they may jeopardize the patient or may require intervention to prevent one of the outcomes listed above.

Suspected unexpected serious adverse reactions (SUSARs) are serious events that are not listed in the IB and that the Investigator identifies as related to investigational product or procedure. United States Title 21 Code of Federal Regulations (CFR) 312.32 and European Union Clinical Trial Directive 2001/20/EC and the associated detailed guidances or national regulatory requirements in participating countries require the reporting of SUSARs.

All AEs (serious and nonserious) are collected from the signing of the ICF until 60 days after the last dose of study drug for patients with ET or until 56 days after the last dose of study drug for patients who complete the study. All AEs are recorded on the eCRF upon the Investigator or his/her staff becoming aware of their occurrence.

All SAEs are recorded regardless of the Investigator's assessment of causality. No time limit exists on reporting SAEs that are thought to be causally related to the study drug. Investigators are at liberty to report SAEs irrespective of causality at any time.

For all SAEs, the Investigator must provide the following: appropriate and requested follow-up information, causality of the SAE(s), treatment of/intervention for the SAE(s), outcome of the SAE(s), and supporting medical records and laboratory/diagnostic information.

Pregnancy data is collected during this study for all patients and female spouse/partner of male patients. Exposure during pregnancy (also referred to as exposure in utero) can be the result of either maternal exposure or transmission of drug product via semen following paternal exposure. Pregnancy in itself is not regarded as an AE unless there is a suspicion that the investigational product may have interfered with the effectiveness of a contraceptive medication. However, complications of pregnancy and abnormal outcomes of pregnancy are AEs and may meet the criteria for an SAE (e.g., ectopic pregnancy, spontaneous abortion, intrauterine fetal demise, neonatal death, or congenital anomaly). Elective abortions without complications should not be reported as AEs.

### 8. Pharmacokinetics and Pharmacodynamics Assessments

Blood samples for determination of serum drug concentrations and PD assessments are collected before and after administration of study drug at the time points indicated in the Schedule of Assessments (see Tables 1 and 2). The actual date and time (24-hour clock time) of each sampling is recorded. The number of PK sampling time points for any given patient does not exceed the currently planned number of time points.

The blood samples for PK and PD assessment are collected from the arm opposite to the arm used for infusing drug. Assessments for PK/PD are as follows: (1) changes in serum ravulizumab concentration over time and (2) changes in free C5 concentrations.

### 9. Exploratory Assessments

For exploratory biomarker analyses, summary statistics are presented for actual, change and percentage change from baseline.

The relationship between ravulizumab concentration and exploratory biomarkers or the correlation between clinical benefit and key exploratory biomarkers can be assessed by graphical display. Exploratory analysis and potential relationships between clinical outcomes, PK/PD, genetic profile, and biomarker levels can also be performed. APC activity and autoantibody results are summarized if evaluated.

Exploratory genetics can be performed to investigate genetic variants in genes known to be associated with aHUS, as well as to identify novel genetic variants associated with aHUS, complement dysregulation, or metabolism or efficacy of ravulizumab.

Genetic mutations of known clinical relevance in aHUS are communicated to the patient or patient's guardian by Investigator together with appropriate genetic counseling. Genetic variants of unknown clinical significance are not be communicated to patients or their Investigator.

Additional signs or symptoms of aHUS are assessed using the Resource Utilization Patient Questionnaire and Patient-reported aHUS Symptoms Questionnaire.

Components of extra-renal signs or symptoms of aHUS, including vital signs and clinical laboratories, can be summarized descriptively at baseline and postbaseline time points and for changes from baseline. By-patient listings can be provided.

Analysis for signs, symptomology, and resource utilization can include standard approaches to categorical outcomes with or without repeated measures.

If a Day 1 assessment is missing, the Screening assessment is used as the baseline assessment.

For evaluation of Complete TMA Response during the 26-week Initial Evaluation Period (primary endpoint), patients missing an efficacy assessment that is part of the definition of Complete TMA Response while still on-study, have their last observation carried forward (LOCF). For patients who have discontinued from the study prior to Week 26, their data up to the time of discontinuation is used to assess Complete TMA Response.

Missing data for QoL instruments is handled as specified in the instructions for each instrument.

An interim analysis is planned for this study at the end of the 26-week Initial Evaluation Period after all patients have completed or withdrawn from the 26-week Initial Evaluation Period. Additionally, a second analysis to summarize long-term efficacy, safety, and PK parameters is performed at the end of the 2-year Extension Period.

### EXAMPLE 2: Data for Phase 3, Single Arm, Multicenter Study of ravulizumab (ALXN1210) in Complement Inhibitor-Naive Adult Patients with Atypical Hemolytic Uremic Syndrome (aHUS)

The following is a summary of data from a single arm study of ravulizumab (ALXN1210-aHUS-311) in complement inhibitor treatment-naive patients with atypical hemolytic uremic syndrome (aHUS), that was conducted substantially according to the protocol described above in Example 1. The initial evaluation period was 26 weeks, followed by an extension period of up to 2 years. The study design is shown in Figure 1.

The objective of the study was to assess the efficacy of ravulizumab in complement inhibitor treatment-naive adult patients with aHUS to inhibit complement-mediated thrombotic microangiopathy (TMA) as characterized by thrombocytopenia, hemolysis, and renal impairment. The primary endpoint was complete TMA response within the initial 26 weeks evaluation period. The primary, secondary, and safety endpoints for the study are summarized in Figure 2. A summary of the inclusion and exclusion criteria is set forth in Figure 3.

The enrollment requirement was (1) at least 6 adolescents (deferred to ALXN1210-aHUS-312), (2) at least 10 patients with prior kidney transplant (8 enrolled), and (3) at least 30 patients meeting TMA lab criteria at Day 1 based on central lab results (32 enrolled).

The data cut includes data from the initial evaluation period on all patients, plus any available extension period data up to October 2019.

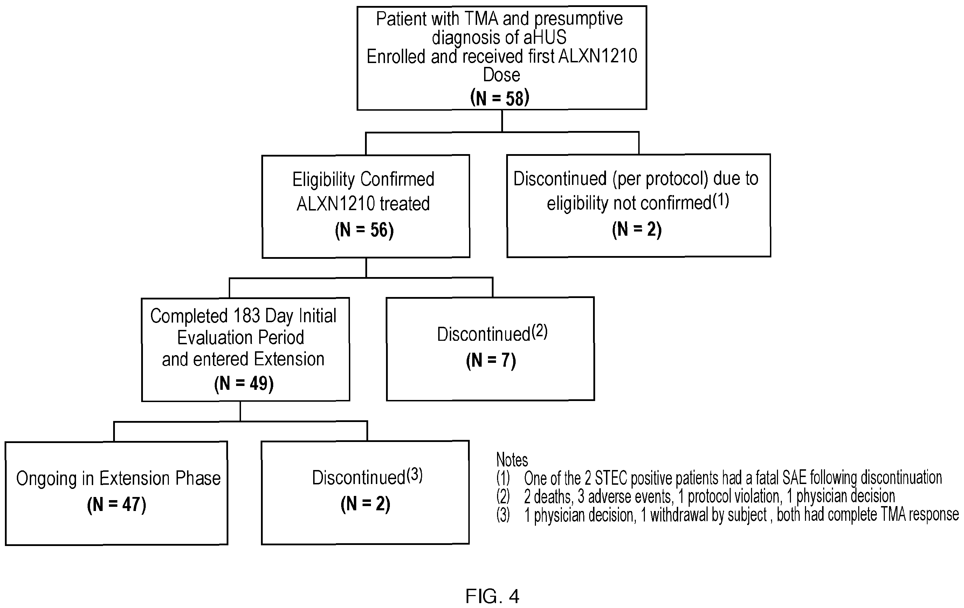

Fifty-eight (58) subjects were enrolled and received at least one dose (included in safety set), two subjects were enrolled and replaced (deemed ineligible post-first dose and discontinued as pre-specified in protocol), fifty-six (56) subjects were in the full analysis set, eleven (11) subjects discontinued treatment, and nine (9) subjects discontinued from the study. A schematic of the patient disposition is set forth in Figure 4. Treatment compliance was 100%. The baseline demographics are set forth in Table 8, the baseline disease characteristics are set forth in Table 9, and the baseline laboratory values are set forth in Table 10.

**Table 8: Baseline Demographics**

| **Variable** | | **Statistics** | **Overall (N=56)** |
|---|---|---|---|
| Age at Time of First Infusion (yrs) | | Mean (SD) | 40.1 (19.5-76.6) |
| | Category | n(%) | |
| | 18 to < 30 years | | 11 (19.6) |
| | 30 to < 40 years | | 17 (30.4) |
| | 40 to < 50 years | | 15 (26.8) |
| | 50 to < 60 years | | 5 (8.9) |
| | >= 60 years | | 8 (14.3) |
| | | | |
| Sex: males | | n (%) | 19 (33.9) |
| | | | |
| Ethnicity, Not Hispanic or Latino | | n (%) | 41 (73.2) |
| | | | |
| Race | | n (%) | |
| | Asian | | 15 (26.8) |
| | White | | 29 (51.8) |
| | Unknown | | 8 (14.3) |
| | Other | | 4 (7.1) |
| | | | |
| Weight at Time of First Infusion (kg) | | Mean (SD) | 72.9 (17.6) |

**Table 9: Baseline Disease Characteristics**

| **Variable** | | **Statistics** | **Overall (N=56)** |
|---|---|---|---|
| Age(years) at Time of First aHUS Symptoms | | Mean (SD) | 41.5 (15.8) |
| | | | |
| Pre-treatment Extra-Renal Signs or Symptoms of aHUS | | n(%) | 52 (92.9) |
| | | | |
| Kidney Transplant Prior to Entering the Study | | n(%) | 8 (14.3) |
| | | | |
| CKD Stage at Baseline | | n(%) | |
| | 1 | | 0 (0.0) |
| | 2 | | 3 (5.4) |
| | 3a | | 1 (1.8) |
| | 3b | | 2 (3.6) |
| | 4 | | 9 (16.1) |
| | 5 | | 40 (71.4) |
| missing | | | 1 (1.8) |
| | | | |
| Dialysis Within 5 Days of First Dose | | n(%) | 29 (51.8) |

**Table 10: Baseline Laboratory Values**

| **Variable** | **Statistics** | **Overall (N=56)** |
|---|---|---|
| Baseline Platelets (x 10⁹/L) | Mean (SD) | 118.5 (86.44) |
| | Min, Max | 18, 473 |
| Baseline LDH (U/L) | Mean (SD) | 702.4 (557.96) |
| | Min, Max | 229.5, 3249 |
| Baseline Creatinine (umol/L) | Mean (SD) | 362.5 (240.26) |
| | Min, Max | 51, 1027 |
| Baseline eGFR (mL/min/1.73m²⁾ | Mean (SD) | 15.9 (14.8) |
| | Min, Max | 4, 80 |
| Baseline HGB (g/L) | Mean (SD) | 86.3 (14.87) |
| | Min, Max | 60.5, 140 |
| Met TMA criteria (central Lab) at Day 1 | n(%) | 32 (57.1) |

| | | |
|---|---|---|
| TMA criteria: Platelet count < 150 × 109/L, LDH ≥ 1.5 × upper limit of normal (ULN), Hemoglobin ≤ lower limit of normal (LLN), and Serum creatinine level ≥ ULN. | | |

The TMA response definitions are set forth in Table 11. A Response was achieved when all criteria were concurrently met, and each criterion was met for at least 28 days. Platelet values obtained from the day of a blood transfusion of platelets through 3 days after the transfusion were excluded from all analyses. All serum creatinine values obtained while a patient was on dialysis were excluded from all analyses. When a patient was on dialysis at baseline, then the first valid creatinine value used as the baseline value was the first assessment ≥ 6 days post-dialysis. If a patient was on dialysis during the entire 26-week initial evaluation period, then the baseline creatinine was not calculated.

**Table 11: Complete TMA Response Definitions**

| **Response Endpoint** | **Platelet criterion** | **LDH criterion** | **Kidney function criterion** |
|---|---|---|---|
| **Complete TMA response (Primary)** | Normalization 9 (≥150 x 10⁹ /L) | Normalization (≤246 U/L) | ≥25% improvement from BL in serum creatinine |
| **Modified complete TMA response (secondary)** | Normalization | Normalization | Patients OFF dialysis at BL: |
| | | | ≥25% improvement from baseline in serum creatinine |
| | | | Patients ON dialysis at BL: |
| | | | Off dialysis |
| **Hematologic normalization and renal function preservation (Primary endpoint in C10-004, reported in AJKD)** | Normalization | Normalization | Decrease in creatinine or increase less than 25% from BL |

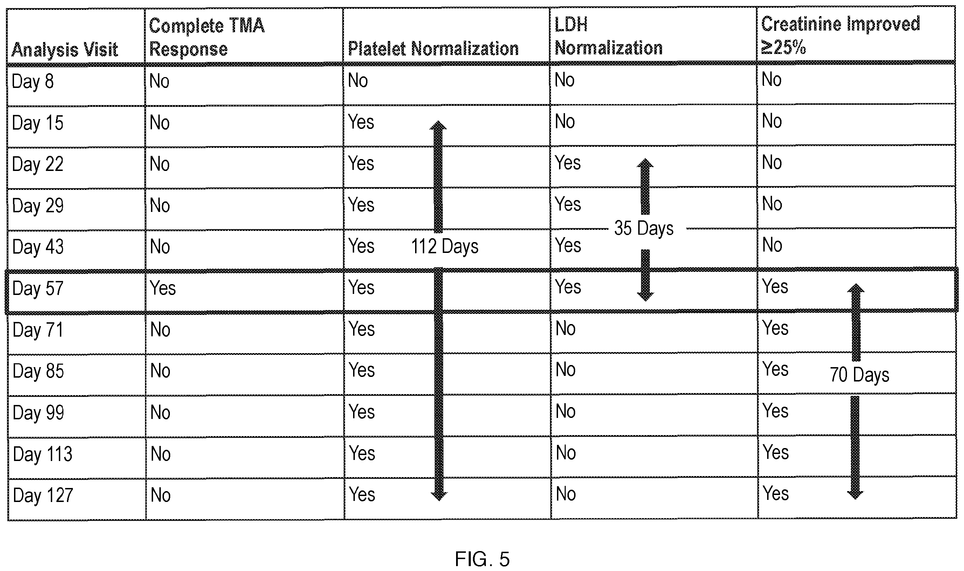

The key efficacy results are set forth in Tables 12-14. Figure 5 is a derivation example showing the results of a confirmed complete TMA response at Day 57, including platelet normalization for 112 days, LDH normalization for 35 days, and creatinine improvement ≥ 25% for 70 days.

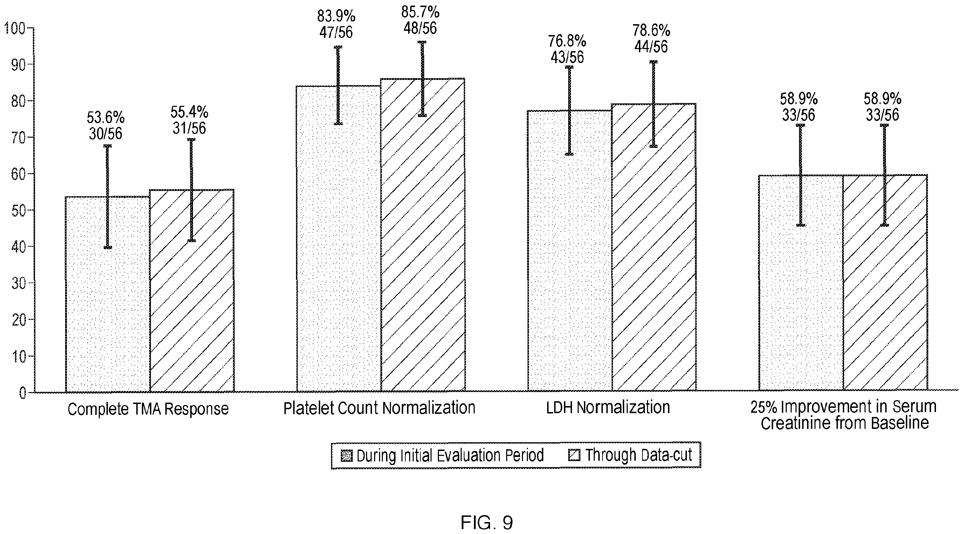

The criteria for Complete TMA Response were met when all criteria were concurrently met, and each criterion was met for at least 28 days. Components of complete TMA response, as well as other secondary efficacy endpoints, show consistent response to treatment. As shown in Figure 9, 53.6% (30/56) of patients achieved a complete TMA response during the initial evaluation period. 33.9% (19/56) of patients had a partial response.

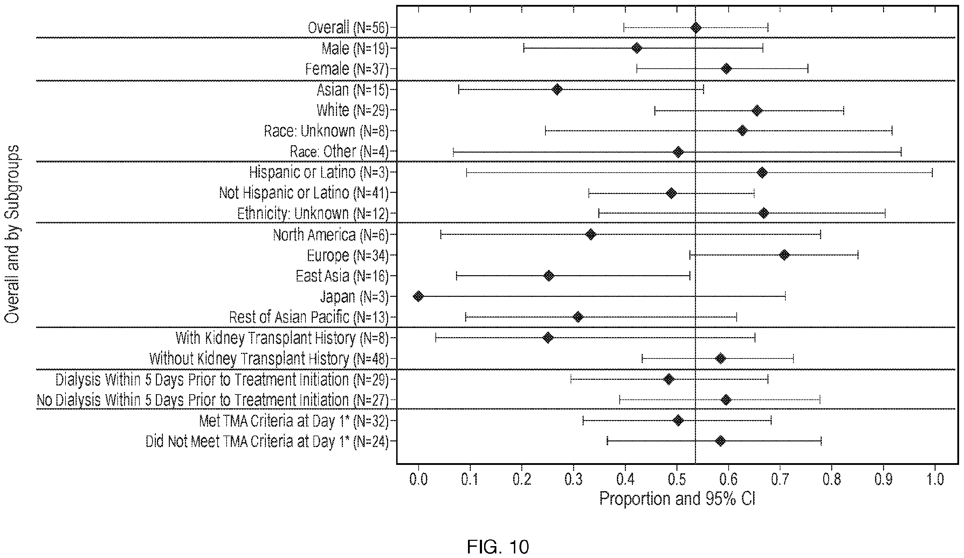

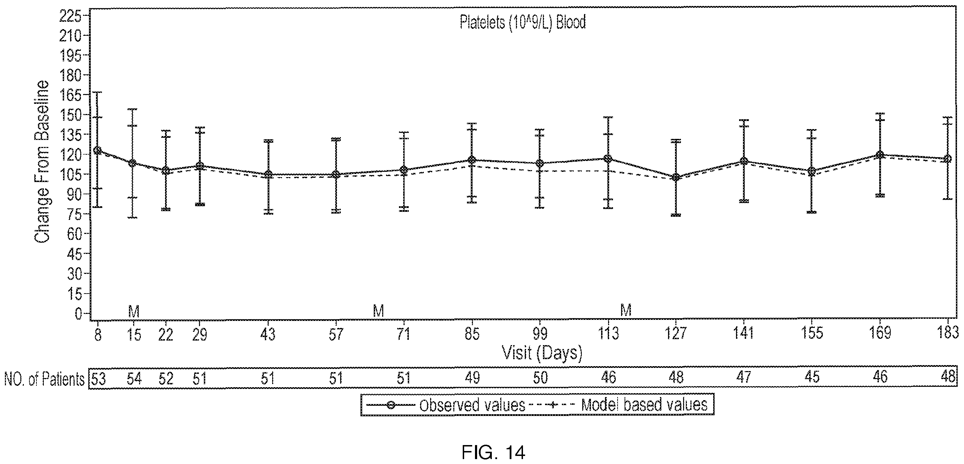

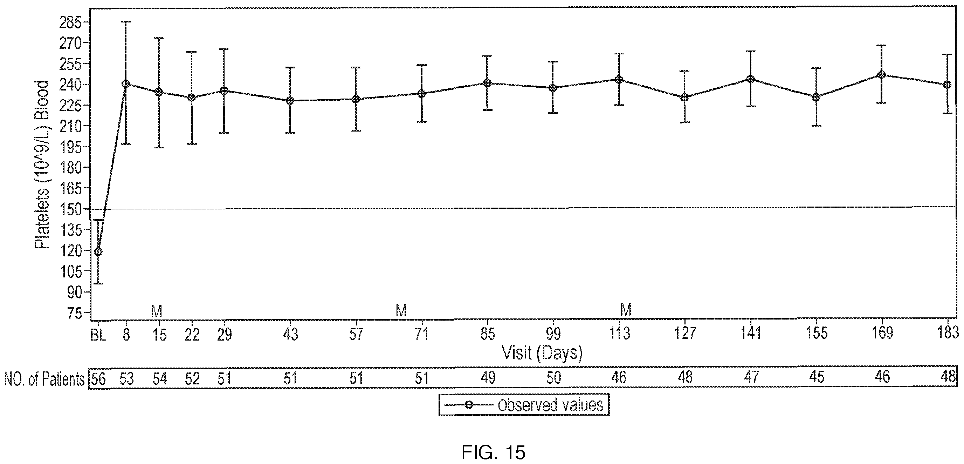

Hematologic normalization includes concurrent normalization of platelet count and normalization of LDH. Each criterion was met for at least 28 days. 73.2% (41/56) of subjects achieved hematologic normalization during the initial evaluation period (see Table 12). 83.9% (47/56) of subjects achieved platelet count normalization during the initial evaluation period (see Table 12, Figure 10, Figure 14, and Figure 15).

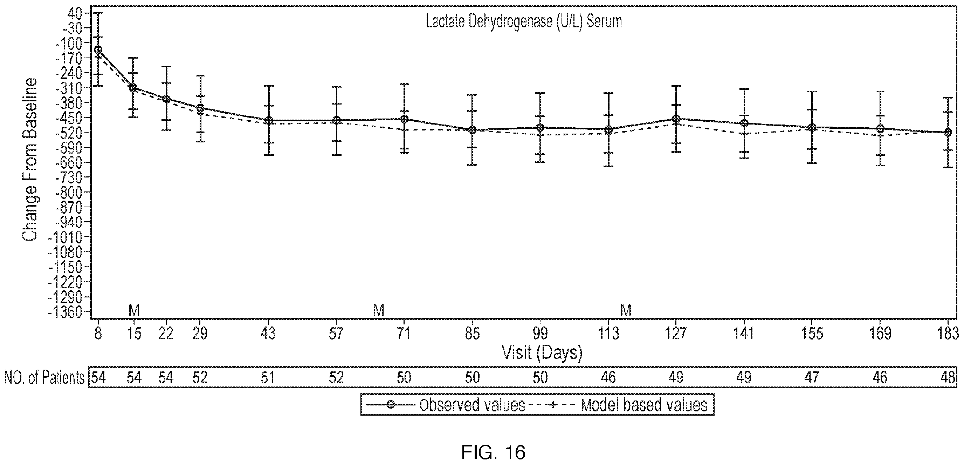

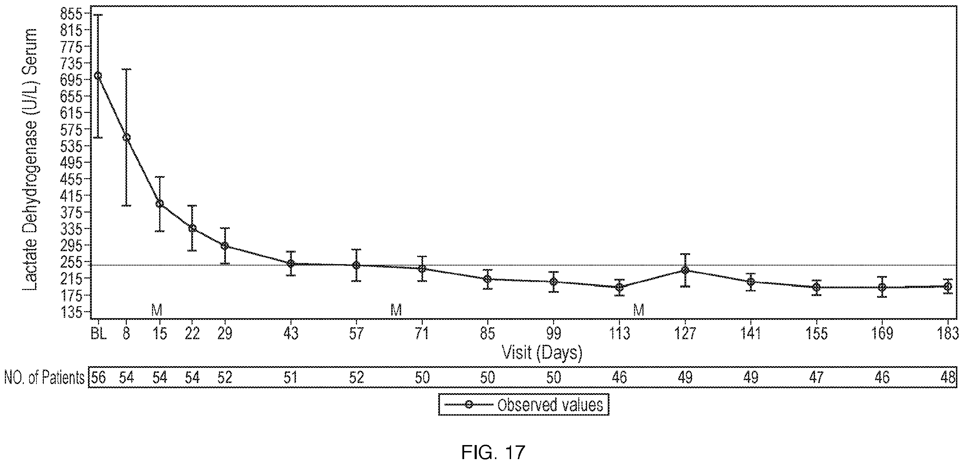

76.8% (43/56) of subjects achieved LDH normalization during the initial evaluation period (see Table 12, Figure 10, Figure 16, and Figure 17).

**Table 12: Key Efficacy Results: Primary Complete TMA Response During Primary Evaluation Period**

| | | | **Responder** | |
|---|---|---|---|---|
| | | **Total** | **n** | **Proportion (95% CI)** |
| **Complete TMA Response** | | 56 | 30 | 0.536 (0.396, 0.675) |
| | **Platelet Count Normalization** | 56 | 47 | 0.839 (0.734, 0.944) |
| | **LDH Normalization** | 56 | 43 | 0.768 (0.648, 0.887) |
| | **25% Improvement in Serum Creatinine from Baseline** | 56 | 33 | 0.589 (0.452, 0.727) |
| **Hematologic Normalization** | | 56 | 41 | 0.732 (0.607, 0.857) |

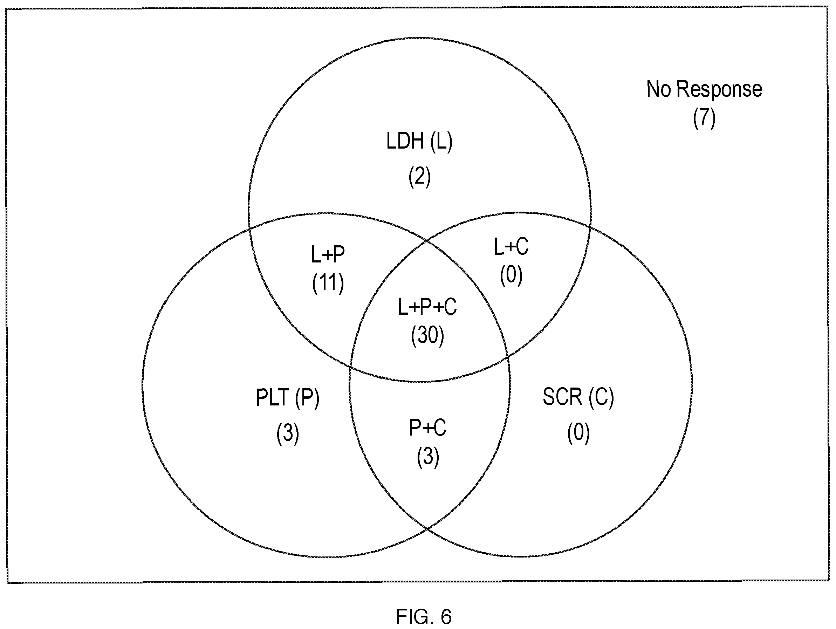

As shown in Figure 6, there were thirty (30) complete TMA responders. Forty (40) of the fifty-six (56) subjects (71.4%) achieved a hemoglobin (HGB) response (≥ 20 g/L increase). Of the thirty (30) complete TMA responders, four (4) did not have a hemoglobin (HGB) response. Figure 10 shows the complete TMA response overall broken down by subgroups during the 26-week initial evaluation period.

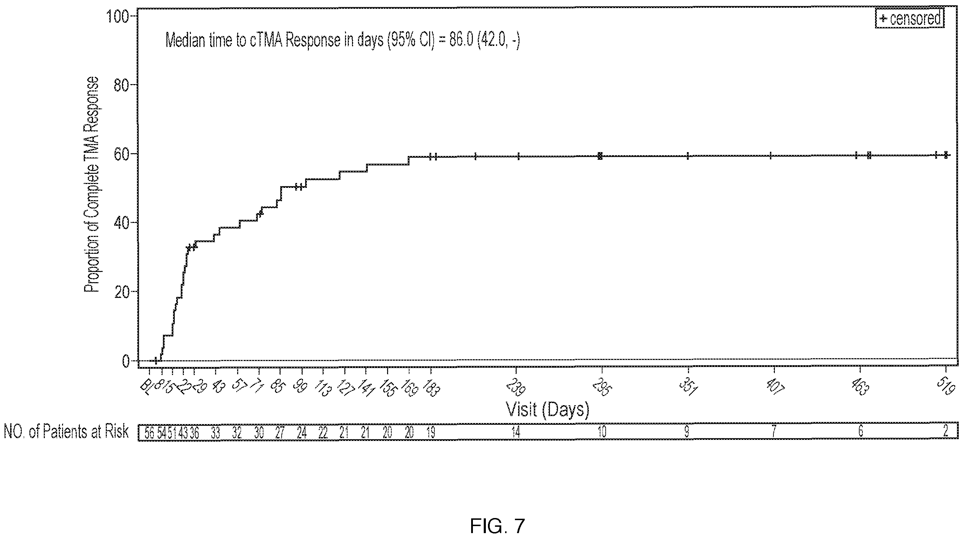

Figure 7 shows the time to complete TMA response. The median time to complete TMA response was 86 days. Patients that did not have a response were censored at the date of last visit or study discontinuation.

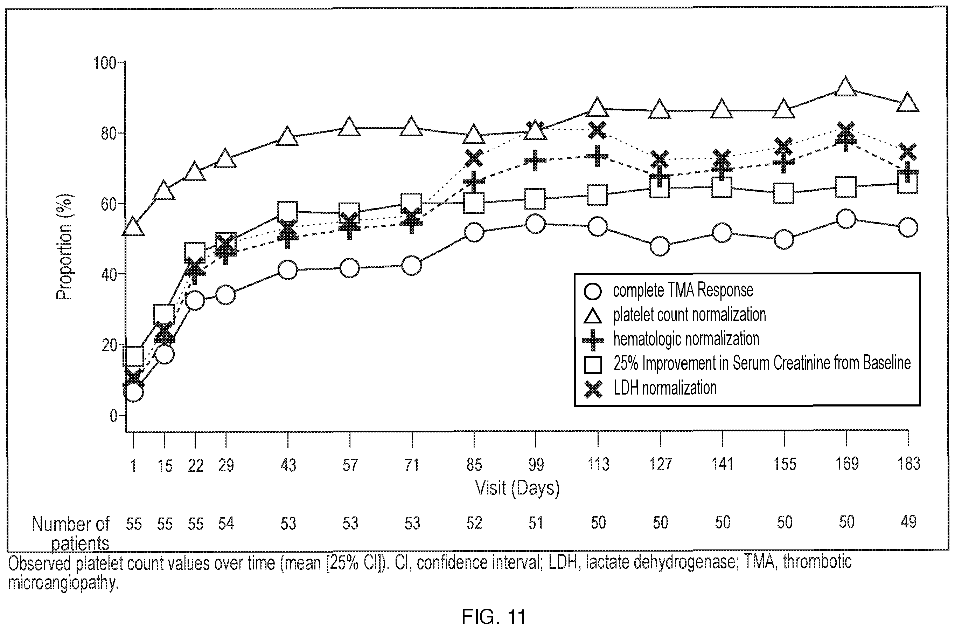

Figure 11 shows the complete TMA status over time (open circle), including platelet count normalization (open triangle), hematologic normalization (+), 25% improvement in serum creatinine from baseline (open square), and LDH normalization (X).

There were seven (7) non-responders. The data for the non-responders is set forth in Table 13.

**Table 13: Key Efficacy Results: Non-responders (0/3 components) During Primary Evaluation Period**

| **Demographic** | **Frequency (N=7)** | | **Comments** |
|---|---|---|---|
| **Asian** | | 5 | |
| **Male** | | 4 | |
| **CKD @ Baseline** | | • 6: Stage 5 | |
| | | • 1: Stage 4 | |
| **Age (yrs)** | 76, 74, 73, 67, 57, 57, 46 | | Older part of distribution |
| **Prior Kidney Transplant** | | 2 | |
| **Discontinuations** | | 6 | 0044-602, 0747-601 |
| | | • 2 deaths | 0573-602, 0738-602 |
| | | • 2 AEs | 044-603 |
| | | • 1 Physician Decision | 044-604 |
| | | • 1 Protocol Violation | |
| **Dialysis** | | | |
| **Baseline** | | 5 | |
| **End of FUP** | | 6 | 1 remained OFF (044-604). |
| | | | Had Normal PTLS at D22, 85, and 99. Reduction in LDH but not normal, Reduction in SCR but <25%. PV was due to receiving fresh plasma. |

As shown in Table 14, 58.6% (17/29) patients who were ON dialysis at baseline were weaned off by the last available follow-up. Of the 27 patients who were off dialysis at baseline, 21 (78%) remained off dialysis at last follow up.

**Table 14: Key Efficacy Results: Dialysis Status Over Time**

| | | **Dialysis Status at Last FUP** | | **Total** |
|---|---|---|---|---|
| | | ON | OFF | |
| Dialysis Status at Baseline | ON | 12 | 17 | 29 |
| | OFF | 6 | 21 | 27 |
| Total | | 18 | 38 | 56 |

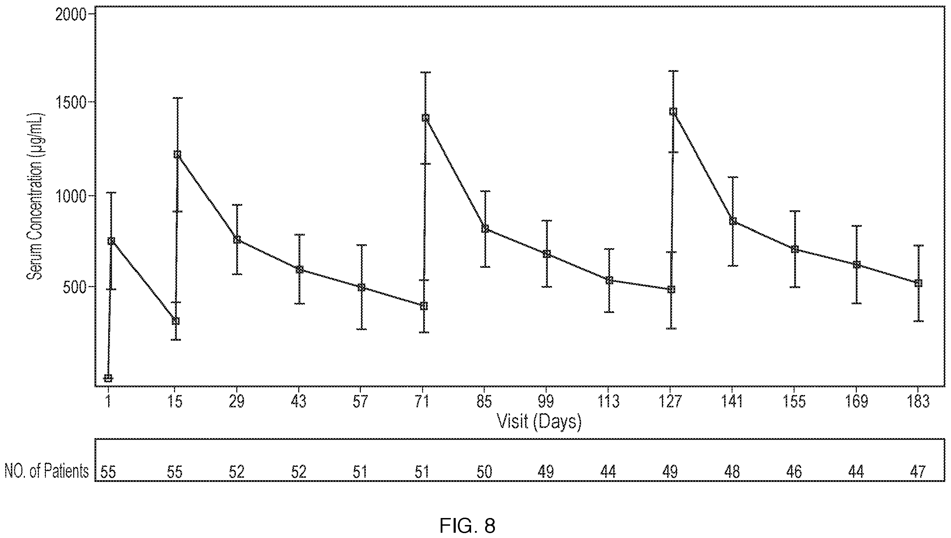

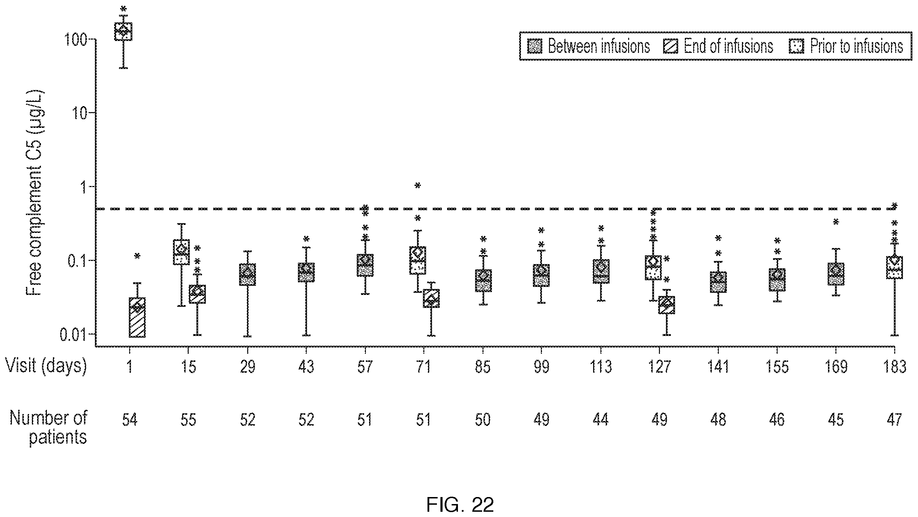

With respect to pharmacokinetics/pharmacodynamics, 99.53% of all free C5 results obtained after the first dose through the initial evaluation period were ≤ 0.5 mg/mL, the defined threshold for terminal complement inhibition (see Figure 22). Weight based dosing resulted in maximal, steady state and trough exposures as predicted with no unexpected pharmacokinetic findings (see Figure 8).

An overview of the key safety results is set forth in Table 15. Patients evaluated for safety include all patients that received ≥ 1 dose of the study drug (N=58). Two of these patients were excluded from the efficacy analysis per protocol due to ineligibility (identification of STEC-HUS).

**Table 15: Key Safety Results: Overview of Key Safety**

| | **Overall (N=58)** | |
|---|---|---|
| Adverse Event Categories | n (%) | Events |
| Any Adverse Event (AE) | 58 (100.0) | 818 |
| -Treatment Related | 20 (34.5) | 58 |
| -Not Treatment Related | 58 (100.0) | 760 |
| Any Serious Adverse Event (SAE) | 30 (5 1.7) | 71 |
| Fatal TEAEs ⁽¹⁾ | 3 (5.2) | 3 |
| Deaths, Pretreatment | 1(1.7) | 1 |
| TEAEs Resulting in Drug d/c | 3 (5.2) | 3 |
| TESAEs Resulting in Drug d/c | 3 (5.2) | 3 |
| TEAEs Resulting in Study d/c | 3 (5.2) | 3 |
| TESAEs Resulting in Study d/c | 3 (5.2) | 3 |
| TEAEs During Study Drug Infusion | 4 6.9) | 6 |
| TESAEs During Drug Infusion | 0 (0.0) | 0 |
| Meningococcal infections | 0 (0.0) | 0 |
| Related AEs | 20 (34.5) | 58 |
| Related SAEs | 2 (3.4) | 2 |

| | | |
|---|---|---|
| (1) Overall, there were 4 deaths observed: 1 from pre-treatment AE (Cerebral arterial thrombosis), 3 from non-related treatment emergent AEs where 2 were septic shock and 1 Intracranial hemorrhage) | | |

There were four deaths: 1 from pre-treatment adverse event (Cerebral arterial thrombosis) and 3 from non-related treatment emergent adverse events (2 septic shock, 1 intracranial hemorrhage). A summary of the deaths in patients having received a minimum of one dose of ravulizumab is set forth in Table 16.

**Table 16: Summary of Deaths in Patients Who Received a Minimum of One Ravulizumab Dose**

| **Cause of Death** | **Age** | **Time on Treatment** | **Key Timepoint** |
|---|---|---|---|
| Cerebral Artery Thrombosis | 77 | Patient received 1 dose but was excluded from efficacy analysis due to positive Shigo toxin test | Prior to the first dose: in ICU for cerebral arterial thrombosis and seizures. |
| | | | On day of the first dose: receiving mechanical ventilation. |
| | | | Seizures and cortical infarcts approximately 10 days later, supportive care was withdrawn. |
| | | | Death: Day 15 |
| Septic Shock | 76 | Patient received 2 doses of study drug | Prior to the first dose: Recent shock (septic or hypovolemic). |
| | | | ARDs, and multiple infections. Patient was on antibiotics, cardiovascular medications, insulin, sirolimus, prednisolone and inotropes. |
| | | | On day of the first dose: receiving mechanical ventilation. |
| | | | Day 6: new septic shock due to Corynebocterium and Candido lusitaniae in the catheter (tip taken for culture prior to the first dose). |
| | | | Death: Day 25 |
| Septic Shock | 73 | Patient received 1 dose | Prior to the first dose: Recent ischemic stroke, encephalopathy, respiratory failure, and on multiple antibiotics for infection. |
| | | | On day of the first dose: receiving mechanical ventilation, pseudomonas in pulmonary aspirate. |
| | | | Onset of Septic shock: Day 2 |
| | | | Death: Day 3 |
| Cerebral Artery Thrombosis | 46 | Patient received 3 doses of study drug | Prior to the first dose: uncontrolled hypertension; CKD Stage 5, requiring dialysis at initiation of study drug; thrombocytopenia, anemia, and hypercalcemia. |
| | | | Day 93: patient was admitted with loss of consciousness. Right intraventricular hemorrhage and intracranial hemorrhage were identified. Following surgery, hypertension and loss of consciousness persisted, and supportive care was withdrawn. |
| | | | Death: Day 107 |

As shown in Table 17, the most frequent adverse events were headache (N=21), diarrhea (N=18), vomiting (N=15), nausea (N=13) and hypertension (N=13). As shown in Table 18, the most common serious adverse event was pneumonia (N=3). Three subjects discontinued the study due to an adverse event. There were no meningococcal cases.

**Table 17: Key Safety Results: Overview of Key Safety [AEs Present in At Least 4 Patients]**

| | **Overall (N=58)** | |
|---|---|---|
| **Preferred Term** | n (%) | E |
| Any AE | 58 (100.0) | 818 |
| Diarrhoea | 18 (31.0) | 24 |
| Vomiting | 15 (25.9) | 18 |
| Nausea | 13 (22.4) | 16 |
| Constipation | 8 (13.8) | 10 |
| Abdominal pain | 7 (12.1) | 10 |
| Dyspepsia | 4 ( 6.9) | 4 |
| Headache | 21 (36.2) | 28 |
| Dizziness | 4 (6.9) | 4 |
| Urinary tract infection | 10 (17.2) | 21 |
| Nasopharyngitis | 8 (13.8) | 12 |
| Pneumonia | 4 (6.9) | 5 |
| Pyrexia | 10 (17.2) | 11 |
| Oedema peripheral | 9 (15.5) | 13 |
| Fatigue | 7 (12.1) | 8 |
| Pain | 4 ( 6.9) | 5 |
| Cough | 10 (17.2) | 10 |
| Dyspnoea | 10 (17.2) | 13 |
| Hypokalaemia | 9 (15.5) | 18 |
| Hyperkalaemia | 4 (6.9) | 8 |
| Vitamin D deficiency | 4 (6.9) | 4 |
| Alopecia | 6 (10.3) | 6 |
| Dry skin | 6 (10.3) | 6 |
| Rash | 5 (8.6) | 5 |
| Hypertension | 13 (22.4) | 20 |
| Hypotension | 4 (6.9) | 4 |
| Arthralgia | 10 (17.2) | 12 |
| Back pain | 6 (10.3) | 6 |
| Muscle spasms | 5 ( 8.6) | 5 |
| Pain in extremity | 5 ( 8.6) | 6 |
| Alanine aminotransferase increased | 5 (8.6) | 7 |
| Aspartate aminotransferase increased | 4 (6.9) | 4 |
| Anaemia | 8 (13.8) | 8 |
| Thrombocytopenia | 4 (6.9) | 4 |
| Anxiety | 8 (13.8) | 12 |
| Insomnia | 4 (6.9) | 4 |
| Vision blurred | 4 (6.9) | 4 |

**Table 18: Key Safety Results: Overview of Key Safety [SAEs Present in At Least 2 Patients]**

| | | **Overall (N=58)** | |
|---|---|---|---|
| **Preferred Term** | | **n (%)** | **E** |
| | Any SAE | 30 (51.7) | 71 |
| | Pneumonia | 3 (5.2) | 3 |
| | Septic shock | 2 (3.4) | 2 |
| | Urinary tract infection | 2 (3.4) | 4 |
| | Atypical hemolytic uremic syndrome | 2 (3.4) | 2 |
| | Hypertension | 3 (5.2) | 5 |
| | Malignant hypertension | 2 (3.4) | 5 |

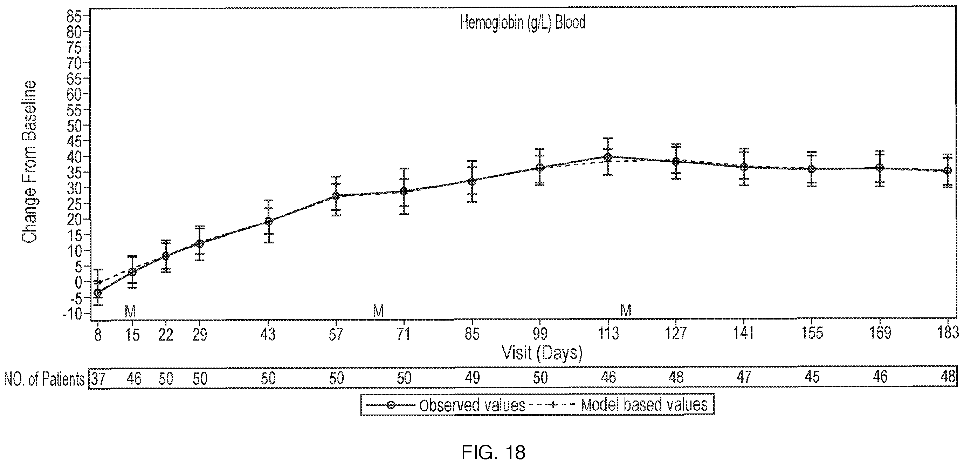

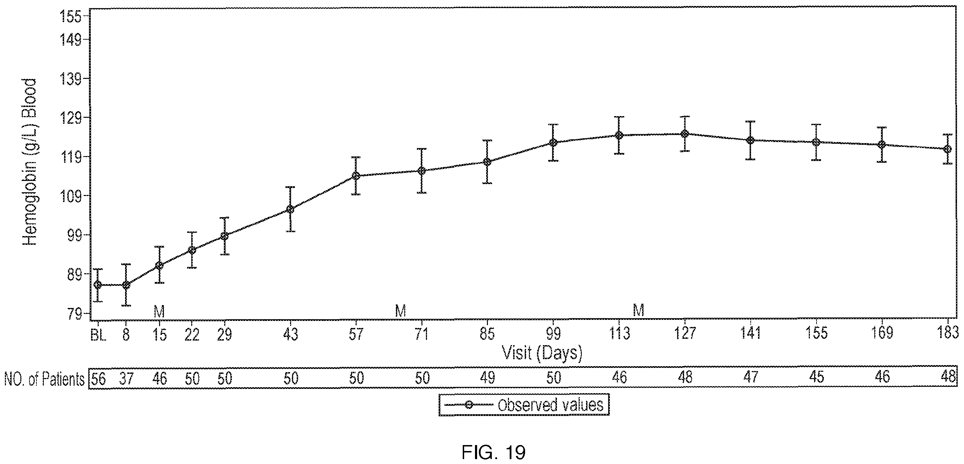

71.4% (40/56) of subjects achieved hemoglobin (HGB) response during the initial evaluation period (see Table 19 and Figure 6). Figure 18 shows the observed and model based mean change in HGB from baseline and 95% confidence interval over time. Figure 19 shows the observed mean HGB and 95% confidence interval over time.

**Table 19: Key Efficacy Results: HGB Response (Increase from Baseline of HGB ≥ With Confirmatory Result)**

| | **N Proportion (95%CI)** |
|---|---|
| HGB Responses; Week 26 | 40 0.714 (0.587, 0.842) |
| HGB Responses; Data Cut-Off or EOS | 43 |
| | 0.768 (0.648, 0.887) |

Chronic kidney disease (CKD) stage is classified based on the National Kidney Foundation Chronic Kidney Disease Stage. The stages of CKD and corresponding estimated glomerular filtration rate (eGFR) values are as follows: Stage 1: eGFR >=90 (normal), Stage 2: eGFR 60-89, Stage 3A: eGFR 45-59, Stage 3B: eGFR 30-44, Stage 4: eGFR 15-29, and Stage 5: eGFR <15 (including dialysis: End stage). Stage 1 is considered the best category. Stage 5 is considered the worst category. An improvement in eGFR (e.g., > 15) corresponds with an improvement in CKD stage (e.g., a lower CKD stage).

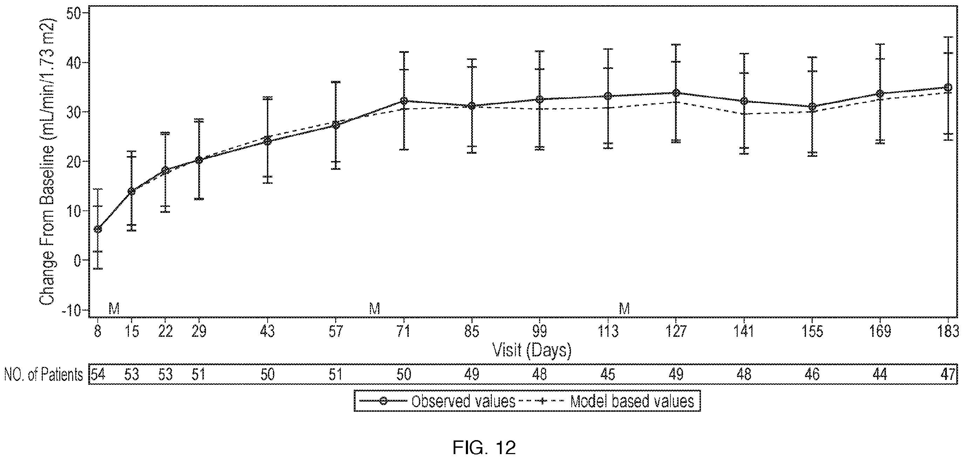

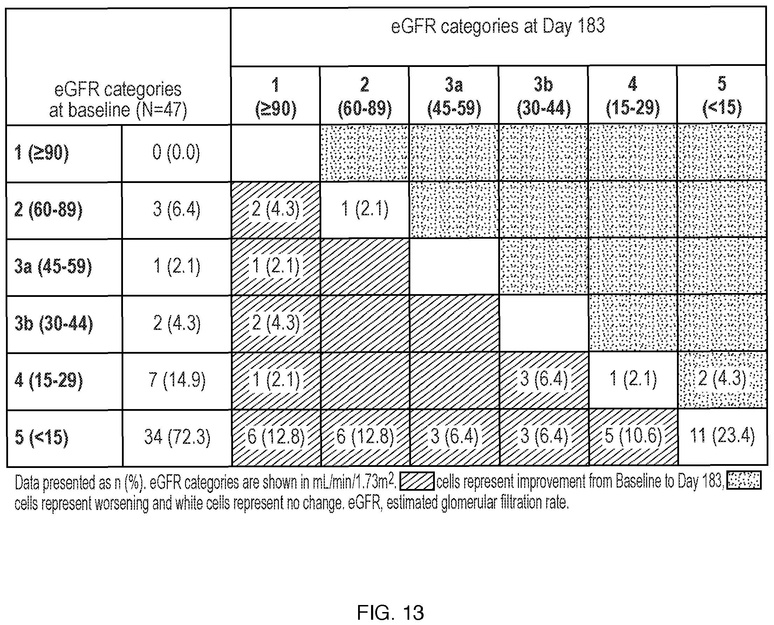

Figures 12 shows the mean eGFR from baseline and 95% confidence interval. Figure 13 shows the shift in CKD / eGFR categories from baseline to Day 183. The data is presented is n (%). eGFR categories are shown in mL/min/1.73m². Baseline is derived based on the last available eGFR before starting treatment. The lower triangle (denoted by angled black lines) represents improvement from Baseline to Day 183, the upper triangle (denoted by dots) represents worsening, and the white cells represent no change.

Moreover, as indicated in Table 20 and Figure 13, thirty-two (32) out of forty-seven (47) subjects improved in CKD stage change from baseline to Day 183 (six by 5 stages, seven by 4 stages, five by 3 stages, four by 2 stages, and ten by 1 stage). Thirteen (13) out of forty-seven (47) subjects stayed the same. Two (2) out of thirteen (13) worsened.

**Table 20: Key Safety Results: Secondary, CKD Shift**

| **Visit** | **Status^{(a)}** | **Statistic** | **Overall** |
|---|---|---|---|
| Day 183 | Improved^{(b)} | n/m | 32/47 |
| | | Proportion (95% CI)^{(d)} | 0.681 (0.529, 0.809) |
| | Worsened^{(c)} | n/m | 2/13 |
| | | Proportion (95% CI)^{(d)} | 0.154 (0.019, 0.454) |
| | Stayed the Same | n/m | 13/47 |
| | | Proportion (95% CI)^{(d)} | 0.277 (0.156, 0.426) |

| | | | |
|---|---|---|---|
| (a) Compared to CKD stage at baseline. (b) Excluded those with Stage 1 at baseline as they could not improve. (c) Excluded those with Stage 5 at baseline as they could not worsen. (d) 95% confidence intervals (95% CIs) for the proportion were based on exact confidence limits using the Clopper-Pearson method. | | | |

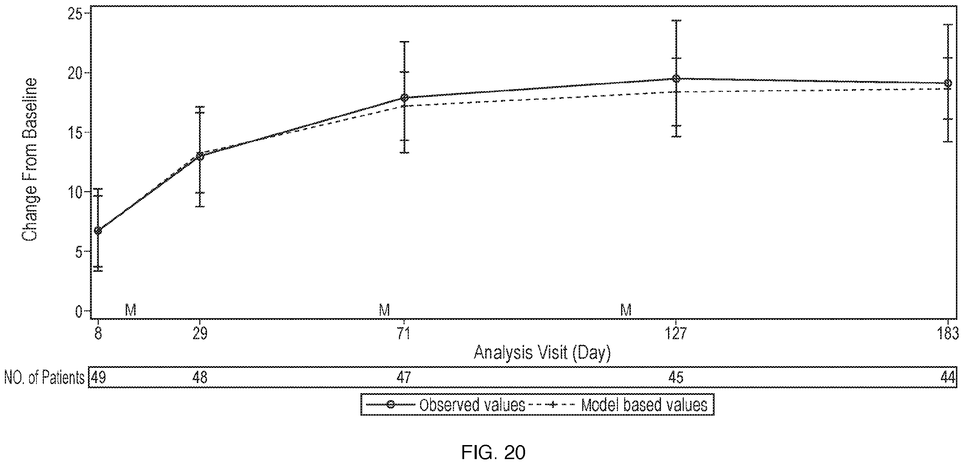

Table 21 and Figure 20 show the change in fatigue over time. The data in Figure 20 is shown as mean (error bars, 95% CI). A rapid improvement in fatigue was observed, i.e., a median 9-point improvement by Day 8. A clinically meaningful improvement in fatigue (≥ 3 points) was observed in 84.1 % (37/44) of patients at Day 183. The median increase was 20 points from Baseline to Day 183.

**Table 21: Key Safety Results: FACIT - 3 Point Improvement from Baseline**

| **Visit** | **Statistic** | **Overall (N=56)** |
|---|---|---|
| Day 8 | n/m | 31/49 |
| | Proportion (95%CI) | 0.633 (0.483, 0.766) |
| | | |
| Day 29 | n/m | 37/48 |
| | Proportion (95%CI) | 0.771 (0.627, 0.880) |
| | | |
| Day 71 | n/m | 38/47 |
| | Proportion (95%CI) | 0.809 (0.667, 0.909) |
| | | |
| Day 127 | n/m | 37/45 |
| | Proportion (95%CI) | 0.822 (0.679, 0.920) |
| | | |
| Day 183 | n/m | 37/44 |
| | Proportion (95%CI) | 0.841 (0.699, 0.934) |

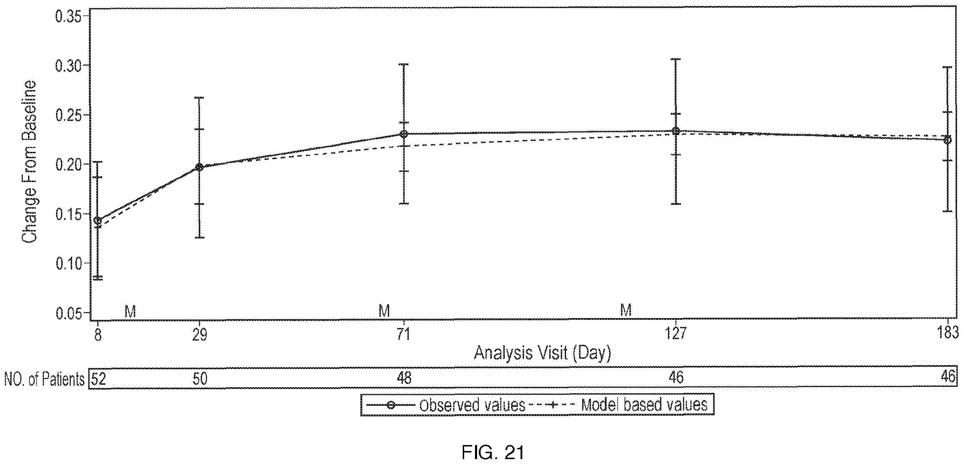

EQ-5D-3L is assessed using the index scored according to the Time Trade-Off value set for the United States (US TTO) as well as the response on the Visual Analogue Scale (VAS) question. US TTO > 0.94 indicates full health. Baseline is from the Day 1 value. Figure 21 shows the mean EQ-5D-3L and 95% confidence interval. With respect to immunogenicity, one patient with a treatment emergent positive result for antidrug antibodies (ADAs) was observed, with no neutralizing antibodies and no apparent effect on pharmacokinetics/pharmacodynamics (see Table 22).

**Table 22: Key Safety Results: Overview of Safety: Immunogenicity Antidrug Antibodies**

| | **Overall (N=58)** | | |
|---|---|---|---|
| | | | |
| **Visit** | **m** | **Positive n (%)** | **Negative n (%)** |
| Baseline | 57 | 18 (31.6) | 39 (68.4) |
| Day 71 | 52 | 2 (3.8) | 50 (96.2) |
| Day 127 | 49 | 0 (0.0) | 49 (100.0) |
| Day 183 | 47 | 0 (0.0) | 47 (100.0) |
| Day 351 | 17 | 0 (0.0) | 17 (100.0) |
| Up to Day 183 | 54 | 2 (3.7) | 52 (96.3) |
| Entire Follow-up | 54 | 2 (3.7) | 52 (96.3) |

Figure 22 depicts serum free complement C5 concentrations over time (semi-log scale). Dashed horizontal line indicates serum free C5 concentration of 0.5 µg/mL, with complete inhibition of terminal complement defined as serum free C5 concentration ≤ 0.5 µg/mL. The following free C5 samples on day 1 were excluded as they were considered biologically implausible. The exclusions were corroborated with the paired PK data, as the PK and free C5 samples were collected from the same blood draw. [N=2, Day 1 pre-dose samples / N=1, Day 1 end of infusion sample]. As evidenced by Figure 22, ravulizumab showed immediate, complete, and sustained terminal complement inhibition over the 8-week dosing interval.

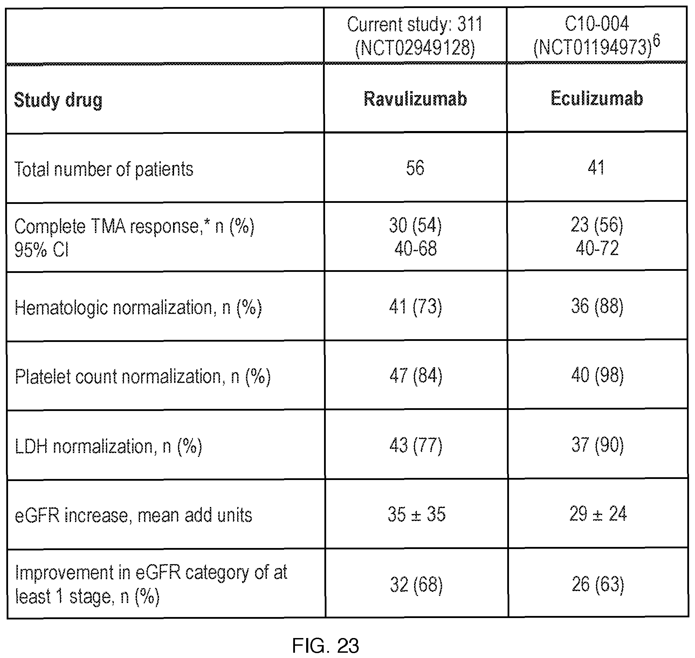

Finally, the study population between the current study with ravulizumab (ALXN1210-aHUS-311) and the eculizumab adult study (C10-004) were similar at Day 183. However, as shown in Figure 23, ravulizumab resulted in improved readouts compared to eculizumab with respect to the following secondary clinical parameters: a) eGFR category / chronic kidney disease (CKD) staging and (b) estimated glomerular filtration rate (eGFR) increase. Specifically, of the patients treated with ravulizumab in the present study, 68% achieved an improvement in eGFR category / CKD staging of at least one stage and there was a mean increase in eGFR of 35 ± 35. In contrast, of the patients treated with eculizumab in study C10-004, only 63% achieved an improvement in eGFR category / CKD staging of at least one stage and there was a mean increase in eGFR of 29 ± 24. A "complete TMA response" in the current study is the equivalent of a "modified complete TMA response" in C10-004.

In sum, ravulizumab provided immediate and complete inhibition of C5 that was sustained over the 8-week dosing interval. A complete TMA response was achieved in 54% of patients, which is similar to data for eculizumab (56% in study C10-004). There was a rapid improvement in platelet count. In addition, renal function substantially improved. Specifically, 58.6% of the patients on dialysis at baseline did not require dialysis at the end of the study. Moreover, no unexpected safety concerns were identified. Thus, the results from this study support the use of ravulizumab at 8-weekly dosing intervals in adult patients with complement-mediated TMA.

### SEQUENCE SUMMARY

| |
|---|
| **SEQ ID NO:1** |
| GYIF SNYW IQ |
| **SEQ ID NO:2** |
| EILPGSGSTEYTENFKD |
| **SEQ ID NO:3** |
| YFFGSSPNWYFDV |
| **SEQ ID NO:4** |
| GASENIYGALN |
| **SEQ ID NO:5** |
| GATNLAD |
| **SEQ ID NO:6** |
| QNVLNTPLT |
| **SEQ ID NO:7** |
| |
| **SEQ ID NO:8** |
| |
| **SEQ ID NO:9** |
| ASTKGPSVFPLAPCSRSTSESTAALGCLVKDYFPEPVTVSWNSGALTSGVH TFPAVLQSSGLYSLSSVVTVPSSNFGTQTYTCNVDHKPSNTKVDKTVERKC CVECPPCPAPPVAGPSVFLFPPKPKDTLMISRTPEVTCVVVDVSQEDPEVQF NWYVDGVEVHNAKTKPREEQFNSTYRVVSVLTVLHQDWLNGKEYKCKV SNKGLPSSIEKTISKAKGQPREPQVYTLPPSQEEMTKNQVSLTCLVKGFYPS DIAVEWESNGQPENNYKTTPPVLDSDGSFFLYSRLTVDKSRWQEGNVFSCS VMHEALHNHYTQKSLSLSLGK |
| **SEQ ID NO:10** |
| QVQLVQSGAEVKKPGASVKVSCKASGYIFSNYWIQWVRQAPGQGLEWM GEILPGSGSTEYTENFKDRVTMTRDTSTSTVYMELSSLRSEDTAVYYCAR YFFGSSPNWYFDVWGQGTLVTVSSASTKGPSVFPLAPCSRSTSESTAALGCL VKDYFPEPVTVSWNSGALTSGVHTFPAVLQSSGLYSLSSVVTVPSSNFGTQTYT CNVDHKPSNTKVDKTVERKCCVECPPCPAPPVAGPSVFLFPPKPKDTLMISR TPEVTCVVVDVSQEDPEVQFNWYVDGVEVHNAKTKPREEQFNSTYRVVSVLT VLHQDWLNGKEYKCKVSNKGLPSSIEKTISKAKGQPREPQVYTLPPSQEEMT KNQVSLTCLVKGFYPSDIAVEWESNGQPENNYKTTPPVLDSDGSFFLYSRLTV |
| DKSRWQEGNVFSCSVMHEALHNHYTQKSLSLSLGK |
| **SEQ ID NO:11** |
| |
| **SEQ ID NO:12** |
| |
| **SEQ ID NO:13** |
| ASTKGPSVFPLAPCSRSTSESTAALGCLVKDYFPEPVTVSWNSGALTSGV HTFPAVLQSSGLYSLSSVVTVPSSNFGTQTYTCNVDHKPSNTKVDKTVER KCCVECPPCPAPPVAGPSVFLFPPKPKDTLMISRTPEVTCVVVDVSQEDPE VQFNWYVDGVEVHNAKTKPREEQFNSTYRVVSVLTVLHQDWLNGKEYK CKVSNKGLPSSIEKTISKAKGQPREPQVYTLPPSQEEMTKNQVSLTCLVKG FYPSDIAVEWESNGQPENNYKTTPPVLDSDGSFFLYSRLTVDKSRWQEGN VFSCSVLHEALHSHYTQKSLSLSLGK |
| **SEQ ID NO:14** |
| QVQLVQSGAEVKKPGASVKVSCKASGHIFSNYWIQWVRQAPGQGLEWM GEILPGSGHTEYTENFKDRVTMTRDTSTSTVYMELSSLRSEDTAVYYCAR YFFGSSPNWYFDVWGQGTLVTVSS ASTKGPSVFPLAPCSRSTSESTAALGCL VKDYFPEPVTVSWNSGALTSGVHTFPAVLQSSGLYSLSSVVTVPSSNFGTQTYT CNVDHKPSNTKVDKTVERKCCVECPPCPAPPVAGPSVFLFPPKPKDTLMISR TPEVTCVVVDVSQEDPEVQFNWYVDGVEVHNAKTKPREEQFNSTYRVVSVLT VLHQDWLNGKEYKCKVSNKGLPSSIEKTISKAKGQPREPQVYTLPPSQEEMT KNQVSLTCLVKGFYPSDIAVEWESNGQPENNYKTTPPVLDSDGSFFLYSRLTV DKSRWQEGNVFSCSVLHEALHSHYTQKSLSLSLGK |
| **SEQ ID NO:15** |
| ASTKGPSVFPLAPCSRSTSESTAALGCLVKDYFPEPVTVSWNSGALTSGVH TFPAVLQSSGLYSLSSVVTVTSSNFGTQTYTCNVDHKPSNTKVDKTVERKC CVECPPCPAPPVAGPSVFLFPPKPKDTLYITREPEVTCVVVDVSHEDPEVQF NWYVDGMEVHNAKTKPREEQFNSTFRVVSVLTVVHQDWLNGKEYKCKV SNKGLPAPIEKTISKTKGQPREPQVYTLPPSREEMTKNQVSLTCLVKGFYP SDIAVEWESNGQPENNYKTTPPMLDSDGSFFLYSKLTVDKSRWQQGNVF SCSVMHEALHNHYTQKSLSLSPGK |
| **SEQ ID NO:16** |
| QVQLVQSGAEVKKPGASVKVSCKASGYIFSNYWIQWVRQAPGQGLEWM GEILPGSGSTEYTENFKDRVTMTRDTSTSTVYMELSSLRSEDTAVYYCAR YFFGSSPNWYFDVWGQGTLVTVSSASTKGPSVFPLAPCSRSTSESTAALG CLVKDYFPEPVTVSWNSGALTSGVHTFPAVLQSSGLYSLSSVVTVTSSNF GTQTYTCNVDHKPSNTKVDKTVERKCCVECPPCPAPPVAGPSVFLFPPKP KDTLYITREPEVTCVVVDVSHEDPEVQFNWYVDGMEVHNAKTKPREEQ FNSTFRVVSVLTVVHQDWLNGKEYKCKVSNKGLPAPIEKTISKTKGQPRE PQVYTLPPSREEMTKNQVSLTCLVKGFYPSDIAVEWESNGQPENNYKTTP PMLDSDGSFFLYSKLTVDKSRWQQGNVFSCSVMHEALHNHYTQKSLSLS PGK |
| **SEQ ID NO:17** |
| GASENIYHALN |
| **SEQ ID NO:18** |
| EILPGSGHTEYTENFKD |
| **SEQ ID NO:19** |
| |
| GHIFSNYWIQ **SEQ ID NO:20** |
| QVQLVQSGAEVKKPGASVKVSCKASGHIFSNYWIQWVRQAPGQGLEW MGEILPGSGHTEYTENFKDRVTMTRDTSTSTVYMELSSLRSEDTAVYYC ARYFFGSSPNWYFDVWGQGTLVTVSSASTKGPSVFPLAPCSRSTSESTAALG CLVKDYFPEPVTVSWNSGALTSGVHTFPAVLQSSGLYSLSSVVTVPSSNFGTQT YTCNVDHKPSNTKVDKTVERKCCVECPPCPAPPVAGPSVFLFPPKPKDTLMIS RTPEVTCVVVDVSQEDPEVQFNWYVDGVEVHNAKTKPREEQFNSTYRVVSVL TVLHQDWLNGKEYKCKVSNKGLPSSIEKTISKAKGQPREPQVYTLPPSQEEMT KNQVSLTCLVKGFYPSDIAVEWESNGQPENNYKTTPPVLDSDGSFFLYSRLTV DKSRWQEGNVFSCSVMHEALHNHYTQKSLSLSLGK |
| **SEQ ID NO:21** |
| SYAIS |
| **SEQ ID NO:22** |
| GIGPFFGTANYAQKFQG |
| **SEQ ID NO:23** |
| DTPYFDY |
| **SEQ ID NO:24** |
| SGDSIPNYYVY |
| **SEQ ID NO:25** |
| DDSNRPS |
| **SEQ ID NO:26** |
| QSFDSSLNAEV |
| **SEQ ID NO:27** |
| QVQLVQSGAEVKKPGSSVKVSCKASGGTFSSYAISVWRQAPGQGLEWMGGIGPF FGTANYAQKFQGRVTITADESTSTAYMELSSLRSEDTAVYYCARDTPYFD YWGQGTLVTVSS |
| **SEQ ID NO:28** |
| DIELTQPPSVSVAPGQTARISCSGDSIPNYYVYWYQQKPGQAPVLVIYDDSNRPSG IPERFSGSNSGNTATLTISGTQAEDEADYYCQSFDSSLNAEVFGGGTK LTVL |
| **SEQ ID NO:29** |
| NYIS |
| **SEQ ID NO:30** |
| IIDPDDSYTEYSPSFQG |
| **SEQ ID NO:31** |
| YEYGGFDI |
| **SEQ ID NO:32** |
| SGDNIGNSYVH |
| **SEQ ID NO:33** |
| KDNDRPS |
| **SEQ ID NO:34** |
| GTYDIESYV |
| **SEQ ID NO:35** |
| EVQLVQSGAEVKKPGESLKISCKGSGYSFTNYISWVRQMPGKGLEWMGIIDPDDS YTEYSPSFQGQVTI SADKSISTAYLQWSSLKASDTAMYYCARYEYGGFDI WGQGTLVTVSS |
| **SEQ ID NO:36** |
| SYELTQPPSVSVAPGQTARISCSGDNIGNSYVHWYQQKPGQAPVLVIYKDNDRPS GIPERFSGSNSGNT ATLTISGTQAEDEADYYCGTYDIESYVFGGGTKLTV L |
| **SEQ ID NO:37** |
| SSYYVA |
| **SEQ ID NO:38** |
| AIYTGSGATYKASWAKG |
| **SEQ ID NO:39** |
| DGGYDYPTHAMHY |
| **SEQ ID NO:40** |
| QASQNIGSSLA |
| **SEQ ID NO:41** |
| GASKTHS |
| **SEQ ID NO:42** |
| QSTKVGSSYGNH |
| **SEQ ID NO:43** |
| QVQLVESGGGLVQPGGSLRLSCAASGFTSHSSYYVAWVRQAPGKGLEWVGAIYT GSGATYKASWAKGRFTISKDTSKNQVVLTMTNMDPVDTATYYCASDGGYDYPT HAMHYWGQGTLVTVSS |
| **SEQ ID NO:44** |
| DVVMTQSPSSLSASVGDRVTITCQASQNIGSSLAWYQQKPGQAPRLLIYGASKTH SGVPSRFSGSGSGTDFTLTISSLQPEDVATYYCQSTKVGSSYGNHFGGGTKVEIK |
| **SEQ ID NO:45** |
| QVQLVESGGGLVQPGRSLRLSCAASGFTVHSSYYMAWVRQAPGKGLEWVGAIF TGSGAEYKAEWAKGRVTISKDTSKNQVVLTMTNMDPVDTATYYCASDAGYDYP THAMHYWGQGTLVTVSSASTKGPSVFPLAPSSKSTSGGTAALGCLVKDYFPEPVT VSWNSGALTSGVHTFPAVLQSSGLYSLSSVVTVPSSSLGTQTYICNVNHKPSNTK VDKKVEPKSCDKTHTCPPCPAPELRRGPKVFLFPPKPKDTLMISRTPEVTCVVVDV SHEDPEVKFNWYVDGVEVHNAKTKPREEQYNSTYRVVSVLTVLHQDWLNGKEY KCKVSNKGLPSSIEKTISKAKGQPREPQVYTLPPSREEMTKNQVSLTCLVKGFYPS DIAVEWESNGQPENNYKTTPPVLDSDGSFFLYSKLTVDKSRWQQGNVFSCSVLHE ALHAHYTRKELSLSP |
| **SEQ ID NO:46** |
| DIQMTQSPSSLSASVGDRVTITCRASQGISSSLAWYQQKPGKAPKLLIYGASETES GVPSRFSGSGSGTDFTLTISSLQPEDFATYYCQNTKVGSSYGNTFGGGTKVEIKRT VAAPSVFIFPPSDEQLKSGTASVVCLLNNFYPREAKVQWKVDNALQSGNSQESVT EQDSKDSTYSLSSTLTLSKADYEKHKVYACEVTHQGLSSPVTKSFNRGEC |
| **SEQ ID NO:47** |
| QVQLQESGPGLVKPSETLSLTCTVSGDSVSSSYWTWIRQPPGKGLEWIGYIYYSGS SNYNPSLKSRATISVDTSKNQFSLKLSSVTAADTAVYYCAREGNVDTTMIFDYWG QGTLVTVSS |
| **SEQ ID NO:48** |
| AIQMTQSPSSLSASVGDRVTITCRASQGIRNDLGWYQQKPGKAPKLLIYAASSLQS GVPSRFAGRGSGTDFTLTISSLQPEDFATYYCLQDFNYPWTFGQGTKVEIK |
| **SEQ ID NO:49** |
| QVQLQESGPGLVKPSETLSLTCTVSGDSVSSSYWTWIRQPPGKGLEWIGYIYYSGS SNYNPSLKSRATISVDTSKNQFSLKLSSVTAADTAVYYCAREGNVDTTMIFDYWG QGTLVTVSSASTKGPSVFPLAPCSRSTSESTAALGCLVKDYFPEPVTVSWNSGALT SGVHTFPAVLQSSGLYSLSSVVTVPSSSLGTKTYTCNVDHKPSNTKVDKRVESKY GPPCPPCPAPEFLGGPSVFLFPPKPKDTLMISRTPEVTCVVVDVSQEDPEVQFNWY VDGVEVHNAKTKPREEQFNSTYRVVSVLTVLHQDWLNGKEYKCKVSNKGLPSSI |
| EKTISKAKGQPREPQVYTLPPSQEEMTKNQVSLTCLVKGFYPSDIAVEWESNGQP ENNYKTTPPVLDSDGSFFLYSRLTVDKSRWQEGNVFSCSVMHEALHNHYTQKSL SLSLGK |
| **SEQ ID NO:50** |
| AIQMTQSPSSLSASVGDRVTITCRASQGIRNDLGWYQQKPGKAPKLLIYAASSLQS GVPSRFAGRGSGTDFTLTISSLQPEDFATYYCLQDFNYPWTFGQGTKVEIKRTVA APSVFIFPPSDEQLKSGTASVVCLLNNFYPREAKVQWKVDNALQSGNSQESVTEQ DSKDSTYSLSSTLTLSKADYEKHKVYACEVTHQGLSSPVTKSFNRGEC |

## Claims

1. An anti-C5 antibody for use in a method of treating atypical hemolytic uremic syndrome (aHUS) in a human adult patient who is 18 years or older, wherein the anti-C5 antibody is ravulizumab, and the anti-C5 antibody is administered intravenously:
(a) once on Day 1 at a dose of: 2400 mg to a patient weighing ≥ 40 to < 60 kg, 2700 mg to a patient weighing ≥ 60 to < 100 kg, or 3000 mg to a patient weighing ≥ 100 kg; and
(b) on Day 15 and every eight weeks thereafter at a dose of 3000 mg to a patient weighing ≥ 40 to < 60 kg, 3300 mg to a patient weighing ≥ 60 to < 100 kg, or 3600 mg to a patient weighing ≥ 100 kg.

2. The anti-C5 antibody for use according to claim 1, wherein the patient has previously been treated with eculizumab.

3. The anti-C5 antibody for use according to claim 2, wherein:
(a) the treatment starts at least two weeks after the patient's last dose of eculizumab;
(b) the patient has been treated with eculizumab for at least 6 months prior to Day 1 of the treatment; and/or
(c) the patient has previously been treated with eculizumab at a dose of 900 mg every 2 weeks.

4. The anti-C5 antibody for use according to any one of the preceding claims, wherein the anti-C5 antibody is administered to a patient weighing ≥ 40 to < 60 kg:
(a) once on Day 1 at a dose of 2400 mg; and
(b) on Day 15 and every eight weeks thereafter at a dose of 3000 mg.

5. The anti-C5 antibody for use according to any one of claims 1-3, wherein the anti-C5 antibody is administered to a patient weighing ≥ 60 to < 100 kg:
(a) once on Day 1 at a dose of 2700 mg; and
(b) on Day 15 and every eight weeks thereafter at a dose of 3300 mg.

6. The anti-C5 antibody for use according to any one of claims 1-3, wherein the anti-C5 antibody is administered to a patient weighing ≥ 100 kg:
(a) once on Day 1 at a dose of 3000 mg; and
(b) on Day 15 and every eight weeks thereafter at a dose of 3600 mg.

7. The anti-C5 antibody for use according to any one of the preceding claims, wherein:
(a) the treatment maintains a serum trough concentration of the anti-C5 antibody of 100 µg/ml or greater, or 200 µg/ml or greater, during the treatment;
(b) the treatment maintains a free C5 concentration of 0.309 to 0.5 µg/mL or below; and/or
(c) the treatment reduces free C5 concentration by greater than 99% or greater than 99.5% throughout the treatment period.

8. The anti-C5 antibody for use according to any one of the preceding claims, wherein the anti-C5 antibody is administered at a dose of 3000 mg, 3300 mg, or 3600 mg every eight weeks after the treatment for up to two years.

9. The anti-C5 antibody for use according to any one of the preceding claims, wherein the treatment is a total of 26 weeks.

10. The anti-C5 antibody for use according to any one of the preceding claims, wherein the treatment results in:
(a) terminal complement inhibition;
(b) a reduction of hemolysis compared to baseline as assessed by lactate dehydrogenase (LDH) levels;
(c) a normalization of LDH levels; and/or
(d) an elimination of breakthrough hemolysis during the treatment period.

11. The anti-C5 antibody for use according to any one of the preceding claims, wherein the treatment produces:
(a) a shift toward normal levels of a hemolysis-related hematologic biomarker selected from the group consisting free hemoglobin, haptoglobin, reticulocyte count, PNH red blood cell (RBC) clone and D-dimer;
(b) a shift toward normal levels of Factor Ba, soluble tumor necrosis factor receptor 1 [sTNFR1]), soluble vascular adhesion molecule 1 [sVCAM1], thrombomodulin, D-dimer, and cystatin C;
(c) an increase in hemoglobin stabilization compared to baseline;
(d) a reduction in the need for blood transfusions compared to baseline;
(e) a reduction in major adverse vascular events (MAVEs); and/or
(f) a change from baseline in quality of life, as assessed via the Functional Assessment of Chronic Illness Therapy (FACIT)-Fatigue Scale, version 4 and the European Organisation for Research and Treatment of Cancer, Quality of Life Questionnaire-Core 30 Scale.

12. The anti-C5 antibody for use according to any one of the preceding claims, wherein the treatment produces at least one therapeutic effect selected from the group consisting of a reduction or cessation in severe hypertension, proteinuria, uremia, lethargy, fatigue, irritability, thrombocytopenia, microangiopathic hemolytic anemia, and renal function impairment compared to baseline.

13. The anti-C5 antibody for use according to any one of the preceding claims, wherein the treatment results in:
(a) platelet normalization;
(b) a ≥25% improvement from baseline in serum creatinine;
(c) a complete TMA response; and/or
(d) a modified complete TMA response.

14. The anti-C5 antibody for use according to any one of the preceding claims, wherein the patient's chronic kidney disease (CKD) improves by one or more stages after initiating treatment.

15. The anti-C5 antibody for use according to any one of the preceding claims, wherein the treatment results in:
(a) a shift towards normal levels of eGFR; (estimated glomerular filtration rate) and/or
(b) a EQ-5D-3L Time Trade-Off value set for the United States (US TTO) of > 0.94.

## Patentansprüche

1. Anti-C5-Antikörper zur Verwendung in einem Verfahren zur Behandlung des atypischen hämolytisch-urämischen Syndroms (aHUS) bei einem menschlichen erwachsenen Patienten, der 18 Jahre oder älter ist, wobei der Anti-C5-Antikörper Ravulizumab ist und der Anti-C5-Antikörper intravenös wie folgt verabreicht wird:
(a) einmal an Tag 1 in einer Dosis von: 2400 mg an einen Patienten mit einem Gewicht von ≥ 40 bis < 60 kg, 2700 mg an einen Patienten mit einem Gewicht von ≥ 60 bis < 100 kg, oder 3000 mg an einen Patienten mit einem Gewicht von ≥ 100 kg; und
(b) am Tag 15 und danach alle acht Wochen in einer Dosis von 3000 mg an einen Patienten mit einem Gewicht von ≥ 40 bis < 60 kg, 3300 mg an einen Patienten mit einem Gewicht von ≥ 60 bis < 100 kg, oder 3600 mg an einen Patienten mit einem Gewicht von ≥ 100 kg.

2. Anti-C5-Antikörper zur Verwendung nach Anspruch 1, wobei der Patient zuvor mit Eculizumab behandelt worden ist.

3. Anti-C5-Antikörper zur Verwendung nach Anspruch 2, wobei:
(a) die Behandlung mindestens zwei Wochen nach der letzten Eculizumab-Dosis des Patienten beginnt;
(b) der Patient vor Tag 1 der Behandlung mindestens 6 Monate lang mit Eculizumab behandelt wurde; und/oder
(c) der Patient zuvor mit Eculizumab in einer Dosis von 900 mg alle 2 Wochen behandelt wurde.

4. Anti-C5-Antikörper zur Verwendung nach einem der vorhergehenden Ansprüche, wobei der Anti-C5-Antikörper an einen Patienten mit einem Gewicht von ≥ 40 bis < 60 kg wie folgt verabreicht wird:
(a) einmal an Tag 1 in einer Dosis von 2400 mg; und
(b) am Tag 15 und danach alle acht Wochen in einer Dosis von 3000 mg.

5. Anti-C5-Antikörper zur Verwendung nach einem der Ansprüche 1 bis 3, wobei der Anti-C5-Antikörper an einen Patienten mit einem Gewicht von ≥ 60 bis < 100 kg wie folgt verabreicht wird:
(a) einmal an Tag 1 in einer Dosis von 2700 mg; und
(b) am Tag 15 und danach alle acht Wochen in einer Dosis von 3300 mg.

6. Anti-C5-Antikörper zur Verwendung nach einem der Ansprüche 1 bis 3, wobei der Anti-C5-Antikörper an einen Patienten mit einem Gewicht von ≥ 100 kg wie folgt verabreicht wird:
(a) einmal an Tag 1 in einer Dosis von 3000 mg; und
(b) am Tag 15 und danach alle acht Wochen in einer Dosis von 3600 mg.

7. Anti-C5-Antikörper zur Verwendung nach einem der vorhergehenden Ansprüche, wobei:
(a) die Behandlung eine Serum-Trogkonzentration des Anti-C5-Antikörpers von 100 µg/ml oder mehr oder 200 µg/ml oder mehr während der Behandlung aufrechterhält;
(b) die Behandlung eine freie C5-Konzentration von 0,309 bis 0,5 µg/ml oder weniger aufrechterhält; und/oder
(c) die Behandlung die Konzentration von freiem C5 um mehr als 99 % oder mehr als 99,5 % während des gesamten Behandlungszeitraums reduziert.

8. Anti-C5-Antikörper zur Verwendung nach einem der vorhergehenden Ansprüche, wobei der Anti-C5-Antikörper in einer Dosis von 3000 mg, 3300 mg oder 3600 mg alle acht Wochen nach der Behandlung für bis zu zwei Jahre verabreicht wird.

9. Anti-C5-Antikörper zur Verwendung nach einem der vorhergehenden Ansprüche, wobei die Behandlung insgesamt 26 Wochen dauert.

10. Anti-C5-Antikörper zur Verwendung nach einem der vorhergehenden Ansprüche, wobei die Behandlung zu Folgendem führt:
(a) einer terminalen Komplementinhibition;
(b) einer Verringerung der Hämolyse im Vergleich zum Ausgangswert, wie anhand der Laktatdehydrogenase (LDH)-Spiegel beurteilt;
(c) einer Normalisierung der LDH-Werte; und/oder
(d) einer Eliminierung der Durchbruchshämolyse während des Behandlungszeitraums.

11. Anti-C5-Antikörper zur Verwendung nach einem der vorhergehenden Ansprüche, wobei die Behandlung Folgendes erzeugt:
(a) eine Verschiebung in Richtung normaler Werte eines hämolysebezogenen hämatologischen Biomarkers, ausgewählt aus der Gruppe bestehend aus freiem Hämoglobin, Haptoglobin, Retikulozytenzahl, PNH-Klon der roten Blutkörperchen (RBC) und D-Dimer;
(b) eine Verschiebung hin zu normalen Spiegeln von Faktor Ba, löslichem Tumor-Nekrose-Faktor-Rezeptor 1 [sTNFRl], löslichem vaskulärem Adhäsionsmolekül 1 [sVCAMl], Thrombomodulin, D-Dimer und Cystatin C;
(c) eine Erhöhung der Hämoglobinstabilisierung im Vergleich zum Ausgangswert;
(d) eine Verringerung des Bedarfs an Bluttransfusionen im Vergleich zum Ausgangswert;
(e) eine Verringerung der wichtigsten unerwünschten vaskulären Ereignisse (MAVEs); und/oder
(f) eine Veränderung der Lebensqualität im Vergleich zum Ausgangswert, bewertet mit der Ermüdungs-Skala der funktionellen Bewertung von chronischer Krankheitstherapie (Functional Assessment of Chronic Illness Therapy (FACIT)-Fatigue Scale) Version 4 und der Lebensqualitätsfragebogen-Kern-30-Skala (Quality of Life Questionnaire-Core 30 Scale) der Europäischen Organisation zur Krebsforschung und -behandlung.

12. Anti-C5-Antikörper zur Verwendung nach einem der vorhergehenden Ansprüche, wobei die Behandlung mindestens eine therapeutische Wirkung hervorruft, ausgewählt aus der Gruppe bestehend aus einer Verringerung oder Beendigung von schwerem Bluthochdruck, Proteinurie, Urämie, Lethargie, Müdigkeit, Reizbarkeit, Thrombozytopenie, mikroangiopathischer hämolytischer Anämie und Beeinträchtigung der Nierenfunktion im Vergleich zum Ausgangswert.

13. Anti-C5-Antikörper zur Verwendung nach einem der vorhergehenden Ansprüche, wobei die Behandlung zu Folgendem führt:
(a) Normalisierung der Blutplättchen;
(b) eine Verbesserung des Serumkreatinins um ≥25% gegenüber dem Ausgangswert;
(c) eine vollständige TMA-Antwort; und/oder
(d) ein modifiziertes vollständiges TMA-Ansprechen.

14. Anti-C5-Antikörper zur Verwendung nach einem der vorhergehenden Ansprüche, wobei sich die chronische Nierenerkrankung (Chronic Kidney Disease, CKD) des Patienten nach Beginn der Behandlung um eine oder mehrere Stufen verbessert.

15. Anti-C5-Antikörper zur Verwendung nach einem der vorhergehenden Ansprüche, wobei
die Behandlung zu Folgendem führt:
(a) einer Verschiebung hin zu normalen Werten der geschätzten glomerulären Filtrationsrate (estimated Glomerular Filtration Rate; eGFR) und/oder
(b) einem EQ-5D-3L-Zeitausgleichswert für die Vereinigten Staaten (US Time Trade-Off, US TTO) von > 0,94.

## Revendications

1. Anticorps anti-C5 pour une utilisation dans un procédé de traitement de syndrome hémolytique et urémique atypique (SHUa) chez un patient adulte humain âgé de 18 ans ou plus, dans lequel l'anticorps anti-C5 est le ravulizumab, et l'anticorps anti-C5 est administré par voie intraveineuse :
(a) une fois au jour 1 à une dose de : 2 400 mg à un patient de poids ≥ 40 à < 60 kg, 2 700 mg à un patient de poids ≥ 60 à < 100 kg, ou 3 000 mg à un patient de poids ≥ 100 kg ; et
(b) au jour 15 et toutes les huit semaines par la suite, à une dose de 3 000 mg à un patient de poids ≥ 40 à < 60 kg, 3 300 mg à un patient de poids ≥ 60 à < 100 kg, ou 3 600 mg à un patient de poids ≥ 100 kg.

2. Anticorps anti-C5 pour une utilisation selon la revendication 1, dans lequel le patient a été précédemment traité avec de l'éculizumab.

3. Anticorps anti-C5 pour une utilisation selon la revendication 2, dans lequel :
(a) le traitement commence au moins deux semaines après la dernière dose d'éculizumab du patient ;
(b) le patient a été traité avec de l'éculizumab pendant au moins 6 mois avant le jour 1 du traitement ; et/ou
(c) le patient a été précédemment traité avec de l'éculizumab à une dose de 900 mg toutes les 2 semaines.

4. Anticorps anti-C5 pour une utilisation selon l'une quelconque des revendications précédentes, dans lequel l'anticorps anti-C5 est administré à un patient de poids ≥ 40 à < 60 kg :
(a) une fois au jour 1 à une dose de 2 400 mg ; et
(b) au jour 15 et toutes les huit semaines par la suite, à une dose de 3 000 mg.

5. Anticorps anti-C5 pour une utilisation selon l'une quelconque des revendications 1 à 3, dans lequel l'anticorps anti-C5 est administré à un patient de poids ≥ 60 à < 100 kg :
(a) une fois au jour 1 à une dose de 2 700 mg ; et
(b) au jour 15 et toutes les huit semaines par la suite, à une dose de 3 300 mg.

6. Anticorps anti-C5 pour une utilisation selon l'une quelconque des revendications 1 à 3, dans lequel l'anticorps anti-C5 est administré à un patient de poids ≥ 100 kg :
(a) une fois au jour 1 à une dose de 3 000 mg ; et
(b) au jour 15 et toutes les huit semaines par la suite, à une dose de 3 600 mg.

7. Anticorps anti-C5 pour une utilisation selon l'une quelconque des revendications précédentes, dans lequel :
(a) le traitement maintient une concentration sérique minimale de l'anticorps anti-C5 de 100 µg/ml ou plus, ou de 200 µg/ml ou plus, pendant le traitement ;
(b) le traitement maintient une concentration de C5 libre de 0,309 à 0,5 µg/mL ou moins ; et/ou
(c) le traitement réduit la concentration de C5 libre de plus de 99 % ou de plus de 99,5 % pendant toute la période de traitement.

8. Anticorps anti-C5 pour une utilisation selon l'une quelconque des revendications précédentes, dans lequel l'anticorps anti-C5 est administré à une dose de 3 000 mg, 3 300 mg, ou 3 600 mg toutes les huit semaines après le traitement, pendant une durée jusqu'à deux ans.

9. Anticorps anti-C5 pour une utilisation selon l'une quelconque des revendications précédentes, dans lequel le traitement est d'une durée totale de 26 semaines.

10. Anticorps anti-C5 pour une utilisation selon l'une quelconque des revendications précédentes, dans lequel le traitement entraîne :
(a) une inhibition du complément terminal ;
(b) une réduction de l'hémolyse comparativement à la ligne de base, évaluée par le taux de lactate déshydrogénase (LDH) ;
(c) une normalisation du taux de LDH ; et/ou
(d) une élimination de poussée d'hémolyse pendant la période de traitement.

11. Anticorps anti-C5 pour une utilisation selon l'une quelconque des revendications précédentes, dans lequel le traitement produit :
(a) une transition vers un taux normal d'un biomarqueur hématologique lié à l'hémolyse sélectionné dans le groupe constitué par l'hémoglobine, l'haptoglobine, la numération réticulocytaire, les clones de globules rouges (GR) de HPN et les D-dimères ;
(b) une transition vers un taux normal de facteur Ba, de récepteur soluble de facteur de nécrose tumorale 1 [sTNFR1], de molécule soluble d'adhésion vasculaire 1 [sVCAM1], de thrombomoduline, de D-dimères, et de cystatine C ;
(c) une augmentation de la stabilisation d'hémoglobine comparativement à la ligne de base ;
(d) une réduction de la nécessité de transfusions sanguines comparativement à la ligne de base ;
(e) une réduction des événements vasculaires indésirables majeurs (MAVE) ; et/ou
(f) un changement de qualité de vie par rapport à la ligne de base, évalué par l'intermédiaire de l'échelle d'évaluation fonctionnelle de thérapie de maladies chroniques (FACIT)-fatigue (Functional Assessment of Chronic Illness Therapy (FACIT)-Fatigue Scale), version 4 et Questionnaire-Core 30 sur la qualité de vie de l'Organisation européenne pour la recherche et le traitement du cancer.

12. Anticorps anti-C5 pour une utilisation selon l'une quelconque des revendications précédentes, dans lequel le traitement produit au moins un effet thérapeutique sélectionné dans le groupe constitué par une réduction ou un arrêt de l'hypertension sévère, de la protéinurie, de l'urémie, de la léthargie, de la fatigue, de l'irritabilité, de la thrombocytopénie, de l'anémie hémolytique microangiopathique, et de l'insuffisance rénale comparativement à la ligne de base.

13. Anticorps anti-C5 pour une utilisation selon l'une quelconque des revendications précédentes, dans lequel le traitement entraîne :
(a) une normalisation des plaquettes ;
(b) une amélioration de ≥ 25 % de la créatinine sérique par rapport à la ligne de base ;
(c) une réponse MAT complète ; et/ou
(d) une réponse MAT complète modifiée.

14. Anticorps anti-C5 pour une utilisation selon l'une quelconque des revendications précédentes, dans lequel la maladie rénale chronique (MRC) du patient s'améliore d'un ou de plusieurs stades après l'initiation du traitement.

15. Anticorps anti-C5 pour une utilisation selon l'une quelconque des revendications précédentes, dans lequel le traitement entraîne :
(a) une transition vers un taux normal de DFGe (débit de filtration glomérulaire estimé) ; et/ou
(b) une valeur d'arbitrage temporel EQ-5D-3L fixée pour les États-Unis (US TTO) de > 0,94.
